# EUROPÄISCHE PATENTSCHRIFT

(11) **EP 2 018 385 B1**
(45) Veröffentlichungstag und Bekanntmachung des Hinweises auf die Patenterteilung: **15.05.2013**
(21) Anmeldenummer: 07727385.2
(22) Anmeldetag: 27.03.2007
(51) Int. Cl.: C07D 513/04, A61K 31/429, A61P 29/00

(54) **THIAZOLYL-DIHYDRO-QUINAZOLINE**
THIAZOLYL-DIHYDROQUINAZOLINES
THIAZOLYL-DIHYDRO-QUINAZOLINES

(30) Priorität: 06.04.2006 EP 06112300
(43) Veröffentlichungstag der Anmeldung: 28.01.2009
(73) Patentinhaber: BOEHRINGER INGELHEIM INTERNATIONAL GMBH, 55216 Ingelheim am Rhein (DE); Boehringer Ingelheim Pharma GmbH & Co. KG, 55216 Ingelheim am Rhein (DE)
(72) Erfinder: BRANDL, Trixi, CH-4056 Basel (CH); MAIER, Udo, 89250 Senden (DE); HOFFMANN, Matthias, 88441 Mittelbiberach (DE); SCHEUERER, Stefan, 88447 Warthausen (DE); JOERGENSEN, Anne, T., DK-2450 Kobenhavn SV (DK); PAUTSCH, Alexander, 89075 Ulm (DE); BREITFELDER, Steffen, 88448 Attenweiler (DE); GRAUERT, Matthias, 88400 Biberach (DE); HOENKE, Christoph, 55218 Ingelheim Am Rhein (DE); ERB, Klaus, 88441 Mittelbiberach (DE); PIEPER, Michael, 88400 Biberach (DE); PRAGST, Ingo, 35041 Marburg (DE)
(74) Vertreter: Simon, Elke Anna Maria
(86) Internationale Anmeldenummer: PCT/EP2007/052912
(87) Internationale Veröffentlichungsnummer: WO 2007/115929

(56) Entgegenhaltungen:
- WO-A-01/57008
- WO-A-2005/005438
- WO-A-2005/037843
- WO-A-2006/040279
- WO-A-2006/040281
- US-A- 4 380 640

## Beschreibung

Die vorliegende Erfindung betrifft neue Thiazolyl-dihydro-quinazoline der allgemeinen Formel (I) 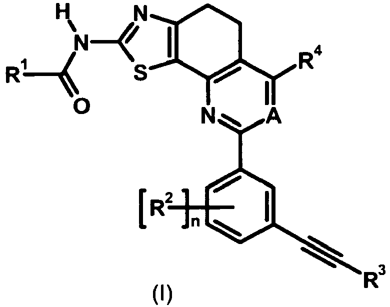 wobei X und die Reste R¹ bis R⁴ die in den Ansprüchen und der Beschreibung genannten Bedeutungen haben, deren Isomere, sowie Verfahren zur Herstellung dieser Thiazolyl-dihydro-quinazoline und deren Verwendung als Arzneimittel.

### HINTERGRUND DER ERFINDUNG

Phosphatidylinositol-3-Kinasen (PI3-Kinasen) sind eine Subfamilie der Lipidkinasen, die die Übertragung eines Phosphatrestes auf die 3'-Position des Inositolrings von Phosphoinositiden katalysieren.

Sie sind bei zahlreichen zellulären Vorgängen wie z.B. Zellwachstums- und Differenzierungsvorgängen, der Steuerung von cytoskelettalen Veränderungen und der Regulation intrazellulärer Transportvorgänge (Vanhaesebroeck et al., Annu Rev Biochem. 2001;70:535-602) beteiligt.

PI3-Kinasen können bei vielen Tumoren, wie z.B. Brustkrebs, Ovar- oder auch Pankreaskarzinom, bei Tumorarten, wie Kolon-, Mamma- oder Lungenkarzinomen, aber auch vor allem bei Autoimmunerkrankungen, wie z.B. Morbus Crohn oder rheumatoide Arthritis, oder im kardiovaskulären System wie beispielsweise bei der Entstehung der kardialen Hypertrophie (Oudit et al., Circulation. 2003 Oct 28;108(17):2147-52), eine Rolle spielen. PI3-Kinase Modulatoren können eine Möglichkeit zur anti-inflammatorischen Therapie mit vergleichsweise geringen Nebenwirkungen darstellen (Ward and Finan, Curr Opin Pharmacol. 2003 Aug;3(4):426-34).

PI3-Kinase Inhibitoren zur Behandlung entzündlicher Krankheiten sind in der Literatur bekannt. So offenbart die WO 03/072557 5-Phenylthiazolderivate, WO 04/029055 zeigt annelierte Azolpyrimidine und WO 04/007491 Azolidinone-vinyl verknüpfte Benzolderivate. Weiterhin werden durch die beiden Schriften WO 04/052373 und WO 04/056820 Benzoxazin- und Benzoxazin-3-onderivate offenbart.

Ein Thiazol-System wird in WO 2005/037843 beschrieben, die offenbarten Verbindungen sind als Inhibitoren von Proteinkinasen aktiv. Ebenso wird ein Verfahren zur Herstellung der Verbindungen bereitgestellt sowie eine pharmazeutisch Zusammensetzung umfassend die Verbindungen und Verfahren zur Verwendung der Verbindungen und Zusammensetzungen bei der Behandlung von verschiedenen Krankheiten, Zuständen oder Störungen.

In WO2005005438 wird ebenso ein Thiazol-System beschrieben, das Proteinkinasen inhibiert. Weitere Aspekte der Offenbarung beziehen sich auf pharmazeutische Zusammensetzungen und die therapeutische Verwendung zur Behandlung von proliferativen Störungen, Viruserkrankungen, ZNS-Erkrankungen, Diabetes, Schlaganfall und Herz-Kreislauf-Erkrankungen.

Aufgabe der vorliegenden Erfindung ist es neue Verbindungen bereitzustellen, die aufgrund ihrer pharmazeutischen Wirksamkeit als PI3-Kinase Modulator zur Anwendung auf therapeutischem Gebiet zur Behandlung von entzündlichen oder allergischen Erkrankungen gelangen können. Beispielhaft seien hier entzündliche und allergische Atemwegserkrankungen, entzündliche und allergische Hauterkrankungen, entzündliche Augenerkrankungen, Erkrankungen der Nasenschleimhaut, entzündliche oder allergische Krankheitszustände, bei denen Autoimmun-Reaktionen beteiligt sind oder Nierenentzündungen genannt.

### BESCHREIBUNG DER ERFINDUNG

Überraschenderweise wurde gefunden, dass die vorstehend genannte Aufgabe durch Verbindungen der Formel (I), worin die Reste R¹ bis R⁴ die nachstehend genannten Bedeutungen haben, gelöst wird.
Es wurde insbesondere gefunden, dass Verbindungen der Formel (I)als Inhibitoren von PI3-Kinase, insbesondere als Inhibitoren der PI3-Kinase gamma wirken. Somit können die erfindungsgemäßen Verbindungen beispielsweise zur Behandlung von Atemwegserkrankungen verwendet werden.

Die vorliegende Erfindung betrifft daher Verbindungen der allgemeinen Formel (I), 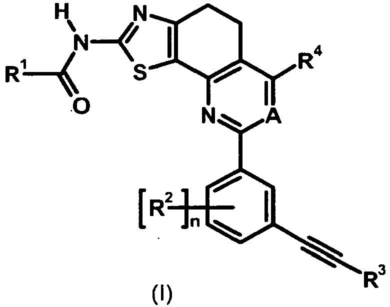 worin
- n: 1, 2, 3, 4,
- A: CH oder N,
- R¹: Wasserstoff oder einen Rest bestehend aus C₁₋₄-Alkyl, OR^{1.1} und NR^{1.1}R^{1.2};
- R^{1.1}, R^{1.2}: gleich oder verschieden, H oder C₁₋₄-Alkyl;
oder
- NR^{1.1}R^{1.2}: einen 5- bis 6- gliedrigen Heterocyclus, gegebenenfalls enthaltend ein weiteres N-Atom;
- R²: gleich oder verschieden, Wasserstoff oder einen Rest ausgewählt aus der Gruppe bestehend aus F, Cl, Br, I, CN, CF₃, CF₂H, CFH₂ und NH₂;
oder
einen Rest ausgewählt aus der Gruppe bestehend aus -O-C₁₋₄-Alkyl, C₁₋₄-Alkyl und C₂₋₆-Alkenyl;
- R⁴: Wasserstoff, OH, NH₂, oder
einen Rest ausgewählt aus der Gruppe bestehend aus C₁₋₆-Alkyl, C₂₋₆-Alkenyl, C₃₋₆-Cycloalkyl, -N(C₁₋₄-Alkyl)₂ und -NH(C₁₋₄-Alkyl);
- R³: einen Rest ausgewählt aus der Gruppe bestehend aus: 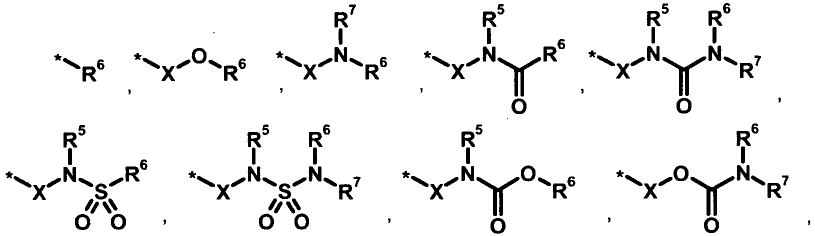 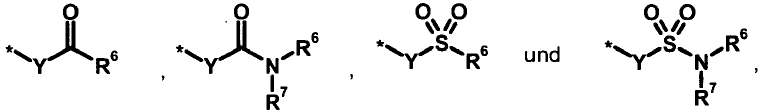 worin
X einen Rest ausgewählt aus der Gruppe bestehend aus C₁₋₆-Alkylen, C₂₋₅-Alkenylen, C₁₋₅-Alkinylen, C₃₋₇-Cycloalkylen, C₅₋₇-Cycloalkenylen und -C₁₋₄-alkylen-C₃₋₇-cycloalkylen-;
Y eine Bindung oder X;
R⁵, R⁶, R⁷ gleich oder verschieden, Wasserstoff oder einen Rest ausgewählt aus der Gruppe bestehend aus C₁₋₆-Alkyl, C₂₋₆-Alkenyl, C₂₋₆-Alkinyl, C₁₋₆-Haloalkyl, C₃₋₈-Cycloalkyl, C₃₋₈-Cycloalkenyl, C₃₋₇-Cycloalkyl-C₁₋₄-alkyl-, Aryl, Heteroaryl, Spiro, Heterocycloalkyl-, Aryl-C₁₋₆-alkyl-, Heteroaryl-C₁₋₆-alkyl- und Heterocycloalkyl-C₁₋₆-alkyl-,
oder
NR⁶R⁷ bilden einen fünf-, sechs- oder siebengliedrigen Ring bestehend aus, Kohlenstoffatomen und gegebenenfalls einem Stickstoff -,Sauerstoff- oder Schwefelatom als weitere Heteroatome oder
einen Ring ausgewählt aus der Gruppe bestehend aus: 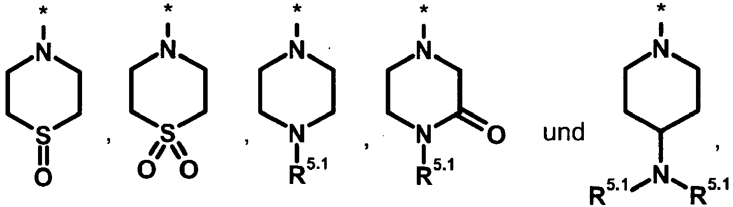 worin,
- R^{5.1}: gleich oder verschieden, Wasserstoff oder einen Rest ausgewählt aus der Gruppe bestehend aus C₁₋₆-Alkyl, C₃₋₈-Cycloalkyl, -CO-C₁₋₃-Alkyl und CONH₂;
oder
R⁵ und R⁶ bilden gemeinsam eine gesättigte oder ungesättigte Alkylen-Brücke und gegebenenfalls ein weiteres Stickstoff-, Sauerstoff- oder Schwefel-Atom enthalten kann;
oder R³ ist gleich 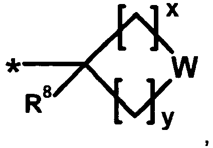 worin
- x, y: gleich oder verschieden 0, 1, 2, 3, 4 oder 5;
- W: O, NR⁹ oder CR⁹R¹⁰;
- **R⁸**: H, OR^{8.1}, NR^{8.1}R^{8.2} oder C₁₋₆-Alkyl;
R^{8.1}, R^{8.2} gleich oder verschieden, Wasserstoff, COR^{8.1.1}, CONR^{8.1.1}R^{8.1.2}, SO₂NR^{8.1.1}R^{8.1.2} oder SO₂R^{8.1.1}
oder einen Rest ausgewählt aus der Gruppe bestehend aus C₁₋₆-Alkyl, C₃₋₆-Alkenyl, C₃₋₆-Alkinyl, C₃₋₈-Cycloalkyl und C₃₋₇-Cycloalkyl-C₁₋₄-alkyl-, oder
NR^{8.1}R^{8.2} bilden zusammen einen fünf-, sechs- oder siebengliedrigen Ring der gegebenenfalls ein weiteres Heteroatom enthalten kann;
R^{8.1.1}, R^{8.1.2} gleich oder verschieden, Wasserstoff oder einen Rest ausgewählt aus der Gruppe bestehend aus C₁₋₆-Alkyl, C₃₋₈-Cycloalkyl und C₃₋₇-Cycloalkyl-C₁₋₄-alkyl-, oder
NR^{8.1.1}R^{8.1.2} bilden zusammen einen fünf-, oder sechsgliedrigen Ring, der gegebenenfalls ein weiteres Heteroatom enthalten kann;
- **R⁹, R¹⁰**: gleich oder verschieden, einen Rest, gegebenenfalls substituiert mit OMe, CN, F, Cl oder Br, ausgewählt aus der Gruppe bestehend aus C₁₋₆-Alkyl, C₂₋₆-Alkenyl, C₂₋₆-Alkinyl, C₃₋₈-Cycloalkyl, C₃₋₈-Cycloalkenyl, C₃₋₇-Cycloalkyl-C₁₋₄-alkyl-, Aryl, Heteroaryl, Spiro, Heterocycloalkyl, Aryl-C₁₋₆-alkyl- und Heteroaryl-C₁₋₆-alkyl-; oder
- R⁹, R¹⁰: gleich oder verschieden, Wasserstoff, COR^{9.1}, CONR^{9.1}R^{9.2}, SO₂R^{9.1} oder SO₂NR^{9.1}R^{9.2};
R^{9.1}, R^{9.2} gleich oder verschieden, Wasserstoff oder einen Rest ausgewählt aus der Gruppe bestehend aus C₁₋₆-Alkyl, C₂₋₆-Alkenyl, C₂₋₆-Alkinyl, C₁₋₆-Haloalkyl, C₃₋₈-Cycloalkyl, C₃₋₇-Cycloalkyl-C₁₋₄-alkyl-, Aryl, Heteroaryl, Spiro, Heterocycloalkyl, Aryl-C₁₋₆-alkyl- und Heteroaryl-C₁₋₆-alkyl-; oder
NR^{9.1}R^{9.2} bilden zusammen einen fünf- oder sechsgliedrigen Ring, der gegebenenfalls ein weiteres Heteroatom enthalten kann,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, Solvate und Hydrate,
bedeuten.

Bevorzugt sind Verbindungen der Formel (IA) nach Anspruch 1, 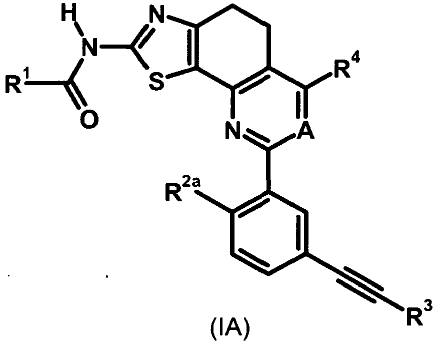 worin
- A: CH, N
- R¹, R³, und R⁴: die angegebenen Bedeutungen haben können und
- R^{2a}: einen Rest ausgewählt aus der Gruppe bestehend aus F, Cl, Br, I, CN, CF₃, CF₂H, CFH₂ und
oder
einen Rest ausgewählt aus der Gruppe bestehend aus C₁₋₄-Alkyl und C₂₋₆-Alkenyl,
bedeutet.

Weiterhin bevorzugt sind Verbindungen der Formel (I)oder (IA), worin
- R³: die angegebenen Bedeutungen haben kann und

- n: 1 oder 2,
- R¹: C₁₋₄-Alkyl oder NR^{1.1}R^{1.2};
- R^{1.1}, R^{1.2}: gleich oder verschieden, H oder C₁₋₄-Alkyl;
- R² bzw. R^{2a}: gleich oder verschieden, Wasserstoff, F oder Cl;
und
- R⁴: Wasserstoff;
bedeuten.

Weiterhin bevorzugt sind Verbindungen der Formel (I)oder (IA), worin R¹, R², R^{2a} und R⁴ die angegebenen Bedeutungen haben können und
- R³: einen Rest ausgewählt aus der Gruppe bestehend aus: 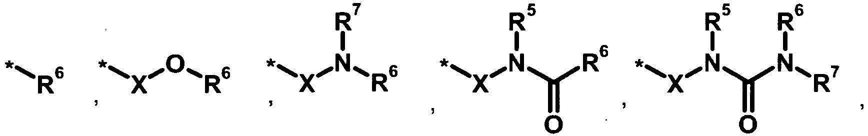 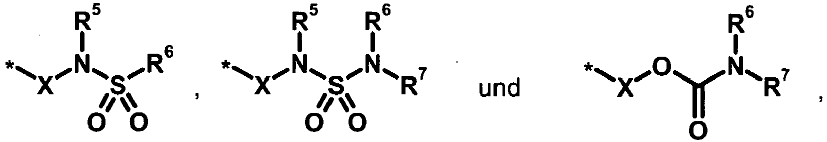 worin
X C₁₋₃-Alkylen
R⁵, R⁶, R⁷ gleich oder verschieden, Wasserstoff oder einen Rest ausgewählt aus der Gruppe bestehend aus C₁₋₆-Alkyl, C₂₋₆-Alkenyl, C₁₋₆-Haloalkyl, C₃₋₈-Cycloalkyl, C₃₋₇-Cycloalkyl-C₁₋₄-alkyl-, Aryl, Heteroaryl, Heterocycloalkyl-, Aryl-C₁₋₅-alkyl-, Heteroaryl-C₁₋₅-alkyl-, Heterocycloalkyl-C₁₋₅-alkyl- und N(C₁₋₃-Alkyl)₂-C₁₋₄-alkyl-, oder
NR⁶R⁷ bilden einen fünf- oder sechsgliedrigen Ring bestehend aus Kohlenstoffatomen und gegebenenfalls einem Stickstoff oder Sauerstoffatom als weiteres Heteroatom, oder
NR⁶R⁷ bilden einen Ring ausgewählt aus der Gruppe bestehend aus: 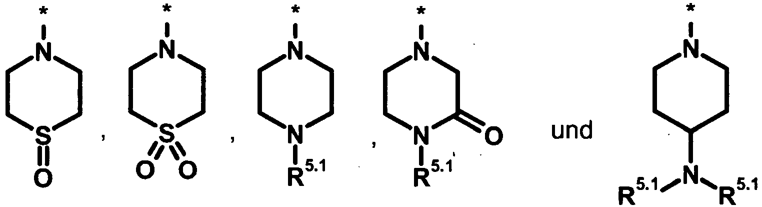
R^{5.1} gleich oder verschieden, Wasserstoff oder einen Rest ausgewählt aus der Gruppe bestehend aus C₁₋₆-Alkyl, C₃₋₈-Cycloalkyl, -CO-C₁₋₃-Alkyl und CONH₂;
bedeutet.

Weiterhin bevorzugt sind Verbindungen der Formel (I), worin
- R¹, R², und R⁴: die angegebenen Bedeutungen haben können und
- R³: einen Rest ausgewählt aus der Gruppe bestehend aus: 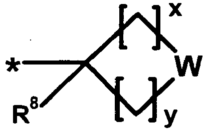
- x, y: gleich oder verschieden 0, 1, 2 oder 3
- W: NR⁹ oder CR⁹R¹⁰;
- **R⁸**: H, OR^{8.1} oder NR^{8.1}R^{8.2}
R^{8.1}, R^{8.2} gleich oder verschieden, Wasserstoff, COR^{8.1.1}, CONR^{8.1.1}R^{8.1.2}, oder C₁₋₆-Alkyl;
NR^{8.1}R^{8.2} bilden zusammen einen fünf- oder sechs- gliedrigen Ring der gegebenenfalls ein weiteres Heteroatom enthalten kann;
R^{8.1.1}, R^{8.1.2} gleich oder verschieden, Wasserstoff oder ein C₁₋₆-Alkyl,
- **R⁹, R¹⁰**: gleich oder verschieden, gegebenenfalls substituiert mit OMe, CN, F, Cl oder Br, ausgewählt aus der Gruppe bestehend aus C₁₋₆-Alkyl, C₃₋₈-Cycloalkyl, C₃₋₇-Cycloalkyl-C₁₋₄-alkyl- oder
- R⁹, R¹⁰: gleich oder verschieden, Wasserstoff, COR^{9.1}, CONR^{9.1}R^{9.2}, SO₂R^{9.1} oder SO₂NR^{9.1}R^{9.2};
- R^{9.1}, R^{9.2}: gleich oder verschieden, Wasserstoff oder einen Rest ausgewählt aus der Gruppe bestehend aus C₁₋₆-Alkyl und C₃₋₈-Cycloalkyl,
oder
NR^{9.1}R^{9.2} bilden zusammen einen fünf- oder sechsgliedrigen Ring, der gegebenenfalls Sauerstoff als ein weiteres Heteroatom enthalten kann,
bedeuten.

Ein weiterer Gegenstand der Erfindung sind Verbindungen der Formel (I) zur Verwendung als Arzneimittel.

Ein weiterer Gegenstand der Erfindung ist die Verwendung der Verbindungen der Formel (I) zur Herstellung eines Arzneimittels zur Behandlung von Erkrankungen bei deren Pathologie eine Aktivität von PI3-Kinasen beteiligt ist, in denen therapeutisch wirksame Dosen der Verbindungen der Formel (I) einen therapeutischen Nutzen entfalten können. Ein weiterer Gegenstand der Erfindung ist die Verwendung der Verbindungen der Formel (I), zur Herstellung eines Arzneimittels zur Behandlung von entzündlichen und allergischen Erkrankungen der Atemwege.

Ein weiterer Gegenstand der Erfindung ist die Verwendung der Verbindungen der Formel (I), zur Herstellung eines Arzneimittels zur Behandlung einer Erkrankung, die ausgewählt ist aus der Gruppe bestehend aus Chronischer Bronchitis, akuter Bronchitis, Bronchitis aufgrund bakterieller oder viraler Infektion oder Pilzen oder Helminthen, allergischer Bronchitis, toxischer Bronchitis, chronisch obstruktiver Bronchitis (COPD), Asthma (intrinsisch oder allergisch), pediatrischem Asthma, Bronchiektasien, allergischer Alveolitis, allergischer oder nicht-allergischer Rhinitis, chronischer Sinusitis, zystischer Fibrose oder Mukoviszidose, alpha-1-Antitrypsin-Mangel, Husten, Lungenemphysem, interstitieller Lungenerkrankungen, Alveolitis, hyperreaktiver Atemwege, Nasenpolypen, Lungenödemen, Pneumonitis aufgrund unterschiedlicher Genese wie Strahlen-induziert oder durch Aspiration oder infektiöse, Kollagenosen wie Lupus erythematodes, systemische Sklerodermie, Sarkoidose und M. Boeck.

Ein weiterer Gegenstand der Erfindung ist die Verwendung der Verbindungen der Formel (I), zur Herstellung eines Arzneimittels zur Behandlung entzündlicher und allergischer Erkrankungen der Haut.

Ein weiterer Gegenstand der Erfindung ist die Verwendung der Verbindungen der Formel (I), zur Herstellung eines Arzneimittels zur Behandlung einer Erkrankung, die ausgewählt ist aus der Gruppe bestehend aus Psoriasis, Kontakt-Dermatitis, atopische Dermatitis, Alopecia areata (kreisrunder Haarausfall), Erythema exsudativum multiforme (Stevens-Johnson-Syndrom), Dermatitis herpetiformis, Sklerodermie, Vitiligo, Nesselsucht (Urticaria), Lupus erythematodes, follikuläre und flächenhafte Pyodermien, endogene und exogene Akne, Akne rosacea sowie andere entzündliche und allergische oder proliferative Hauterkrankungen.

Ein weiterer Gegenstand der Erfindung ist die Verwendung der Verbindungen der Formel (I), zur Herstellung eines Arzneimittels zur Behandlung von Entzündungen am Auge.

Ein weiterer Gegenstand der Erfindung ist die Verwendung der Verbindungen der Formel (I), zur Herstellung eines Arzneimittels zur Behandlung einer Erkrankung, die ausgewählt ist aus der Gruppe bestehend aus Bindehautentzündung (Konjunktivitis) verschiedener Arten, wie z.B. durch Infektionen mit Pilzen oder Bakterien, allergische Konjunktivitis, Reizkonjunktivitis, durch Medikamenten induzierten Konjunktivitis, Keratitis und Uveitis. Ein weiterer Gegenstand der Erfindung ist die Verwendung der Verbindungen der Formel (I), zur Herstellung eines Arzneimittels zur Behandlung von Krankheiten der Nasenschleimhaut.

Ein weiterer Gegenstand der Erfindung ist die Verwendung der Verbindungen der Formel (I), zur Herstellung eines Arzneimittels zur Behandlung einer Erkrankung, die ausgewählt ist aus der Gruppe bestehend aus allergischer Rhinitis, allergischer Sinusitis und Nasenpolypen.

Ein weiterer Gegenstand der Erfindung ist die Verwendung der Verbindungen der Formel (I), zur Herstellung eines Arzneimittels zur Behandlung entzündlicher oder allergischer Krankheitszustände, bei denen Autoimmun-Reaktionen beteiligt sind.

Ein weiterer Gegenstand der Erfindung ist die Verwendung der Verbindungen der Formel (I), zur Herstellung eines Arzneimittels zur Behandlung einer Erkrankung, die ausgewählt ist aus der Gruppe bestehend aus Morbus Crohn, Colitis ulzerosa, systemische Lupus erythematodes, chronische Hepatitis, Multiple Sklerose, rheumatoide Arthritis, psoriatrische Arthritis, Osteoarthritis, rheumatoide Spondylitis.

Ein weiterer Gegenstand der Erfindung ist die Verwendung der Verbindungen der Formel (I), zur Herstellung eines Arzneimittels zur Behandlung von Nierenentzündungen.

Ein weiterer Gegenstand der Erfindung ist die Verwendung der Verbindungen der Formel (I), zur Herstellung eines Arzneimittels zur Behandlung einer Erkrankung, die ausgewählt ist aus der Gruppe bestehend aus Glomerulonephritis, interstitielle Nephritis und idiopathisches nephrotisches Syndrom.

Erfindungsgemäß von besonderer Bedeutung ist eine pharmazeutische Formulierung enthaltend eine Verbindung der Formel (I).

Bevorzugt ist eine oral applizierbare pharmazeutische Formulierung enthaltend eine Verbindung der Formel (I).

Ein weiterer Gegenstand der Erfindung ist ein Verfahren zur Herstellung von Verbindungen der allgemeinen Formel (I), 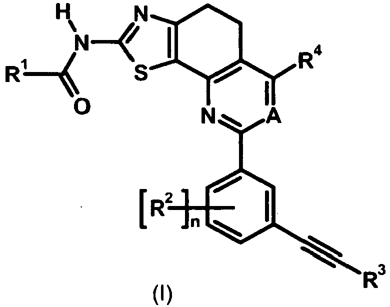 worin
A, R¹ bis R⁴ die angegebenen Bedeutungen haben können,
dadurch gekennzeichnet, dass
(a) eine Verbindung der Formel (II) 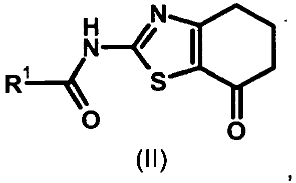 worin R¹ die angegebene Bedeutung hat,
   mit einer Verbindung der Formel 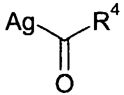 worin R⁴ die angegebenen Bedeutung hat und Ag eine Abgangsgruppe bedeutet, umgesetzt wird, und
(b) die aus Schritt (a) resultierende Verbindung der allgemeinen Formel (III) 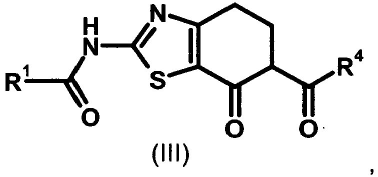 worin R¹ und R⁴ die angegebene Bedeutung haben,
   mit einer Verbindung der allgemeinen Formel 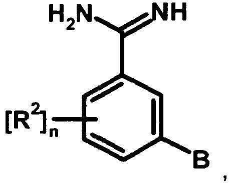 worin R² und n die angegebenen Bedeutungen haben und B eine Abgangsgruppe bedeutet, umgesetzt wird, und
(c) die aus Schritt (b) resultierende Verbindung der allgemeinen Formel (IV) 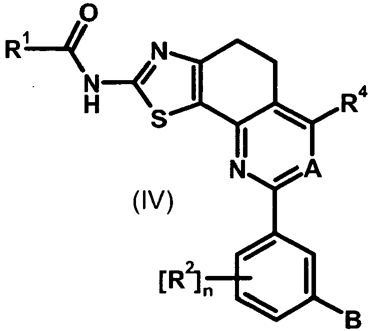 worin R¹, R², R⁴ und n die angegebenen Bedeutungen haben und B eine Abgangsgruppe bedeutet,
   mit einer Verbindung der allgemeinen Formel 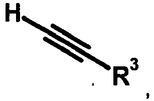 worin R³ die angegebenen Bedeutung hat,
   umgesetzt wird.

Ein weiterer Gegenstand der Erfindung sind Verbindungen gemäß der allgemeinen Formel (II), 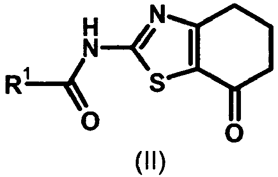 worin
R¹ die angegebenenBedeutungen hat,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Ein weiterer Gegenstand der Erfindung sind Verbindungen gemäß der allgemeinen Formel (III), 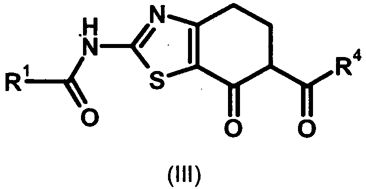 worin
R¹ und R⁴ die angegebenen Bedeutungen haben,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

Ein weiterer Gegenstand der Erfindung sind Verbindungen gemäß der allgemeinen Formel (IV), 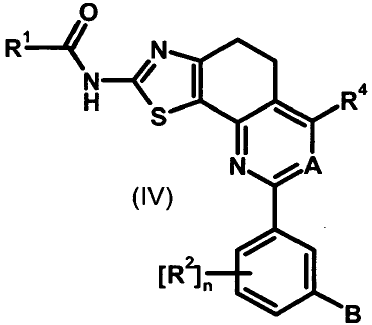 worin
worin R¹, R², R⁴ und die angegebenen Bedeutungen haben und B eine Abgangsgruppe bedeutet,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze.

### VERWENDETE BEGRIFFE UND DEFINITIONEN

Als Alkylgruppen sowie Alkylgruppen, welche Bestandteil anderer Reste sind, werden verzweigte und unverzweigte Alkylgruppen mit 1 bis 10 Kohlenstoffatomen, bevorzugt 1 - 6, besonders bevorzugt 1-4 Kohlenstoffatomen bezeichnet, beispielsweise werden genannt: Methyl, Ethyl, Propyl, Butyl, Pentyl, Hexyl, Heptyl, Octyl, Nonyl und Decyl. Sofern nicht anders genannt, sind von den vorstehend genannten Bezeichnungen Propyl, Butyl, Pentyl, Hexyl, Heptyl, Octyl, Nonyl und Decyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl, die Bezeichnung Pentyl, iso-Pentyl, Neopentyl etc.

In den vorstehend genannten Alkylgruppen können gegebenenfalls ein oder mehrere Wasserstoffatome durch andere Reste ersetzt sein. Beispielsweise können diese Alkylgruppen durch die Halogenatome Fluor, Chlor, Brom oder Iod substituiert sein. Bevorzugt sind die Substituenten Fluor und Chlor. Besonders bevorzugt ist der Substituent Chlor. Es können gegebenenfalls auch alle Wasserstoffatome der Alkylgruppe ersetzt sein.

Als Alkylbrücke werden, soweit nicht anders angegeben, verzweigte und unverzweigte Alkylgruppen mit 4 bis 7 Kohlenstoffatomen, beispielsweise, n-Butyl, iso-Butyl, sec. Butyl und tert.-Butyl, Pentyl, iso-Pentyl, Neopentyl, etc. Brücken bezeichnet. Besonders bevorzugt sind n-Butyl- oder n-Pentyl-Brücken. In den genannten Alkylbrücken können gegebenenfalls 1 bis 2 C-Atome durch ein oder mehrere Hetaroatome ausgewählt aus der Gruppe Sauerstoff oder Schwefel, vorzugsweise Sauerstoff oder Schwefel ersetzt sein.

Unter dem Begriff "C₁₋₆-Alkylen" (auch soweit sie Bestandteil anderer Reste sind) werden verzweigte und unverzweigte Alkylengruppen mit 1 bis 6 Kohlenstoffatomen verstanden und unter dem Begriff "C₁₋₄-Alkylen" verzweigte und unverzweigte Alkylengruppen mit 1 bis 4 Kohlenstoffatomen verstanden. Bevorzugt sind Alkylengruppen mit 1 bis 4 Kohlenstoffatome. Beispielsweise werden hierfür genannt: Methylen, Ethylen, Propylen, 1-Methylethylen, Butylen, 1-Methylpropylen, 1,1-Dimethylethylen, 1,2-Dimethylethylen, Pentylen, 1,1-Dimethylpropylen, 2,2,-Dimethylpropylen, 1,2-Dimethylpropylen, 1,3-Dimethylpropylen oder Hexylen. Sofern nicht anders beschrieben, umfassen die Definitionen Propylen, Butylen, Pentylen und Hexylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1,1-Dimethylethylen, 1,2-Dimethylethylen.

Als Alkenylgruppen (auch soweit sie Bestandteil anderer Reste sind) werden verzweigte und unverzweigte Alkylengruppen mit 2 bis 10 Kohlenstoffatomen, bevorzugt 2 - 6 Kohlenstoffatomen, besonders bevorzugt 2 - 3 Kohlenstoffatomen betrachtet, soweit sie mindestens eine Doppelbindung aufweisen. Beispielsweise werden genannt: Ethenyl, Propenyl, Butenyl, Pentenyl etc. Sofern nicht anders genannt, sind von den vorstehend genannten Bezeichnungen Propenyl, Butenyl etc. sämtliche der möglichen isomeren Formen umfaßt. Beispielsweise umfaßt die Bezeichnung Butylen n-Butenyl, 1-Methylpropenyl, 2-Methylpropenyl, 1.1-Dimethylethenyl, 1.2-Dimethylethenyl etc.

In den vorstehend genannten Alkenylgruppen können, soweit nicht anders beschrieben, gegebenenfalls ein oder mehrere Wasserstoffatome durch andere Reste ersetzt sein. Beispielsweise können diese Alkylgruppen durch die Halogenatome Fluor, Chlor, Brom oder Iod substituiert sein. Bevorzugt sind die Substituenten Fluor und Chlor. Besonders bevorzugt ist der Substituent Chlor. Es können gegebenenfalls auch alle Wasserstoffatome der Alkenylgruppe ersetzt sein.

Unter dem Begriff "C₂₋₆-Alkenylen" (auch soweit sie Bestandteil anderer Reste sind) werden verzweigte und unverzweigte Alkenylengruppen mit 2 bis 6 Kohlenstoffatomen verstanden und unter dem Begriff "C₂₋₄-Alkenylen" verzweigte und unverzweigte Alkylengruppen mit 2 bis 4 Kohlenstoffatomen verstanden. Bevorzugt sind Alkenylengruppen mit 2 bis 4 Kohlenstoffatome. Beispielsweise werden hierfür genannt: Ethenylen, Propenylen, 1-Methylethenylen, Butenylen, 1-Methylpropenylen, 1,1-Dimethylethenylen, 1,2-Dimethylethenylen, Pentenylen, 1,1-Dimethylpropenylen, 2,2,-Dimethylpropenylen, 1,2-Dimethylpropenylen, 1,3-Dimethylpropenylen oder Hexenylen. Sofern nicht anders beschrieben, umfassen die Definitionen Propenylen, Butenylen, Pentenylen und Hexenylen alle denkbaren isomeren Formen der jeweiligen Reste gleicher Kohlenstoffanzahl. So umfasst beispielsweise Propenyl auch 1-Methylethenylen und Butenylen umfasst 1-Methylpropenylen, 1,1-Dimethylethenylen, 1,2-Dimethylethenylen.

Als Alkinylgruppen (auch soweit sie Bestandteil anderer Reste sind) werden verzweigte und unverzweigte Alkinylgruppen mit 2 bis 10 Kohlenstoffatomen bezeichnet, soweit sie mindestens eine Dreifachbindung aufweisen, beispielsweise Ethinyl, Propargyl, Butinyl, Pentinyl, Hexinyl etc., vorzugsweise Ethinyl oder Propinyl.

Bevorzugt sind Alkinylgruppen mit 2 bis 4 Kohlenstoffatome. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc.

In den vorstehend genannten Alkinylgruppen können, soweit nicht anders beschrieben, gegebenenfalls ein oder mehrere Wasserstoffatome durch andere Reste ersetzt sein. Beispielsweise können diese Alkylgruppen durch die Halogenatome Fluor, Chlor, Brom oder Iod substituiert sein. Bevorzugt sind die Substituenten Fluor und Chlor. Besonders bevorzugt ist der Substituent Chlor. Es können gegebenenfalls auch alle Wasserstoffatome der Alkinylgruppe ersetzt sein.

Unter dem Begriff "C₂₋₆-Alkinylen" (auch soweit sie Bestandteil anderer Reste sind) werden verzweigte und unverzweigte Alkinylengruppen mit 2 bis 6 Kohlenstoffatomen verstanden und unter dem Begriff "C₂₋₄-Alkinylen" verzweigte und unverzweigte Alkylengruppen mit 2 bis 4 Kohlenstoffatomen verstanden. Bevorzugt sind Alkinylengruppen mit 2 bis 4 Kohlenstoffatomen. Beispielsweise werden hierfür genannt: Ethinylen, Propinylen, 1-Methylethinylen, Butinylen, 1-Methylpropinylen, 1,1-Dimethylethinylen, 1,2-Dimethylethinylen, Pentinylen, 1,1-Dimethylpropinylen, 2,2,-Dimethylpropinylen, 1,2-Dimethylpropinylen, 1,3-Dimethylpropinylen oder Hexinylen. Sofern nicht anders beschrieben, umfassen die Definitionen Propinylen, Butinylen, Pentinylen und Hexinylen alle denkbaren isomeren Formen der jeweiligen Reste gleicher Kohlenstoffanzahl. So umfasst beispielsweise Propinyl auch 1-Methylethinylen und Butinylen umfasst 1-Methylpropinylen, 1,1-Dimethylethinylen, 1,2-Dimethylethinylen.

Als Cycloalkylreste (auch soweit sie Bestandteil anderer Reste sind) werden gesättigte Cycloalkylreste mit 3 - 8 Kohlenstoffatomen beispielsweise Cyclopropyl, Cyclobutyl, Cyclopentyl, Cyclopentenyl, Cyclohexyl, Cyclohexenyl, Cycloheptyl oder Cyxclooctyl, vorzugsweise Cyclopropyl, Cyclopentyl oder Cyclohexyl bezeichnet, wobei jeder der vorstehend genannten Cycloalkylreste gegebenenfalls einen oder mehrere Substituenten tragen oder an einen Benzolring anneliert sein kann. Ferner können die Cycloalkylreste neben monocyclischen auch bicyclische, überbrückte oder spirocyclische Ringsysteme bilden.

Als Cycloalkenyl (auch soweit sie Bestandteil anderer Reste sind) werden cyclische Alkylgruppen mit 5 bis 8, vorzugsweise 5 oder 6 Kohlenstoffatomen verstanden, die eine oder zwei Doppelbindungen enthalten. Beispielsweise werden hierfür genannt: Cyclopentenyl, Cyclopentadienyl, Cyclohexenyl, Cyclohexadienyl, Cycloheptenyl, Cycloheptadienyl, Cyclooctenyl oder Cyclooctadienyl. Ferner können die Cycloalkenylreste neben monocyclischen auch bicyclische, überbrückte oder spirocyclische Ringsysteme bilden.

Als Haloalkyl (auch soweit sie Bestandteil anderer Reste sind) werden verzweigte und unverzweigte Alkylgruppen mit 1 bis 6 Kohlenstoffatomen bezeichnet, bei denen ein oder mehrere Wasserstoffatome durch ein Halogenatom ausgewählt aus der Gruppe Fluor, Chlor oder Brom, bevorzugt Fluor und Chlor, besonders bevorzugt Fluor ausgetauscht sind. Unter dem Begriff "C₁₋₄-Haloalkyl" werden entsprechend verzweigte und unverzweigte Alkylgruppen mit 1 bis 4 Kohlenstoffatomen verstanden, bei denen analog oben beschrieben ein oder mehrere Wasserstoffatome ausgetauscht sind. Bevorzugt ist C₁₋₄-Haloalkyl. Beispielsweise werden hierfür genannt: CH₂F, CHF₂, CF₃.

Der Begriff Aryl steht für ein aromatisches Ringsystem mit 6 bis 14 Kohlenstoffatomen, vorzugsweise 6 oder 10 Kohlenstoffatomen, bevorzugt Phenyl, das, soweit nicht anders beschrieben, beispielsweise einen oder mehrere Substituenten tragen kann.

Als Heterocycloalkylreste werden, soweit in den Definitionen nicht anders beschrieben, 5-,6- oder 7-gliedrige, gesättigte oder ungesättigte, überbrückte, mono- oder bicyclische Heterocyclen, die als Heteroatome Stickstoff, Sauerstoff oder Schwefel enthalten können, beispielsweise Tetrahydrofuran, Tetrahydrofuranon, γ-Butylrolacton, α-Pyran, γ-Pyran, Dioxolan, Tetrahydropyran, Dioxan, Dihydrothiophen, Thiolan, Dithiolan, Pyrrolin, Pyrrolidin, Pyrazolin, Pyrazolidin, Imidazolin, Imidazolidin, Tetrazol, Piperidin, Pyridazin, Pyrimidin, Pyrazin, Piperazin, Triazin, Tetrazin, Morpholin, Thiomorpholin, Diazepan, Oxazin, Tetrahydro-oxazinyl, Isothiazol, Pyrazolidin genannt, vorzugsweise Pyrazolyl, Pyrrolidinyl, Piperidinyl, Piperazinyl oder Tetrahydro-oxazinyl, genannt, wobei der Heterocyclus gegebenenfalls substituiert sein kann. Dabei kann der Ring über ein Kohlenstoffatom oder falls vorhanden über ein Stickstoffatom mit dem Molekül verknüpft sein.

Soweit nicht anders erwähnt, kann ein heterocyclischer Ring mit einer Ketogruppe versehen sein. Als Beispiel hierfür werden genannt.

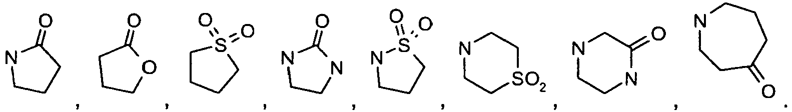

Als Beispiel für 5-1 0-gliedrige bicyclische Heteroringe werden genannt Pyrrolizin, Indol, Indolizin, Isoindol, Indazol, Purin, Chinolin, Isochinolin, Benzimdiazol, Benzofuran, Benzopyran, Benzothiazol, Benzothiazol, Benzoisothiazol, Pyridopyrimidin, Pteridin, Pyrimidopyrimidin,

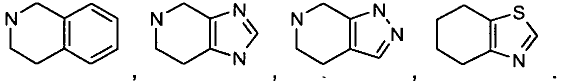

Als Heteroaryl werden 5-10-gliedrige mono- oder bicyclische Heteroarylringe in denen bis zu drei C-Atome durch ein oder meherere Hetaroatome ausgewählt aus der Gruppe Sauerstoff, Stickstoff oder Schwefel ersetzt sein können bezeichnet, wobei diese so viele konjugierte Doppelbindungen enthalten, dass ein aromatisches System gebildet wird. Jeder der vorstehend genannten Heterocyclen kann gegebenenfalls ferner an einen Benzolring anneliert sein, vorzugsweise benzimidazol. Die Heteroarylringe können, soweit nicht anders beschrieben, beispielsweise einen oder mehrere Substituenten tragen.

Der Ring kann über ein Kohlenstoffatom oder falls vorhanden über ein Stickstoffatom mit dem Molekül verknüpft sein. Als Beispiele für fünf- oder sechsgliedrige heterocyclische Aromaten, werden genannt:

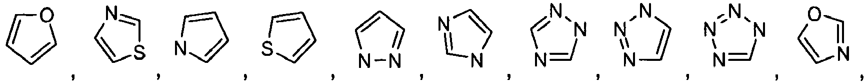

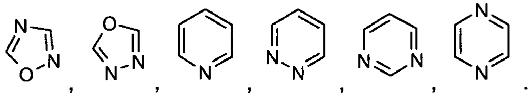

Als Beispiel für 5-10-gliedrige bicyclische Heteroarylringe werden genannt Pyrrolizin, Indol, Indolizin, Isoindol, Indazol, Purin, Chinolin, Isochinolin, Benzimdiazol, Benzofuran, Benzopyran, Benzothiazol, Benzothiazol, Benzoisothiazol, Pyridopyrimidin, Pteridin, Pyrimidopyrimidin.

Unter dem Begriff heterocyclische Spiroringe ("Spiro") werden 5-10 gliedrige, spirocyclische Ringe verstanden die gegebenenfalls ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Soweit nicht anders erwähnt, kann ein spirocyclischer Ring mit einer Ketogruppe versehen sein. Als Beispiele hierfür werden genannt:

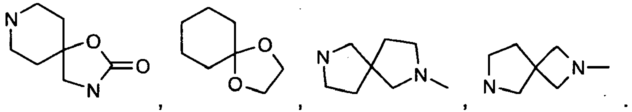

Unter dem Begriff "gegebenenfalls substituiert" wird im Rahmen der Erfindung die genannte Gruppe verstanden, die gegebenenfalls mit einem niedermolekularen Rest substituiert ist. Als niedermolekulare Reste werden als chemisch sinnvoll anzusehende Gruppen verstanden, bestehende aus 1-200 Atomen. Bevorzugt haben solche Gruppen keinen negativen Effekt auf die pharmakologische Wirksamkeit der Verbindungen. Beispielsweise können die Gruppen umfassen:
● Gerade oder verzweigte Kohlenstoffketten, gegebenenfalls unterbrochen durch Heteroatome, gegebenenfalls substituiert mit Ringen, Heteroatomen oder anderen gängigen funktionellen Gruppen.
● Aromatische oder nicht-aromatische Ringsysteme bestehend aus Kohlenstoffatomen und gegebenenfalls Heteroatomen, die wiederum substituiert sein können mit funktionellen Gruppen.
● Mehrere aromatische oder nicht-aromatische Ringsysteme bestehend aus Kohlenstoffatomen und gegebenenfalls Heteroatomen, die durch eine oder mehrere Kohlenstoffketten, gegebenenfalls unterbrochen durch Heteroatome, gegebenenfalls substituiert mit Heteroatomen oder anderen gängigen funktionellen Gruppen verknüpft sein können.

Als Halogen wird im allgemeinen Fluor, Chlor, Brom oder Jod bezeichnet.

Die erfindungsgemäßen Verbindungen können in Form der einzelnen optischen Isomeren, Mischungen der einzelnen Enantiomeren, Diastereomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren, beispielsweise Chlor- oder Bromwasserstoffsäure, oder organische Säuren, wie beispielsweise Oxal-, Fumar-, Diglycol- oder Methansulfonsäure, vorliegen.

A kann N oder CH, vorzugsweise N, bedeuten.

Der Substituent R¹ kann einen Rest ausgewählt aus der Gruppe bestehend aus Wasserstoff oder einen Rest bestehend aus C₁₋₄-Alkyl, OR^{1.1} und NR^{1.1}R^{1.2}; vorzugsweise C₁₋₄-Alkyl und NR^{1.1}R^{1.2} bedeuten. Besonders bevorzugt bedeutet der Substituent R¹ Methyl oder -NH-CH₃, insbesondere bevorzugt Methyl.

Die Substiuenten R^{1.1}, R^{1.2} können gleich oder verschieden, H oder C₁₋₄-Alkyl, vorzugsweise H oder Methyl, bedeuten.

NR^{1.1}R^{1.2} kann auch einen 5- bis 6- gliedrigen Heterocyclus, gegebenenfalls enthaltend ein weiteres N-Atom, bedeuten.

Der Substituent R² kann gleich oder verschieden, Wasserstoff oder
einen Rest ausgewählt aus der Gruppe bestehend aus F, Cl, Br, I, CN, CF₃, CF₂H, CFH₂ und vorzugsweise F, Cl und Wasserstoff,
oder
einen Rest ausgewählt aus der Gruppe bestehend aus C₁₋₄-Alkyl und C₂₋₆-Alkenyl, bedeuten.

Der Substituent R^{2a} kann einen Rest ausgewählt aus der Gruppe bestehend aus F, Cl, Br, I, CN, CF₃, CF₂H, CFH₂ und NH₂, vorzugsweise Wasserstoff, F oder Cl,
oder
einen Rest ausgewählt aus der Gruppe bestehend aus -O-C₁₋₄-Alkyl, C₁₋₄-Alkyl und C₂₋₆-Alkenyl bedeuten.

Der Substituent R^{2b} kann einen Rest ausgewählt aus der Gruppe bestehend aus F, Cl, Br, I, CN, CF₃, CF₂H, CFH₂ und NH₂, vorzugsweise Wasserstoff, F oder Cl,
oder
einen Rest ausgewählt aus der Gruppe bestehend aus -O-C₁₋₄-Alkyl, C₁₋₄-Alkyl und C₂₋₆-Alkenyl bedeuten.

Der Substituent R³ kann einen Rest ausgewählt aus der Gruppe bestehend aus: 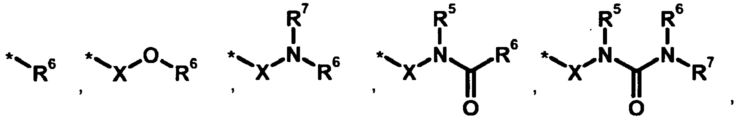 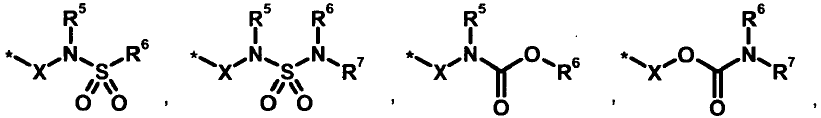 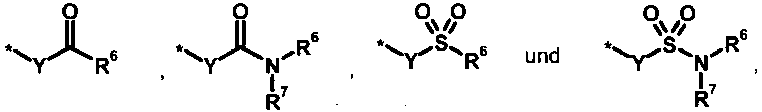 worin
- X: einen Rest ausgewählt aus der Gruppe bestehend aus C₁₋₆-Alkylen, C₂₋₅-Alkenylen, C₁₋₅-Alkinylen, C₃₋₇-Cycloalkylen, C₅₋₇-Cycloalkenylen und -C₁₋₄-alkylen-C₃₋₇-cycloalkylen-, vorzugsweise C₁₋₃-Alkylen,
- Y: eine Bindung oder X,
bedeuten.
- R³: kann bevorzugt einen Rest ausgewählt aus der Gruppe bestehend aus 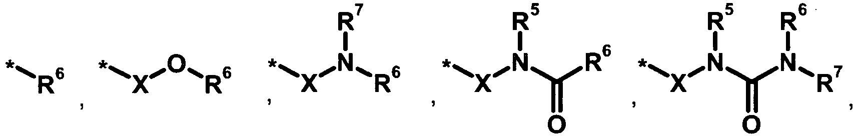 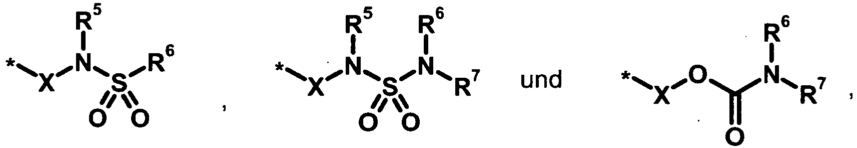
worin
- X: einen Rest vorzugsweise unsubstituiert, ausgewählt aus der Gruppe bestehend aus C₁₋₆-Alkylen, C₂₋₅-Alkenylen, C₁₋₅-Alkinylen, C₃₋₇-Cycloalkylen, C₅₋₇-Cycloalkenylen und -C₁₋₄-alkylen-C₃₋₇-cycloalkylen-, vorzugsweise C₁₋₃-Alkylen,
bedeuten.

Der Substituent R³ ist besonders bevorzugt ein Rest 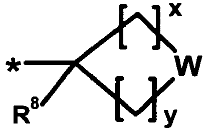 worin
x, y gleich oder verschieden 0, 1, 2, 3, 4 oder 5; vorzugsweise x gleich 0,1 oder 2, besonders bevorzugt 2, und y gleich 2 oder 3, bevorzugt 2, bedeutet.
W kann O, NR⁹ oder CR⁹R¹⁰; vorzugsweise NR⁹ oder CR⁹R¹⁰, bedeuten.

Der Substituent R⁴ kann Wasserstoff, OH, NH₂, oder
einen Rest ausgewählt aus der Gruppe bestehend aus C₁₋₆-Alkyl, C₂₋₆-Alkenyl, C₃₋₆-Cycloalkyl, -N(C₁₋₄-Alkyl)₂ und -NH(C₁₋₄-Alkyl).

Bevorzugt bedeutet der Substituent R⁴ Wasserstoff.

Der Substituent R⁵ kann Wasserstoff oder einen Rest ausgewählt aus der Gruppe bestehend aus C₁₋₆-Alkyl, C₂₋₆-Alkenyl, C₂₋₆-Alkinyl, C₁₋₆-Haloalkyl, C₃₋₈-Cycloalkyl, C₃₋₈-Cycloalkenyl, C₃₋₇-Cycloalkyl-C₁₋₄-alkyl-, Aryl, Heteroaryl, Spiro, Heterocycloalkyl-, Aryl-C₁₋₆-alkyl-, Heteroaryl-C₁₋₆-alkyl- und Heterocycloalkyl-C₁₋₆-alkyl-, vorzugsweise C₁₋₄-Alkyl und Wasserstoff, bevorzugt Methyl und Wasserstoff, bedeuten.

Der Substituent R⁶ kann Wasserstoff oder einen Rest ausgewählt aus der Gruppe bestehend aus C₁₋₆-Alkyl, C₂₋₆-Alkenyl, C₂₋₆-Alkinyl, C₁₋₆-Haloalkyl, C₃₋₈-Cycloalkyl, C₃₋₈-Cycloalkenyl, C₃₋₇-Cycloalkyl-C₁₋₄-alkyl-, Aryl, Heteroaryl, Spiro, Heterocycloalkyl-, Aryl-C₁₋₆-alkyl-, Heteroaryl-C₁₋₆-alkyl- und Heterocycloalkyl-C₁₋₆-alkyl-, vorzugsweise Wasserstoff oder einen Rest, gegebenenfalls substituiert, ausgewählt aus der Gruppe bestehend aus C₁₋₆-Alkyl, C₁₋₆-Haloalkyl, C₃₋₈-Cycloalkyl,C₃₋₇-Cycloalkyl-C₁₋₄-alkyl-, Aryl, Heteroaryl, Heterocycloalkyl- und Aryl-C₁₋₆-alkyl-, bedeuten.

Der Substituent R⁷ kann Wasserstoff oder einen Rest ausgewählt aus der Gruppe bestehend aus C₁₋₆-Alkyl, C₂₋₆-Alkenyl, C₂₋₆-Alkinyl, C₁₋₆-Haloalkyl, C₃₋₈-Cycloalkyl, C₃₋₈-Cycloalkenyl, C₃₋₇-Cycloalkyl-C₁₋₄-alkyl-, Aryl, Heteroaryl, Spiro, Heterocycloalkyl-, Aryl-C₁₋₆-alkyl-, Heteroaryl-C₁₋₆-alkyl- und Heterocycloalkyl-C₁₋₆-alkyl-, vorzugsweise Wasserstoff oder einen Rest, gegebenenfalls substituiert, ausgewählt aus der Gruppe bestehend aus C₁₋₆-Alkyl, C₃₋₈-Cycloalkyl, C₃₋₇-Cycloalkyl-C₁₋₄-alkyl-, Heteroaryl, Heteroaryl-C₁₋₆-alkyl- und Heterocycloalkyl-C₁₋₆-alkyl, bedeuten.

NR⁶R⁷ können einen fünf-, sechs- oder siebengliedrigen Ring ,vorzugsweise einen fünf-, oder sechsgliedrigen Ring, bestehend aus, Kohlenstoffatomen und gegebenenfalls einem Stickstoff -,Sauerstoff- oder Schwefelatom, vorzugsweise einem Stickstoff oder Sauerstoffatom, als weitere Heteroatome bilden, oder

NR⁶R⁷ bilden einen Ring ausgewählt aus der Gruppe bestehend aus: 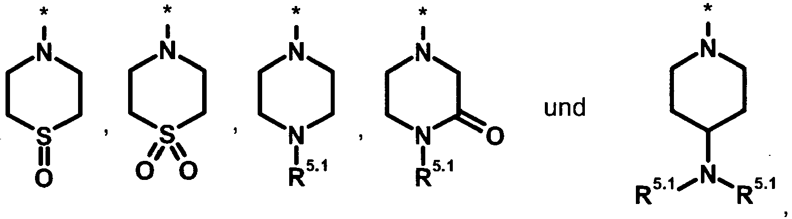 vorzugsweise aus 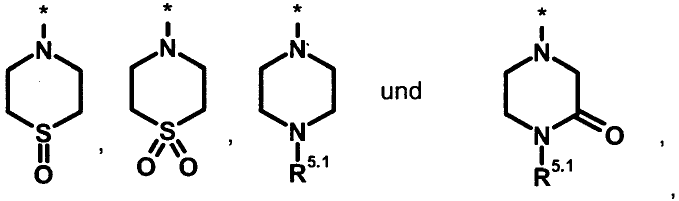 worin
R^{5.1} gleich oder verschieden, Wasserstoff oder einen Rest ausgewählt aus der Gruppe bestehend aus C₁₋₆-Alkyl, C₃₋₈-Cycloalkyl, -CO-C₁₋₃-Alkyl und CONH₂, vorzugsweise Wasserstoff oder einen Rest ausgewählt aus der Gruppe bestehend aus C₁₋₃-Alkyl und C₃₋₆-Cycloalkyl, bedeutet.

Der Substituent R⁸ kann H, OR^{8.1}, NR^{8.1}R^{8.2} oder C₁₋₆-Alkyl; vorzugsweise H, OR^{8.1} oder NR^{8.1}R^{8.2}, besonders bevorzugt NR^{8.1}R^{8.2}, bedeuten, worin
- R^{8.1}, R^{8.2}: gleich oder verschieden, Wasserstoff, COR^{8.1.1}, CONR^{8.1.1}R^{8.1.2}, SO₂NR^{8.1.1}R^{8.1.2} oder SO₂R^{8.1.1}, vorzugsweise Wasserstoff, oder einen Rest ausgewählt aus der Gruppe bestehend aus C₁₋₆-Alkyl, C₃₋₆-Alkenyl, C₃₋₆-Alkinyl, C₃₋₈-Cycloalkyl und C₃₋₇-Cycloalkyl-C₁₋₄-alkyl-, vorzugsweise Wasserstoff, COR^{8.1.1}, CONR^{8.1.1}R^{8.1.2} oder C₁₋₃-Alkyl, bedeuten können, oder
- NR^{8.1}R^{8.2}: bilden zusammen einen fünf-, sechs- oder siebengliedrigen Ring, vorzugsweise einen fünf- oder sechsgliedrigen Ring, der gegebenenfalls ein weiteres Heteroatom, enthalten kann;
- R^{8.1.1}, R^{8.1.2}: gleich oder verschieden, Wasserstoff oder einen Rest ausgewählt aus der Gruppe bestehend aus C₁₋₆-Alkyl, C₃₋₈-Cycloalkyl und C₃₋₇-Cycloalkyl-C₁₋₄-alkyl-, vorzugsweise Wasserstoff oder C₁₋₃-Alkyl oder
- NR^{8.1.1}R^{8.1.2}: bilden zusammen einen fünf-, oder sechsgliedrigen Ring, der gegebenenfalls ein weiteres Heteroatom, enthalten kann;

Der Substituent R⁹ kann einen Rest, gegebenenfalls substituiert mit OMe, CN, F, Cl oder Br, ausgewählt aus der Gruppe bestehend aus C₁₋₆-Alkyl, C₂₋₆-Alkenyl, C₂₋₆-Alkinyl, C₃₋₈-Cycloalkyl, C₃₋₈-Cycloalkenyl, C₃₋₇-Cycloalkyl-C₁₋₄-alkyl-, Aryl, Heteroaryl, Spiro, Heterocycloalkyl, Aryl-C₁₋₆-alkyl- und Heteroaryl-C₁₋₆-alkyl-; vorzugsweise C₁₋₆-Alkyl, C₃₋₆-Cycloalkyl und C₃₋₆-Cycloalkyl-C₁₋₄-alkyl, insbesondere bevorzugt C₅₋₆-Cycloalkyl oder
Wasserstoff, COR^{9.1}, CONR^{9.1}R^{9.2}, SO₂R^{9.1} oder SO₂NR^{9.1}R^{9.2} bedeuten,
worin
- R^{9.1}, R^{9.2}: gleich oder verschieden, Wasserstoff oder einen Rest ausgewählt aus der Gruppe bestehend aus C₁₋₆-Alkyl, C₂₋₆-Alkenyl, C₂₋₆-Alkinyl, C₁₋₆-Haloalkyl, C₃₋₈-Cycloalkyl,C₃₋₇-Cycloalkyl-C₁₋₄-alkyl-, Aryl, Heteroaryl, Spiro, Heterocycloalkyl, Aryl-C₁₋₆-alkyl-und Heteroaryl-C₁₋₆-alkyl-; vorzugsweise Wasserstoff oder einen Rest gegebenenfalls substituiert, ausgewählt aus der Gruppe bestehend aus C₁₋₆-Alkyl und C₃₋₆-Cycloalkyl, bedeuten;
oder
- NR^{9.1}R^{9.2}: bilden zusammen einen fünf- oder sechsgliedrigen Ring, der gegebenenfalls ein weiteres Heteroatom, enthalten kann.

Der Substituent R¹⁰ kann einen Rest, gegebenenfalls substituiert mit OMe, CN, F, Cl oder Br, ausgewählt aus der Gruppe bestehend aus C₁₋₆-Alkyl, C₂₋₆-Alkenyl, C₂₋₆-Alkinyl, C₃₋₈-Cycloalkyl, C₃₋₈-Cycloalkenyl, C₃₋₇-Cycloalkyl-C₁₋₄-alkyl-, Aryl, Heteroaryl, Spiro, Heterocycloalkyl, Aryl-C₁₋₆-alkyl- und Heteroaryl-C₁₋₆-alkyl- oder Wasserstoff, COR^{9.1}, CONR^{9.1}R^{9.2}, SO₂R^{9.1} oder SO₂NR^{9.1}R^{9.2} bedeuten,
worin
- R^{9.1}, R^{9.2}: gleich oder verschieden, Wasserstoff oder einen Rest ausgewählt aus der Gruppe bestehend aus C₁₋₆-Alkyl, C₂₋₆-Alkenyl, C₂₋₆-Alkinyl, C₁₋₆-Haloalkyl, C₃₋₈-Cycloalkyl, C₃₋₇-Cycloalkyl-C₁₋₄-alkyl-, Aryl, Heteroaryl, Spiro, Heterocycloalkyl, Aryl-C₁₋₆-alkyl- und Heteroaryl-C₁₋₆-alkyl-; bedeuten.
oder
- NR^{9.1}R^{9.2}: bilden zusammen einen fünf- oder sechsgliedrigen Ring, der gegebenenfalls ein weiteres Heteroatom enthalten kann

Besonders bevorzugt bedeutet der Rest R¹⁰ Wasserstoff.
Als Abgangsgruppe A wird eine Abgangsgruppe wie beispielsweise Chlor, O-C₁-C₃-Alkyl, Imidazolidin, vorzugsweise O-C₁-C₃-Alkyl bezeichnet.
Als Abgangsgruppe B wird eine Abgangsgruppe wie beispielsweise Chlor, Brom, Iod, Methansulfonyl, Trifluormethansulfonyl oder p-Toluolsulfonyl, vorzugsweise Iod, bezeichnet.

### HERSTELLUNGSVERFAHREN

Die Verbindungen der allgemeinen Formel (I) können nach folgendem Syntheseschema (Schema 1) hergestellt werden, wobei die Substituenten der allgemeinen Formel (I) die zuvor genannten Bedeutungen haben. Diese Verfahren sind als Erläuterung der Erfindung zu verstehen ohne selbige auf deren Gegenstand zu beschränken.

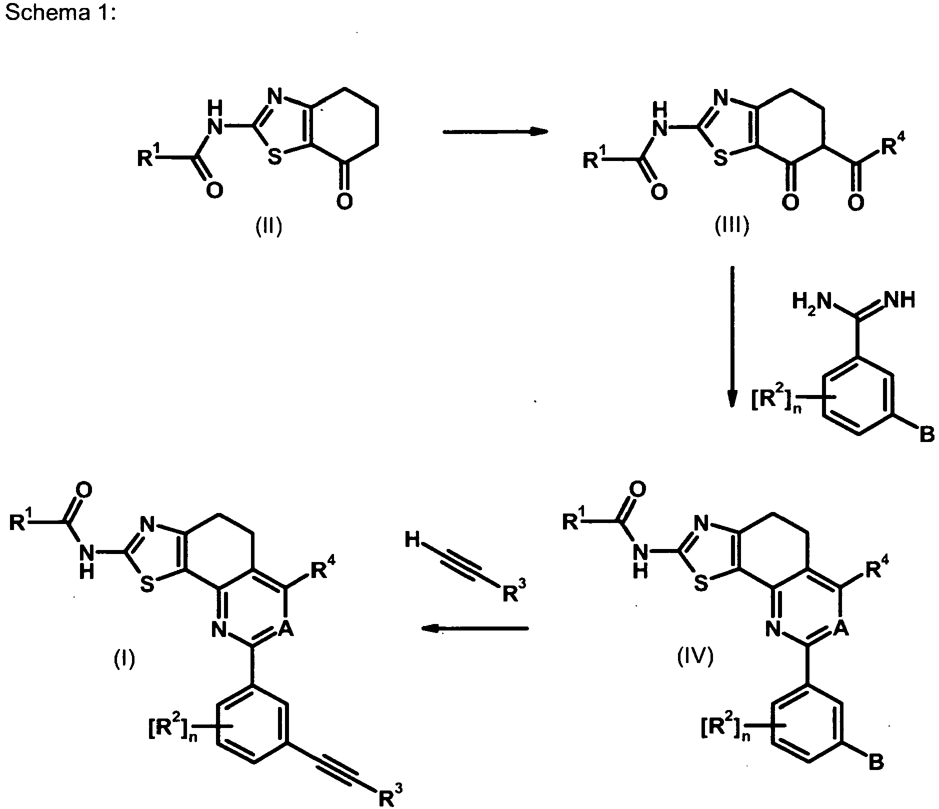

Die neuen Verbindungen der allgemeinen Formel (I) können in Analogie der nachfolgenden Beispiele dargestellt werden. Die nachstehend beschriebenen Beispiele sind als Erläuterung der Erfindung zu verstehen ohne diese jedoch einzuschränken.

### SYNTHESE DER REAGENZIEN

### 1-Cyclopentyl-4-ethynyl-piperidin

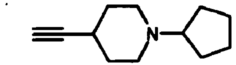

5.0 g (43.4 mmol) Piperidin-4-yl-methanol werden unter Argon-Atmosphäre in 250 mL Dichlormethan vorgelegt und mit 3,7 g (44.0 mmol) Cyclopentanon versetzt. Danach erfolgt die Zugabe von 3.6 g (44.0 mmol) Natriumacetat und 14.0 g (66.0 mmol) Natriumtriacetoxyborhydrid. Die entstandene Suspension wird 16 Stunden bei Raumtemperatur gerührt. Anschließend wird das Reaktionsgemisch mit Natriumhydrogencarbonatlösung extrahiert. Die Wasserphase wird mit Natriumchlorid gesättigt und mit Chloroform / Methanol extrahiert. Die daraus resultierende organische Phase wird getrocknet und zur Trockene eingedampft. Ausbeute: 6.0g

1.1 mL (13.0 mmol) Oxalylchlorid werden unter Stickstoff-Atmosphäre in 200 mL Dichlormethan vorgelegt und auf -78 °C gekühlt. 1.9 mL (27.3 mmol) Dimethylsulfoxid werden in wenig Dichlormethan gelöst zugetropft. Es wird 0.3 Stunden nachgerührt, und dann 2.0 g (10.9 mmol) der zuvor beschriebenen Zwischenstufe in Dichlormethan zugetropft. Das Reaktionsgemisch wird 3 Stunden gerührt, danach werden 7.9 mL (54.6 mmol) Triethylamin tropfenweise zugegeben. Die Kühlung wird entfernt und das Reaktionsgemisch auf Raumtemperatur erwärmt. Anschließend wird mit Wasser versetzt und die Phasen getrennt. Die organische Phase wird mit Natriumhydrogencarbonatlösung (50%) und Wasser gewaschen, getrocknet und zur Trockene eingedampft. Ausbeute: 1.1 g

1.1 g (6.0 mmol) der zuvor beschriebenen Zwischenstufe werden unter Argon-Atmosphäre in 50 mL Methanol gelöst und mit 0.8 g (6.0 mmol) Kaliumcarbonat versetzt. 1.2 g (6.2 mmol) (1-Diazo-2-oxo-propyl)-phosphorsäuredimethylester werden in Methanol gelöst zu dem Gemisch gegeben, dann 4 Stunden bei Raumtemperatur gerührt. Anschließend wird das Reaktionsgemisch auf 200 mL Wasser gegossen und mit Diethylether extrahiert. Die organische Phase wird getrocknet und zur Trockene eingedampft. Ausbeute: 0.9 g

Auf analogem Weg können 4-Ethynyl-1-isopropyl-piperidin und 1-Cyclopentyl-methyl-4-ethynyl-piperidin hergestellt werden.

### 4-Ethynyl-piperidin-1-carbonsäure-tert.butylester

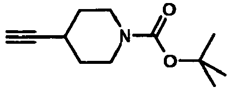

Kann analog zu 1-Cyclopentyl-4-ethynyl-piperidin ausgehend von kommerziellem 1-Boc-4-Piperidinmethanol hergestellt werden.

### 1-Cyclopentyl-4-ethynyl-piperidin-4-ol

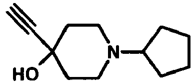

4.0 g (28.0 mmol) Piperidon-4-ethylenacetal werden in 250 mL Dichlormethan vorgelegt und mit 2.4 g (28.5 mmol) Cyclopentanon versetzt. Danach erfolgt die Zugabe von 2.3 g (28.0 mmol) Natriumacetat und 8.9 g (42 mmol) Natriumtriacetoxyborhydrid. Die entstandene Suspension wird 16 Stunden bei Raumtemperatur gerührt. Anschließend wird das Reaktionsgemisch mit Natriumhydrogencarbonatlösung extrahiert und mit Wasser gewaschen. Die organische Phase wird getrocknet und zur Trockene eingedampft. Ausbeute: 5.5 g

5.5 g (26.0 mmol) der zuvor beschriebenen Zwischenstufe werden in 10 mL Aceton vorgelegt und mit 110 mL 0.1 N wässriger Salzsäure versetzt. Das Reaktionsgemisch wird 5 Stunden unter Rückfluss gerührt, nach Abkühlen auf Raumtemperatur mit 5 N Natronlauge basisch gestellt und mit Chloroform / Dichlormethan extrahiert. Die organische Phase wird getrocknet und zur Trockene eingedampft. Ausbeute: 4.1 g

3.1 mL (18.0 mmol) Trimethylsilylacetylen werden unter Stickstoffatmosphäre bei -70 °C in 400 mL trockenem Tetrahydrofuran vorgelegt und mit 12.9 mL (22.4 mmol) n-Butyllithium (2.5 M Lösung in Hexan) versetzt. Nach einer Stunde werden 3.0 g (18.0 mmol) der zuvor beschriebenen Zwischenstufe in 100 mL Tetrahydrofuran gelöst und langsam tropfenweise zu dem Gemisch gegeben. Es wird 1 Stunde bei -70 °C und 16 Stunden bei Raumtemperatur gerührt. Anschließend wird das Reaktionsgemisch mit 300 mL gesättigter Ammoniumchloridlösung versetzt, 0.1 Stunde nachgerührt, dann auf 500 mL Wasser gegossen. Es wird mit Ethylacetat extrahiert, die vereinigten organischen Phasen mit Wasser gewaschen, getrocknet und zur Trockene eingedampft. Ausbeute: 3:0 g

3.0 g (11.0 mmol) der zuvor beschriebenen Zwischenstufe und 4.1 mL (14.0 mmol) Tetrabutylammoniumfluorid werden in Dichlormethan 1 Stunde bei Raumtemperatur gerührt. Anschließend wird das Reaktionsgemisch mit Wasser gewaschen, die organische Phase getrocknet und zur Trockene eingedampft. Ausbeute: 0.9 g

Auf analogem Weg kann 4-Ethynyl-1-isopropyl-piperidin-4-ol hergestellt werden.

### (R)-2-Ethynyl-pyrrolidin

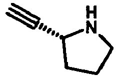

Eine Mischung aus 4.9 g (24.6 mmol) (R)-(+)-1-Boc-2-Pyrrolidincarbaldehyd und 4.0 g (29.0 mmol) Kaliumcarbonat in 40 mL Methanol wird mit 5.3 g (27.3 mmol) (1-Diazo-2-oxo-propyl)-phosphorsäuredimethylester versetzt und 4 Stunden bei Raumtemperatur gerührt. Anschließend wird das Reaktionsgemisch auf Wasser gegossen und mit Diethylether extrahiert. Die organische Phase wird getrocknet und leicht eingeengt. Der Rückstand wird mit 3 mL etherischer Salzsäure (1 M) versetzt, über Nacht bei Raumtemperatur gerührt und anschließend vollständig eingeengt. Ausbeute: 3.9 g (gelbes Öl)

Analog kann (S)-2-Ethynyl-pyrrolidin ausgehend (S)-(-)-1-Boc-2-Pyrrolidincarbaldehyd von hergestellt werden.

### 1-Ethynyl-1-methoxy-cyclohexan

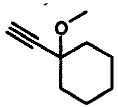

Zu einer Lösung von 2 g (16 mmol) 1-Ethynylcyclohexanol in 25 mL DMF werden bei Raumtemperatur 0.8 g (20 mmol) Natriumhydrid (60 %ig in Mineralöl) gegeben. Nach 20 Minuten werden 1.25 mL (20 mmol) Methyliodid zugegeben und für eine weitere Stunde gerührt. Die Reaktionsmischung wird mit Eis versetzt und mit Ether extrahiert. Die organische Phase wird getrocknet und eingedampft. Der verbliebene Rückstand wird mittels MPLC (Dichlormethan/Methanol 100:5) gereinigt. Ausbeute: 0.6 g (klares Öl)

Auf analogem Weg können 4-Ethynyl-4-methoxy-1-methyl-piperidin und 1-Cyclopentyl-4-ethynyl-4-methoxy-piperidin hergestellt werden.

### Ethyl-(1-ethynyl-cyclohexyl)-amin

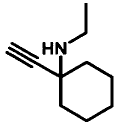

Eine Lösung bestehend aus 20 g (161 mmol) Ethynylcyclohexanol und 25 mL (177 mmol) Triethylamin und 200 mg (1.6 mmol) 4-Dimethylaminopyridin in 200 mL Dichlormethan wird bei 0 °C mit 12.6 mL (177 mmol) Acetylchlorid versetzt. Nach 5 Stunden bei 0 °C wird die Reaktionsmischung mit Wasser versetzt und mit Dichlormethan extrahiert. Die vereinten organischen Phasen werden eingedampft und der Rückstand mittels MPLC (Cyclohexan/Essigester 6:1) gereinigt. Ausbeute: 3 g (gelbes Öl)

Eine Mischung aus 0.4 g (2.4 mmol) der zuvor beschriebenen Zwischenstufe, 3.6 mL (7.2 mmol) Ethylamin (2 M Lösung in THF) und 12 mg (0.12 mmol) Kupfer(I)-chlorid in 5 mL THF wird 3.5 Stunden refluxiert. Die Reaktionsmischung wird eingedampft, in Essigsäureethylester aufgenommen und mit Ammoniumchlorid- und Natriumchlorid-Lösung gewaschen. Die organische Phase wird eingedampft. Ausbeute: 0.15 g (braunes Öl)

Analog können die folgenden Amine hergestellt werden: 1-(1-Ethynyl-cyclohexyl)-pyrrolidin; (1-Ethynyl-cyclohexyl)-dimethylamin; (1-Ethynyl-cyclohexy)-isopropylamin; 1-Ethynyl-cyclohexyl)-methylamin; (1-Ethynyl-cyclopentyl)-dimethylamin

### N-(1-Ethynyl-cyclohexyl)-acetamid

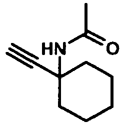

Eine Lösung von 4 g (32 mmol) 1-Ethynylcyclohexylamin in 30 mL Ether wird bei Raumtemperatur mit 1.1 mL (15 mmol) Acetylchlorid versetzt. Die farblose Suspension wird über Nacht bei Raumtemperatur gerührt, der entstandene Feststoff abgesaugt und mit Diethylether/Dichlormethan nachgewaschen. Das Filtrat wird eingeengt und liefert das Produkt als farblosen Feststoff. Ausbeute: 3 g

Analog kann N-But-3-ynyl-N-methyl-acetamid aus But-3-ynyl-methyl-amin hergestellt werden

### 1-(1-Ethynyl-cyclohexyl)-3-methylharnstoff

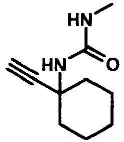

Eine Lösung von 1 g (8 mmol) 1-Ethynylcyclohexylamin und 2 mL (15 mmol) Triethylamin in 10 mL Acetonitril wird bei Raumtemperatur mit 0.5 g (9 mmol) Methylisocyanat versetzt. Die farblose Suspension wird über Nacht bei Raumtemperatur gerührt und anschließend eingedampft. Der Rückstand wird in Dichlormethan aufgenommen und mit wässriger Kaliumcarbonat-Lösung gewaschen. Die organische Phase wird getrocknet und eingeengt.
Ausbeute: 1.4 g (farbloser Feststoff)

Analog kann 1-But-3-ynyl-1,3-dimethyl-hamstoffaus But-3-ynyl-methyl-amin hergestellt werden.

### 1-Prop-2-ynyl-1H-imidazol

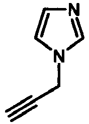

5 g (73 mmol) Imidazol und 1.3 g (4 mmol) Tetrabutylammoniumiodid werden in 200 mL Toluol und 150 mL 50%ige Natriumhydroxidlösung vorgelegt und 15.7 mL (145 mmol) Propargylbromid zugegeben. Es wird 1 Stunde bei Raumtemperatur gerührt, dann mit Toluol und Wasser verdünnt. Die organische Phase wird getrocknet und zur Trockene eingedampft. Der Rückstand wird chromatographisch gereinigt. Ausbeute: 2.5 g

### 2-Chloro-5-iodo-benzamidin

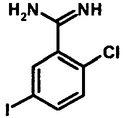

374.8 mL (374.8 mmol) Lithium bis-trimethylsilylamid (1 M in Hexan) werden in 300 mL Diethylether vorgelegt und mit 50.0 g (189.8 mmol) 2-Chloro-5-iodbenzonitril versetzt. Das Reaktionsgemisch wird 1.5 Stunden bei Raumtemperatur unter Argon-Atmosphäre gerührt und danach auf 0 °C abgekühlt. Anschließend wird langsam mit 5 molarer Salzsäure versetzt. Der dabei ausfallende Niederschlag wird abgesaugt und getrocknet. Ausbeute: 56.0 g

Analog können 3-Chlor-5-iod-benzamidin, 2-Fluor-5-iod-benzamidin sowie 3-lod-benzamidin hergestellt werden.

### SYNTHESE DER ZWISCHENVERBINDUNGEN

### N-(7-Oxo-4,5,6,7-tetrahydro-benzothiazol-2-yl)-acetamid

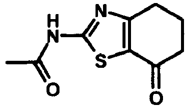

112 g (1.0 mol) 1,3-Cyclohexandion werden in 700 mL Eiswasser suspendiert und 51.6 mL (1.0 mol) Brom bei 0 °C innerhalb von 45 Minuten zugetropft. Die Suspension wird 3.5 Stunden bei max. 10 °C nachgerührt. Anschließend wird abgesaugt und der Feststoff in 800 mL Wasser ausgerührt, abgesaugt, mit 3 L Wasser gewaschen und getrocknet. Der erhaltene Feststoff wird aus Ethanol umkristallisiert. Ausbeute: 37 g (Smp.: 159 - 160 °C)

15.5 g (0.2 mol) Thioharnstoff werden bei Raumtemperatur in 200 mL Ethanol vorgelegt. Zu dieser Suspension werden 37.1 g (0.2 mol) der zuvor beschriebenen Zwischenstufe portionsweise gegeben, dann wird mit 60 mL Ethanol nachgespült. Die allmählich entstehende Lösung wird 2 Stunden unter Rückfluss gerührt und anschließend eingedampft. Der Rückstand wird mit Wasser und Diethylether extrahiert, die Wasserphase mit Natriumcarbonatlösung basisch gestellt. Der hierbei entstandene Feststoff wird abgesaugt, mit Wasser gewaschen, dann mit Methanol ausgerührt und zur Trockene eingedampft.
Ausbeute: 22 g (Smp.: 265 - 268 °C)

230 mL (2.4 mol) Essigsäureanhydrid werden bei Raumtemperatur vorgelegt, 22 g (0.13 mol) der zuvor beschriebenen Zwischenstufe zugegeben und 3 Stunden unter Rückfluss gerührt. Die Suspension geht dabei teilweise in Lösung. Nach dem Abkühlen mittels Eis/Kochsalzbad wird der Feststoff abgesaugt, 2x in je 150 mL Aceton aufgekocht, abgesaugt und getrocknet. Ausbeute: 25 g (Smp.: 268 - 272 °C)

### N-(6-Formyl-7-oxo-4,5,6,7-tetrahydro-benzothiazol-2-yl)-acetamid

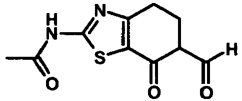

20 g (0.37 mol) Natriummethylat werden in 50 mL Dimethylformamid suspendiert, eine Suspension aus 21 g (0.1 mol) N-(7-Oxo-4,5,6,7-tetrahydro-benzothiazol-2-yl)-acetamid in 100 mL Dimethylformamid zugetropft. Es wird 15 Minuten nachgerührt, dann auf 0 °C gekühlt. Ein Gemisch aus 29.9 mL (0.37 mol) Ameisensäureethylester und 60 mL Benzol wird zugetropft und die Reaktionsmischung mit weiteren 100 mL Benzol verdünnt. Allmählich fällt ein Niederschlag aus und es wird bei 0 °C 3.5 Stunden weitergerührt. Die Suspension wird mit 370 mL 1 molarer Salzsäure hydrolysiert, der dabei ausgefallene Feststoff wird abgesaugt. Die zwei Phasen der Mutterlauge werden getrennt, die Wasserphase mit Dichlormethan extrahiert. Die daraus resultierende organische Phase wird getrocknet und zur Trockene eingedampft. Der Feststoff und der Rückstand aus der Extraktion werden aus Acetonitril umkristallisiert. Ausbeute: 20 g

### N-[8-(2-Chlor-5-iod-phenyl)-4,5-dihydrothiazolo[4,5-h]quinazolin-2-yl]-acetamid

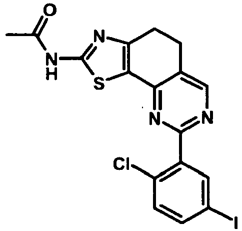

5.0 g (21.0 mmol) N-(6-Formyl-7-oxo-4,5,6,7-tetrahydro-benzothiazol-2-yl)-acetamid und 7.3 g (23.0 mmol) 2-Chlor-5-iodo-benzamidin werden in 50 mL Pyridin mehrere Stunden bei 160° C gerührt. Nach Abkühlen auf Raumtemperatur wird der ausgefallene Feststoff abgesaugt, gewaschen und getrocknet. Ausbeute: 4.7 g

Analog können ausgehend von 3-Chlor-5-iod-benzamidin, 2-Fluor-5-iod-benzamidin sowie 3-lod-benzamidin die folgenden Zwischenstufen hergestellt werden: N-[8-(3-Chlor-5-iod-phenyl)-4,5-dihydro-thiazolo[4,5-h]quinazolin-2-yl]-acetamid; N-[8-(2-Fluor-5-iod-phenyl)-4,5-dihydro-thiazolo[4,5-h]quinazolin-2-yl]-acetamid; N-[8-(3-Iod-phenyl)-4,5-dihydro-thiazolo[4,5-h]quinazolin-2-yl]-acetamid

### SYNTHESE VON VERBINDUNGEN DER FORMEL (I)

Zur Charakterisierung der Verbindungen der Formel (I)wurden folgende HPLC-MS-Methoden verwendet:

### HPLC-MS Analytik

### Methode A

Waters ZMD, Alliance 2690/2695 HPLC, Waters 2700 Autosampler, Waters 996/2996 Diodenarraydetektor

Als mobile Phase wurde eingesetzt:
A: Wasser mit 0.10% TFA
B: Acetonitril mit 0.10% TFA

| Zeit in min | %A | %B | Flussrate in ml/min |
|---|---|---|---|
| 0.0 | 95 | 5 | 1.00 |
| 0.1 | 95 | 5 | 1.00 |
| 3.1 | 2 | 98 | 1.00 |
| 4.5 | 2 | 98 | 1.00 |
| 5.0 | 95 | 5 | 1.00 |

Als stationäre Phase diente eine Säule XTerra®, MS C₁₈ 2.5 µm, 4.6 mm x 30 mm (Säulentemperatur: konstant bei 25°C).

Die Diodenarraydetektion erfolgte im Wellenlängenbereich 210-400 nm.

### Methode B

Waters ZMD, Alliance 2690/2695 HPLC, Waters 2700 Autosampler, Waters 996/2996 Diodenarraydetektor

Als mobile Phase wurde eingesetzt:
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.00 |
| 0.10 | 95 | 5 | 2.00 |
| 2.10 | 2 | 98 | 2.00 |
| 3.00 | 2 | 98 | 2.00 |
| 3.25 | 95 | 5 | 2.00 |

Als stationäre Phase diente eine Säule Merck Chromolith™ SpeedROD RP-18e, 4.6 mm x 50 mm (Säulentemperatur: konstant bei 25°C).

Die Diodenarraydetektion erfolgte im Wellenlängenbereich 210-400 nm.

### BEISPIELE

### Beispiel 1:

### N-{8-[2-Chlor-5-(3-methylamino-prop-1-ynyl)-phenyl]-4,5-dihydro-thiazolo[4,5 h]quinazolin-2-yl}-acetamid

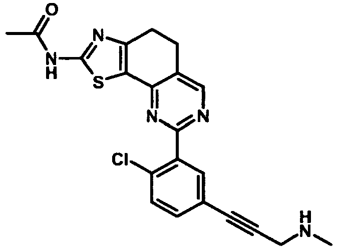

1.0 g (2.1 mmol) N-[8-(2-Chlor-5-iod-phenyl)-4,5-dihydro-thiazolo[4,5-h]quinazolin-2-yl]-acetamid werden unter Argon-Atmosphäre in 50 mL Tetrahydrofuran vorgelegt und mit 0.6 ml (9 mmol) N-Methylpropargylamin und 1 mL (6 mmol) Diisopropylethylamin versetzt. Das Gemisch wird sauerstofffrei gehalten und 29 mg (0.04 mmol) Triphenylphosphinpalladium(II)-chlorid und 8 mg (0.04 mmol) Kupfer(I)-iodid zugesetzt. Es wird 5 Stunden bei 80 °C gerührt. Nach Abkühlen auf Raumtemperatur wird das Reaktionsgemisch mit Wasser und 10%iger Ammoniaklösung versetzt und mit Dichlormethan extrahiert. Die organische Phase wird getrocknet und zur Trockene eingedampft. Der Rückstand wird chromatographisch gereinigt, das erhaltene Produkt mit Diethylether verrieben und abgesaugt.
Ausbeute: 0.27 g (MH+ = 424; RT = 2.31; Methode A)

Auf analogem Weg können folgende Verbindungen hergestellt werden:

**Tabelle 1:**

| | | | | | | |
|---|---|---|---|---|---|---|
| 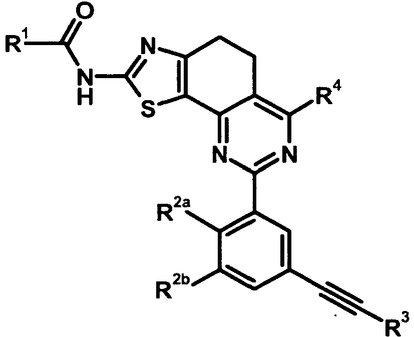 | | | | | | |

| **Beispiel** | **R¹** | **R^{2a}** | **R^{2b}** | **R⁴** | **R³** | **Analytik HPLC-MS** |
|---|---|---|---|---|---|---|
| 1 | H₃C-X₁ | 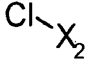 | H | H | 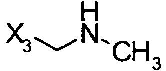 | MH+ = 424 |
| | | | | | | RT = 2.31 |
| | | | | | | Methode A |
| 2 | 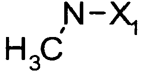 | 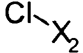 | H | H | 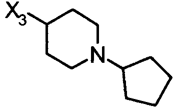 | |
| 3 | H₃C-X₁ | 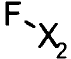 | H | H | 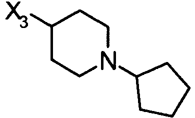 | MH+ = 516 |
| | | | | | | RT = 2.51 |
| | | | | | | Methode A |
| 4 | 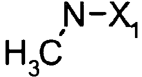 | 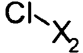 | H | H | 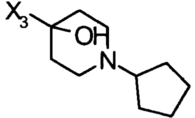 | MH+ = 563 |
| | | | | | | RT = 2.36 |
| | | | | | | Methode A |
| 5 | H₃C-X₁ | 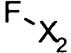 | H | H | 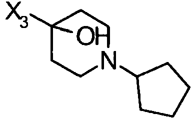 | MH+ = 532 |
| | | | | | | RT = 2.42 |
| | | | | | | Methode A |
| 6 | 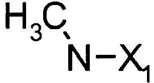 | 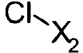 | H | H | 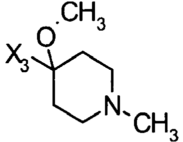 | |
| 7 | H₃C-X₁ | 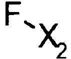 | H | H | 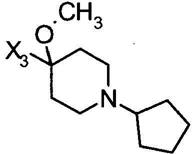 | MH+ = 546 |
| | | | | | | RT = 2.58 |
| | | | | | | Methode A |
| 8 | H₃C-X₁ | 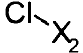 | H | H | 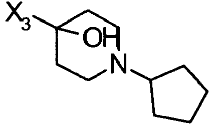 | MH+ = 548 |
| | | | | | | RT = 2.48 |
| | | | | | | Methode A |
| 9 | H₃C-X₁ | 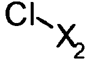 | H | H | 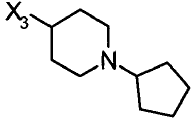 | MH+ = 532 |
| | | | | | | RT = 2.58 |
| | | | | | | Methode A |
| 10 | H₃C-X₁ | 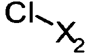 | H | H | 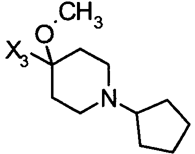 | MH+ = 562 |
| | | | | | | RT = 2.64 |
| | | | | | | Methode A |
| 11 | H₃C-X₁ | H | H | H | 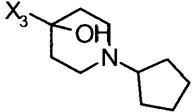 | MH+ = 514 |
| | | | | | | RT = 2.50 |
| | | | | | | Methode A |
| 12 | H₃C-X₁ | 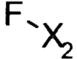 | H | H | 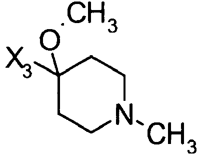 | MH+ = 492 |
| | | | | | | RT = 2.41 |
| | | | | | | Methode A |
| 13 | H₃C-X₁ | 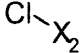 | H | H | 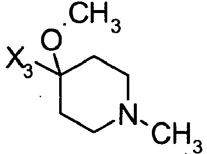 | MH+ = 508 |
| | | | | | | RT = 2.50 |
| | | | | | | Methode A |
| 14 | H₃C-X₁ | 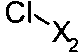 | H | H | 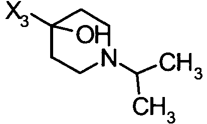 | MH+ = 522 |
| | | | | | | RT = 2.34 |
| | | | | | | Methode A |
| 15 | H₃C-X₁ | 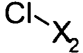 | H | H | 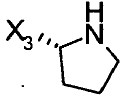 | MH+ = 450 |
| | | | | | | RT = 1.63 |
| | | | | | | Methode B |
| 16 | H₃C-X₁ | H | H | H | 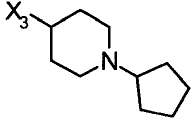 | MH+ = 498 |
| | | | | | | RT = 2.26 |
| | | | | | | Methode A |
| 17 | H₃C-X₁ | 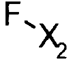 | H | H | 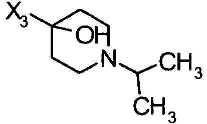 | MH+ = 506 |
| | | | | | | RT = 2.27 |
| | | | | | | Methode A |
| 18 | H₃C-X₁ | 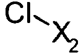 | H | H | 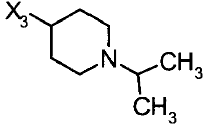 | MH+ = 506 |
| | | | | | | RT = 2.55 |
| | | | | | | Methode A |
| 19 | H₃C-X₁ | 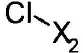 | H | H | 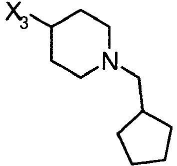 | MH+ = 546 |
| | | | | | | RT = 2.72 |
| | | | | | | Methode A |
| 20 | H₃C-X₁ | 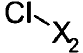 | H | H | 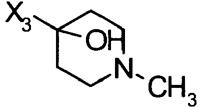 | MH+ = 494 |
| | | | | | | RT = 2.31 |
| | | | | | | Methode A |
| 21 | H₃C-X₁ | 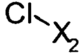 | H | H | 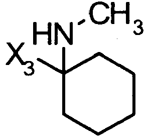 | MH+ = 492 |
| | | | | | | RT = 2.52 |
| | | | | | | Methode A |
| 22 | H₃C-X₁ | 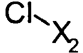 | H | H | 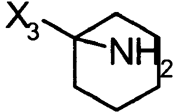 | MH+ = 478 |
| | | | | | | RT = 2.52 |
| | | | | | | Methode A |
| 23 | H₃C-X₁ | 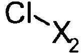 | H | H | 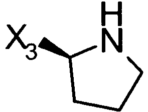 | MH+ = 450 |
| | | | | | | RT = 1.62 |
| | | | | | | Methode B |
| 24 | H₃C-X₁ | 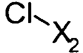 | H | H | 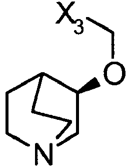 | MH+ = 520 |
| | | | | | | RT = 2.44 |
| | | | | | | Methode A |
| 25 | H₃C-X₁ | 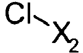 | H | H | 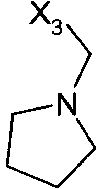 | MH+ = 466 |
| | | | | | | RT = 2.34 |
| | | | | | | Methode A |
| 26 | H₃C-X₁ | 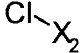 | H | H | 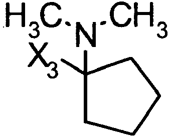 | MH+ = 492 |
| | | | | | | RT = 1.77 |
| | | | | | | Methode B |
| 27 | H₃C-X₁ | H | H | H | 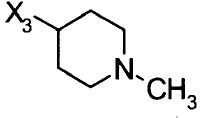 | MH+ = 444 |
| | | | | | | RT = 2.48 |
| | | | | | | Methode A |
| 28 | H₃C-X₁ | 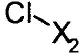 | H | H | 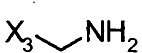 | |
| 29 | H₃C-X₁ | 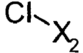 | H | H | 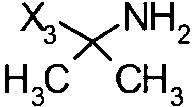 | MH+ = 438 |
| | | | | | | RT = 2.33 |
| | | | | | | Methode A |
| 30 | H₃C-X₁ | H | H | H | 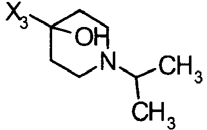 | MH+ = 488 |
| | | | | | | RT = 2.34 |
| | | | | | | Methode A |
| 31 | H₃C-X₁ | 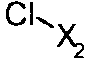 | H | H | 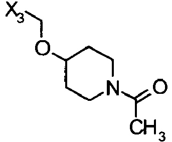 | MH+ = 536 |
| | | | | | | RT = 2.81 |
| | | | | | | Methode A |
| 32 | H₃C-X₁ | 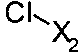 | H | H | 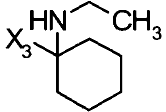 | MH+ = 506 |
| | | | | | | RT = 2.56 |
| | | | | | | Methode A |
| 33 | H₃C-X₁ | 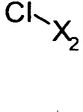 | H | H | 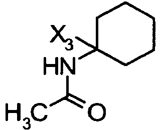 | MH+ = 520 |
| | | | | | | RT = 3.03 |
| | | | | | | Methode A |
| 34 | H₃C-X₁ | 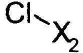 | H | H | 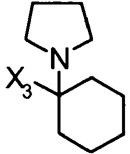 | MH+ = 532 |
| | | | | | | RT = 2.63 |
| | | | | | | Methode A |
| 35 | H₃C-X₁ | 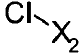 | H | H | 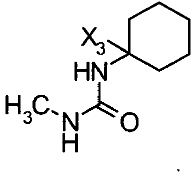 | MH+ = 535 |
| | | | | | | RT = 2.98 |
| | | | | | | Methode A |
| 36 | H₃C-X₁ | H | H | H | 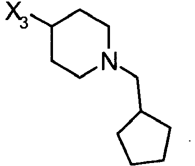 | MH+ = 512 |
| | | | | | | RT = 2.75 |
| | | | | | | Methode A |
| 37 | H₃C-X₁ | 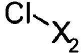 | H | H | 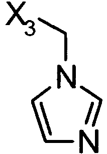 | MH+ = 461 |
| | | | | | | RT = 2.39 |
| | | | | | | Methode A |
| 38 | H₃C-X₁ | H | H | H | 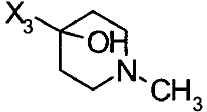 | MH+ = 460 |
| | | | | | | RT = 2.30 |
| | | | | | | Methode A |
| 39 | H₃C-X₁ | H | H | H | 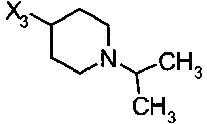 | MH+ = 472 |
| | | | | | | RT = 2.55 |
| | | | | | | Methode A |
| 40 | H₃C-X₁ | 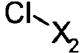 | H | H | 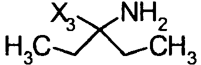 | MH+ = 466 |
| | | | | | | RT = 2.44 |
| | | | | | | Methode A |
| 41 | H₃C-X₁ | 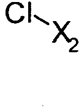 | H | H | 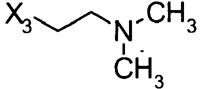 | MH+ = 452 |
| | | | | | | RT = 2.44 |
| | | | | | | Methode A |
| 42 | H₃C-X₁ | 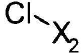 | H | H | 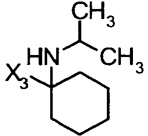 | MH+ = 520 |
| | | | | | | RT = 2.61 |
| | | | | | | Methode A |
| 43 | H₃C-X₁ | 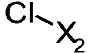 | H | H | 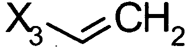 | MH+ = 466 |
| | | | | | | RT = 2.46 |
| | | | | | | Methode A |
| 44 | H₃C-X₁ | 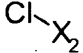 | H | H | 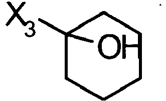 | MH+ = 479 |
| | | | | | | RT = 3.09 |
| | | | | | | Methode A |
| 45 | H₃C-X₁ | 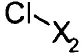 | H | H | 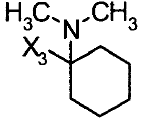 | MH+ = 506 |
| | | | | | | RT = 2.62 |
| | | | | | | Methode A |
| 46 | H₃C-X₁ | 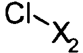 | H | H | 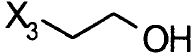 | MH+ = 425 |
| | | | | | | RT = 2.65 |
| | | | | | | Methode A |
| 47 | H₃C-X₁ | H | H | H | 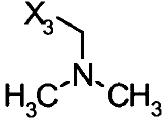 | MH+ = 404 |
| | | | | | | RT = 2.35 |
| | | | | | | Methode A |
| 48 | H₃C-X₁ | H | H | H | 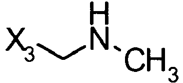 | MH+ = 390 |
| | | | | | | RT = 2.30 |
| | | | | | | Methode A |
| 49 | H₃C-X₁ | 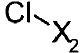 | H | H | 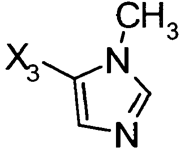 | MH+ = 461 |
| | | | | | | RT = 2.38 |
| | | | | | | Methode A |
| 50 | H₃C-X₁ | 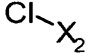 | H | H | 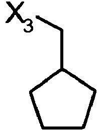 | MH+ = 463 |
| | | | | | | RT = 3.90 |
| | | | | | | Methode A |
| 51 | H₃C-X₁ | 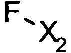 | H | H | 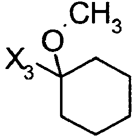 | MH+ = 477 |
| | | | | | | RT = 3.63 |
| | | | | | | Methode A |
| 52 | H₃C-X₁ | 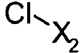 | H | H | 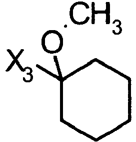 | MH+ = 493 |
| | | | | | | RT = 3.73 |
| | | | | | | Methode A |
| 53 | H₃C-X₁ | H | 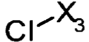 | H | 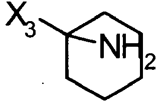 | MH+ = 478 |
| | | | | | | RT = 2.84 |
| | | | | | | Methode A |
| 54 | H₃C-X₁ | H | 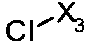 | H | 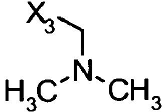 | MH+ = 438 |
| | | | | | | RT = 2.60 |
| | | | | | | Methode A |
| 55 | H₃C-X₁ | H | 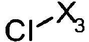 | H | 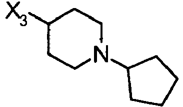 | MH+ = 432 |
| | | | | | | RT = 2.97 |
| | | | | | | Methode A |
| 56 | H₃C-X₁ | 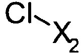 | H | H | 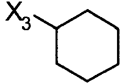 | MH+ = 462 |
| | | | | | | RT = 3.95 |
| | | | | | | Methode A |
| 57 | H₃C-X₁ | 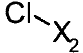 | H | H | 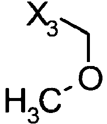 | MH+ = 424 |
| | | | | | | RT = 3.06 |
| | | | | | | Methode A |
| 58 | H₃C-X₁ | 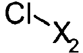 | H | H | 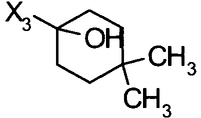 | MH+ = 507 |
| | | | | | | RT = 2.24 |
| | | | | | | Methode B |
| 59 | H₃C-X₁ | 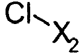 | H | H | 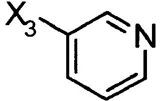 | MH+ = 458 |
| | | | | | | RT = 1.79 |
| | | | | | | Methode B |
| 60 | H₃C-X₁ | 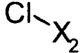 | H | H | 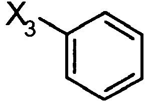 | MH+ = 457 |
| | | | | | | RT = 2.39 |
| | | | | | | Methode B |
| 61 | H₃C-X₁ | 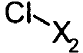 | H | H | 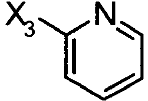 | MH+ = 458 |
| | | | | | | RT = 1.78 |
| | | | | | | Methode B |
| 62 | H₃C-X₁ | 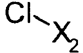 | H | H | 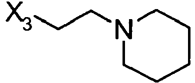 | MH+ = 493 |
| | | | | | | RT = 2.50 |
| | | | | | | Methode A |
| 63 | H₃C-X₁ | 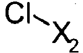 | H | H | 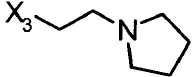 | MH+ = 478 |
| | | | | | | RT = 2.42 |
| | | | | | | Methode A |
| 64 | H₃C-X₁ | 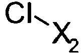 | H | H | 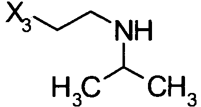 | MH+ = 466 |
| | | | | | | RT = 2.43 |
| | | | | | | Methode A |
| 65 | H₃C-X₁ | 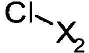 | H | H | 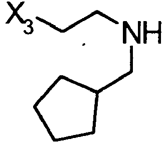 | MH+ = 506 |
| | | | | | | RT = 2.66 |
| | | | | | | Methode A |
| 66 | H₃C-X₁ | 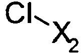 | H | H | 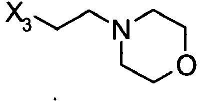 | MH+ = 495 |
| | | | | | | RT = 2.39 |
| | | | | | | Methode A |
| 67 | H₃C-X₁ | 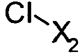 | H | H | 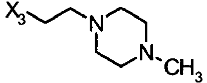 | MH+ = 507 |
| | | | | | | RT = 2.23 |
| | | | | | | Methode A |
| 68 | H₃C-X₁ | 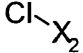 | H | H | 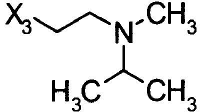 | MH+ = 480 |
| | | | | | | RT = 2.38 |
| | | | | | | Methode A |
| 69 | H₃C-X₁ | 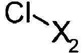 | H | H | 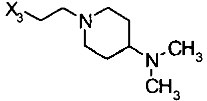 | MH+ = 535 |
| | | | | | | RT = 2.26 |
| | | | | | | Methode A |
| 70 | H₃C-X₁ | 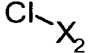 | H | H | 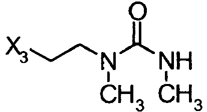 | MH+ = 495 |
| | | | | | | RT = 1.80 |
| | | | | | | Methode B |
| 71 | H₃C-X₁ | 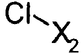 | H | H | 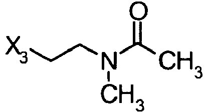 | MH+ = 480 |
| | | | | | | RT = 1.84 |
| | | | | | | Methode B |

### Beispiel 72) N-{8-{5-[3-(Acetyl-methyl-amino)-prop-1-ynyl]-2-chlor-phenyl}-4,5-dihydro-thiazolo[4,5-h]quinazolin-2-yl)-acetamid

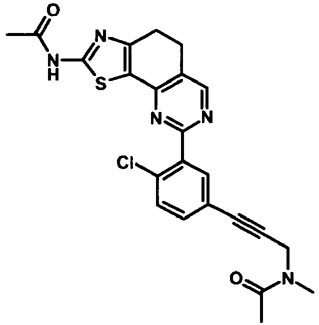

25 µl Essigsäure und 80 mg (0.25 mmol) O-(1H-Benzotriazol-1-yl)-N,N,N',N'-tetramethyluroniumtetrafluorborat (TBTU) werden in 5 mL Dichlormethan vorgelegt, mit 65 µL Diisopropylethylamin versetzt und 0,5 Stunden bei Raumtemperatur gerührt. 65 mg (0.15 mmol) N-{8-[2-Chlor-5-(3-methylamino-prop-1-ynyl)-phenyl]-4,5-dihydro-thiazolo[4,5-h]quinazolin-2-yl}-acetamid werden zugegeben, dann 16 Stunden bei Raumtemperatur gerührt. Anschließend wird das Reaktionsgemisch mit Kaliumcarbonatlösung und Dichlormethan extrahiert. Die organische Phase wird getrocknet und zur Trockene eingedampft. Der Rückstand wird chromatographisch gereinigt. Ausbeute: 12 mg (MH+ = 466; RT = 2.70; Methode A)

Analog können die folgenden Verbindungen, ausgehend von N-{8-[2-Chlor-5-(3-methylamino-prop-1-ynyl)-phenyl]-4,5-dihydro-thiazolo[4,5-h]quinazolin-2-yl}-acetamid (Beispiel 1) oder N-{8-[5-(3-Amino-prop-1-ynyl)-2-chlor-phenyl]-4,5-dihydro-thiazolo[4,5-h]quinazolin-2-yl}-acetamid (Beispiel 28) hergestellt werden.

**Tabelle 2:**

| | | | | | | |
|---|---|---|---|---|---|---|
| 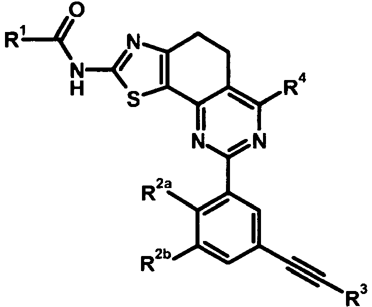 | | | | | | |

| **Beispiel** | **R¹** | **R^{2a}** | **R^{2b}** | **R⁴** | **R³** | **Analytik HPLC-MS** |
|---|---|---|---|---|---|---|
| 72 | H₃C-X₁ | 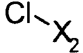 | H | H | 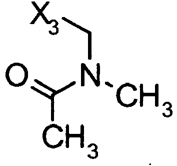 | MH+ = 466 |
| | | | | | | RT = 2.70 |
| | | | | | | Methode A |
| 73 | H₃C-X₁ | 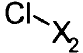 | H | H | 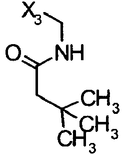 | MH+ = 508 |
| | | | | | | RT = 3.01 |
| | | | | | | Methode A |
| 74 | H₃C-X₁ | H | H | H | 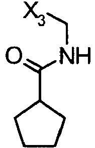 | MH+ = 472 |
| | | | | | | RT = 2.99 |
| | | | | | | Methode A |
| 75 | H₃C-X₁ | 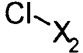 | H | H | 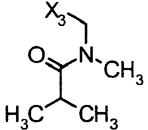 | MH+ = 494 |
| | | | | | | RT = 2.96 |
| | | | | | | Methode A |
| 76 | H₃C-X₁ | H | H | H | 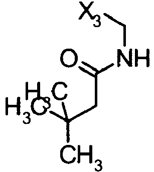 | MH+ = 474 |
| | | | | | | RT = 3.10 |
| | | | | | | Methode A |
| 77 | H₃C-X₁ | 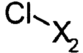 | H | H | 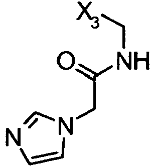 | MH+ = 518 |
| | | | | | | RT = 2.31 |
| | | | | | | Methode A |
| 78 | H₃C-X₁ | 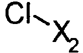 | H | H | 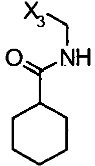 | MH+ = 502 |
| | | | | | | RT = 3.05 |
| | | | | | | Methode A |
| 79 | H₃C-X₁ | 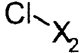 | H | H | 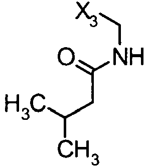 | MH+ = 494 |
| | | | | | | RT = 2.88 |
| | | | | | | Methode A |
| 80 | H₃C-X₁ | 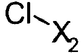 | H | H | 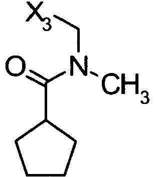 | MH+ = 520 |
| | | | | | | RT = 3.17 |
| | | | | | | Methode A |
| 81 | H₃C-X₁ | 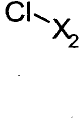 | H | H | 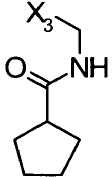 | MH+ = 506 |
| | | | | | | RT = 2.97 |
| | | | | | | Methode A |
| 82 | H₃C-X₁ | 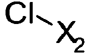 | H | H | 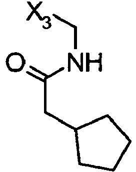 | MH+ = 520 |
| | | | | | | RT = 3.08 |
| | | | | | | Methode A |
| 83 | H₃C-X₁ | 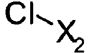 | H | H | 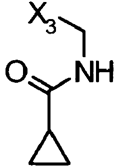 | MH+ = 478 |
| | | | | | | RT = 2.80 |
| | | | | | | Methode A |
| 84 | H₃C-X₁ | 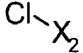 | H | H | 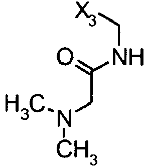 | MH+ = 495 |
| | | | | | | RT = 2.29 |
| | | | | | | Methode A |
| 85 | H₃C-X₁ | 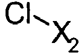 | H | H | 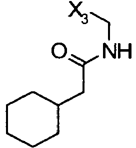 | MH+ = 534 |
| | | | | | | RT = 3.17 |
| | | | | | | Methode A |
| 86 | H₃C-X₁ | 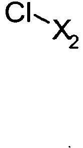 | H | H | 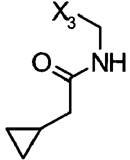 | MH+ = 492 |
| | | | | | | RT = 2.79 |
| | | | | | | Methode A |
| 87 | H₃C-X₁ | 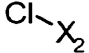 | H | H | 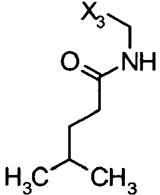 | MH+ = 508 |
| | | | | | | RT = 3.04 |
| | | | | | | Methode A |
| 88 | H₃C-X₁ | H | 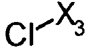 | H | 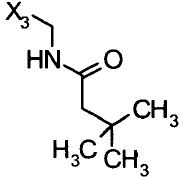 | MH+ = 508 |
| | | | | | | RT = 3.57 |
| | | | | | | Methode A |
| 89 | H₃C-X₁ | H | H | H | 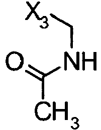 | MH+ = 418 |
| | | | | | | RT = 2.60 |
| | | | | | | Methode A |
| 90 | H₃C-X₁ | 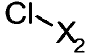 | H | H | 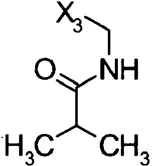 | MH+ = 480 |
| | | | | | | RT = 2.82 |
| | | | | | | Methode A |
| 91 | H₃C-X₁ | 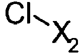 | H | H | 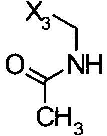 | MH+ = 452 |
| | | | | | | RT = 2.58 |
| | | | | | | Methode A |
| 92 | H₃C-X₁ | H | H | H | 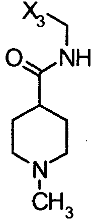 | MH+ = 501 |
| | | | | | | RT = 2.36 |
| | | | | | | Methode A |
| 93 | H₃C-X₁ | 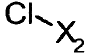 | H | H | 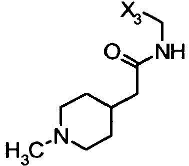 | MH+ = 549 |
| | | | | | | RT = 2.32 |
| | | | | | | Methode A |
| 94 | H₃C-X₁ | 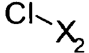 | H | H | 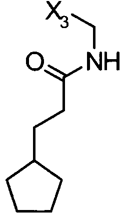 | MH+ = 534 |
| | | | | | | RT = 3.21 |
| | | | | | | Methode A |

### Beispiel 95) N-(8-{2-Chlor-5-[3-(methansulfonyl-methyl-amino)-prop-1-ynyl]-phenyl}-4,5-dihydro-thiazolo[4,5-h]quinazolin-2-yl)-acetamid

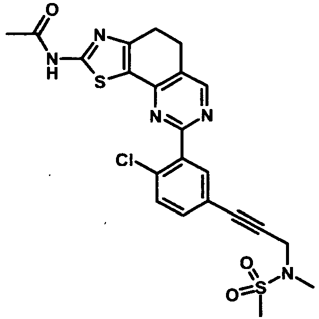

Eine Mischung aus 65 mg (0.15 mmol) N-{8-[2-Chlor-5-(3-methylamino-prop-1-ynyl)-phenyl]-4,5-dihydro-thiazolo[4,5-h]quinazolin-2-yl}-acetamid, 100 µL Triethylamin und 30 µL Methansulfonsäurechlorid in 1 mL Dichlormethan wird über Nacht bei Raumtemperatur gerührt. Die Reaktionsmischung wird mit gesättigter wässriger Natriumhydrogencarbonat-Lösung gewaschen und die organische Phase eingeengt. Der verbliebene Rückstand wird mit Ether verrührt. Ausbeute: 55 mg gelber Feststoff (MH+ = 502; RT = 2.98; Methode A)

Analog können die folgenden Verbindungen, ausgehend von N-{8-[2-Chlor-5-(3-methylamino-prop-1-ynyl)-phenyl]-4,5-dihydro-thiazolo[4,5-h]quinazolin-2-yl}-acetamid (Beispiel 1) oder N-{8-[5-(3-Amino-prop-1-ynyl)-2-chlor-phenyl]-4,5-dihydro-thiazolo[4,5-h]quinazolin-2-yl}-acetamid (Beispiel 28) hergestellt werden.

**Tabelle 3:**

| | | | | | | |
|---|---|---|---|---|---|---|
| 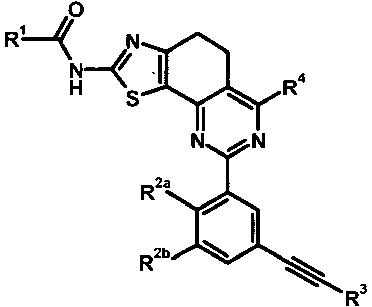 | | | | | | |

| **Beispiel** | **R¹** | **R^{2a}** | **R^{2b}** | **R⁴** | **R³** | **Analytik HPLC-MS** |
|---|---|---|---|---|---|---|
| 95 | H₃C-X₁ | 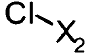 | H | H | 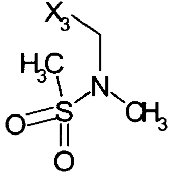 | MH+ = 486 |
| | | | | | | RT = 2.90 |
| | | | | | | Methode A |
| 96 | H₃C-X₁ | 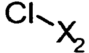 | H | H | 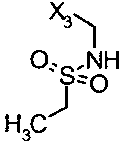 | MH+ = 502 |
| | | | | | | RT = 3.07 |
| | | | | | | Methode A |
| 97 | H₃C-X₁ | 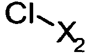 | H | H | 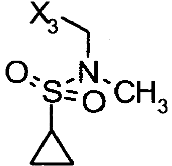 | MH+ = 528 |
| | | | | | | RT = 3.18 |
| | | | | | | Methode A |
| 98 | H₃C-X₁ | 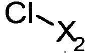 | H | H | 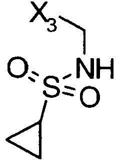 | MH+ = 514 |
| | | | | | | RT = 2.86 |
| | | | | | | Methode A |
| 99 | H₃C-X₁ | 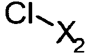 | H | H | 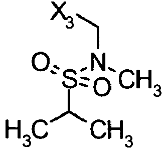 | MH+ = 530 |
| | | | | | | RT = 3.18 |
| | | | | | | Methode A |
| 100 | H₃C-X₁ | 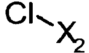 | H | H | 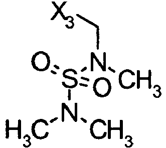 | MH+ = 531 |
| | | | | | | RT = 3.15 |
| | | | | | | Methode A |
| 101 | H₃C-X₁ | 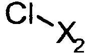 | H | H | 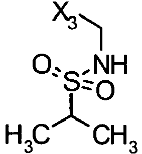 | MH+ = 516 |
| | | | | | | RT = 2.88 |
| | | | | | | Methode A |
| 102 | H₃C-X₁ | 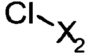 | H | H | 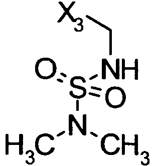 | MH+ = 517 |
| | | | | | | RT = 2.91 |
| | | | | | | Methode A |
| 103 | H₃C-X₁ | 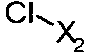 | H | H | 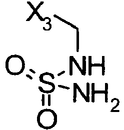 | MH+ = 489 |
| | | | | | | RT = 1.74 |
| | | | | | | Methode B |
| 104 | H₃C-X₁ | 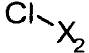 | H | H | 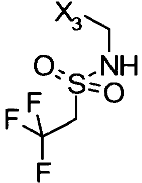 | MH+ = 556 |
| | | | | | | RT = 2.86 |
| | | | | | | Methode A |
| 105 | H₃C-X₁ | 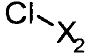 | H | H | 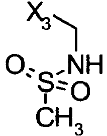 | MH+ = 488 |
| | | | | | | RT = 2.73 |
| | | | | | | Methode A |
| 106 | H₃C-X₁ | 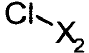 | H | H | 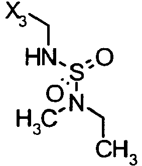 | MH+ = 530 |
| | | | | | | RT = 1.99 |
| | | | | | | Methode B |
| 107 | H₃C-X₁ | 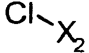 | H | H | 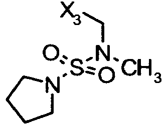 | MH+ = 557 |
| | | | | | | RT = 3.25 |
| | | | | | | Methode A |
| 108 | H₃C-X₁ | 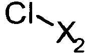 | H | H | 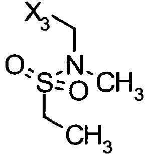 | MH+ = 516 |
| | | | | | | RT = 3.08 |
| | | | | | | Methode A |
| 109 | H₃C-X₁ | H | 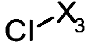 | H | 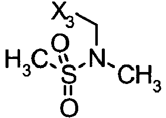 | MH+ = 502 |
| | | | | | | RT = 3.47 |
| | | | | | | Methode A |
| 110 | H₃C-X₁ | 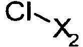 | H | H | 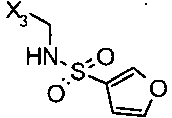 | MH+ = 540 |
| | | | | | | RT = 1.96 |
| | | | | | | Methode B |
| 111 | H₃C-X₁ | 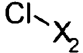 | H | H | 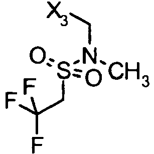 | MH+ = 570 |
| | | | | | | RT = 3.25 |
| | | | | | | Methode A |
| 112 | H₃C-X₁ | H | H | H | 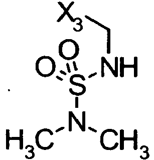 | MH+ = 483 |
| | | | | | | RT = 2.98 |
| | | | | | | Methode A |
| 113 | H₃C-X₁ | 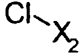 | H | H | 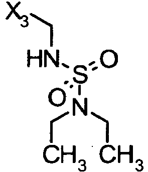 | MH+ = 545 |
| | | | | | | RT = 3.15 |
| | | | | | | Methode A |
| 114 | H₃C-X₁ | 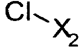 | H | H | 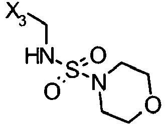 | MH+ = 558 |
| | | | | | | RT = 1.88 |
| | | | | | | Methode B |
| 115 | H₃C-X₁ | 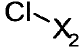 | H | H | 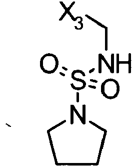 | MH+ = 543 |
| | | | | | | RT = 3.02 |
| | | | | | | Methode A |
| 116 | H₃C-X₁ | 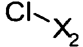 | H | H | 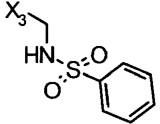 | MH+ = 550 |
| | | | | | | RT = 2.03 |
| | | | | | | Methode B |
| 117 | H₃C-X₁ | H | H | H | 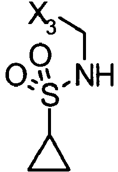 | MH+ = 480 |
| | | | | | | RT = 2.95 |
| | | | | | | Methode A |
| 118 | H₃C-X₁ | H | 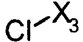 | H | 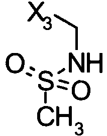 | MH+ = 488 |
| | | | | | | RT = 3.21 |
| | | | | | | Methode A |
| 119 | H₃C-X₁ | 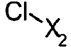 | H | H | 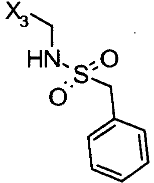 | MH+ = 564 |
| | | | | | | RT = 2.05 |
| | | | | | | Methode B |
| 120 | H₃C-X₁ | 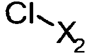 | H | H | 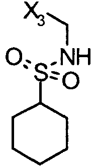 | MH+ = 556 |
| | | | | | | RT = 3.20 |
| | | | | | | Methode A |
| 121 | H₃C-X₁ | 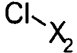 | H | H | 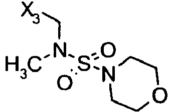 | MH+ = 573 |
| | | | | | | RT = 2.01 |
| | | | | | | Methode B |
| 122 | H₃C-X₁ | 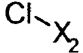 | H | H | 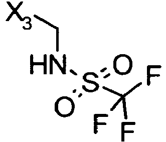 | MH+ = 542 |
| | | | | | | RT=2.10 |
| | | | | | | Methode B |
| 123 | H₃C-X₁ | 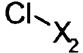 | H | H | 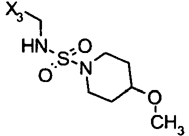 | MH+ = 587 |
| | | | | | | RT = 1.96 |
| | | | | | | Methode B |

### Beispiel 124) N-(8-{2-Chlor-5-[3-(3-methyl-ureido)-prop-1-ynyl]-phenyl}-4,5-dihydrothiazolo[4,5-h]quinazolin-2-yl)-acetamid

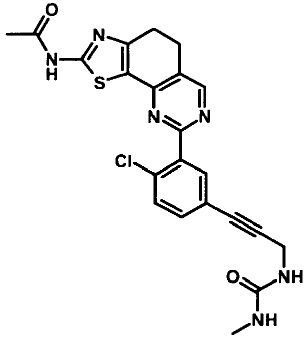

Eine Mischung aus 150 mg (0.37 mmol) N-{8-[5-(3-Amino-prop-1-ynyl)-2-chlor-phenyl]-4,5-dihydro-thiazolo[4, 5-h]quinazolin-2-yl}-acetamid, 0.1 mL (0.68 mmol) Triethylamin und 40 mg (0.70 mmol) Methylisocyanat in 4 mL Acetonitril wird über Nacht bei Raumtemperatur gerührt. Der ausgefallene Feststoff wird abgesaugt und mit Ether nachgewaschen. Ausbeute: 133 mg gelben Feststoff (Schmp.: 133 °C).

Analog können die folgenden Verbindungen, ausgehend von N-{8-[2-Chlor-5-(3-methylamino-prop-1-ynyl)-phenyl]-4,5-dihydro-thiazolo[4,5-h]quinazolin-2-yl}-acetamid (Beispiel 1) hergestellt werden.

**Tabelle 4:**

| | | | | | | |
|---|---|---|---|---|---|---|
| 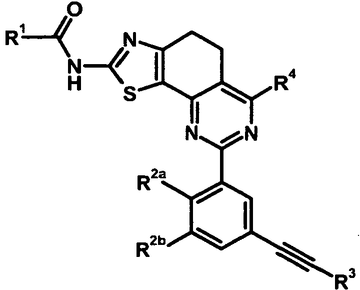 | | | | | | |

| **Beispiel** | **R¹** | **R^{2a}** | **R^{2b}** | **R⁴** | **R³** | **Analytik HPLC-MS** |
|---|---|---|---|---|---|---|
| 124 | H₃C-X₁ | 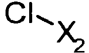 | H | H | 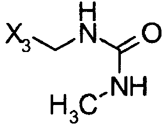 | MH+ = 467 |
| | | | | | | RT = 2.53 |
| | | | | | | Methode A |
| 125 | H₃C-X₁ | 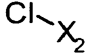 | H | H | 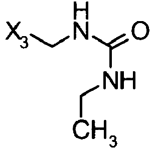 | MH+ = 481 |
| | | | | | | RT = 2.60 |
| | | | | | | Methode A |
| 126 | H₃C-X₁ | 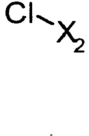 | H | H | 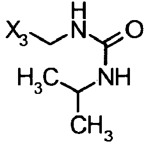 | MH+ = 495 |
| | | | | | | RT = 2.73 |
| | | | | | | Methode A |
| 127 | H₃C-X₁ | 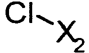 | H | H | 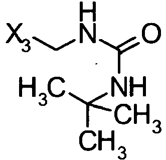 | MH+ = 509 |
| | | | | | | RT = 2.96 |
| | | | | | | Methode A |
| 128 | H₃C-X₁ | 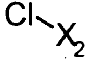 | H | H | 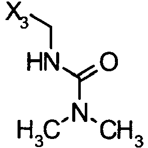 | MH+ = 481 |
| | | | | | | RT = 2.62 |
| | | | | | | Methode A |
| 129 | H₃C-X₁ | 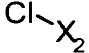 | H | H | 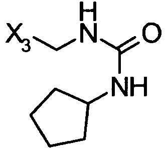 | MH+ = 521 |
| | | | | | | RT = 2.89 |
| | | | | | | Methode A |
| 130 | H₃C-X₁ | 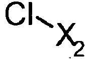 | H | H | 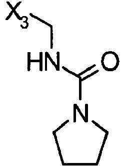 | MH+ = 507 |
| | | | | | | RT = 2.74 |
| | | | | | | Methode A |
| 131 | H₃C-X₁ | 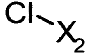 | H | H | 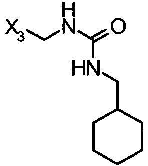 | MH+ = 549 |
| | | | | | | RT = 3.16 |
| | | | | | | Methode A |

### Beispiel 132) N-{8-[2-Chlor-5-(3-cyclopentylamino-prop-1-ynyl)-phenyl]-4,5-dihydro-thiazolo[4,5-h]quinazolin-2-yl}-acetamid

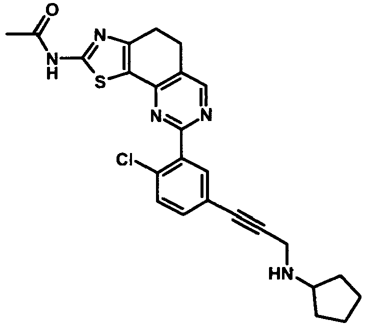

Unter Schutzgasatmosphäre werden 2.0 g (4.1 mmol) N-[8-(2-Chlor-5-iod-phenyl)-4,5-dihydro-thiazolo[4,5-h]quinazolin-2-yl]-acetamid und 0.7 mL Diisopropylethylamin werden in 50 mL THF gelöst und mit 0.7 ml (11.6 mmol) Propargylalkohol, 290 mg (0.4 mmol) Triphenylphosphinpalladium (II)-chlorid und 79 mg (0.4 mmol) Kupfer(I)-iodid versetzt. Die Reaktionsmischung wird 1.5 Stunden auf 80 °C erwärmt und anschließend eingeengt. Der Rückstand wird mit Dichlormethan verrührt und der erhaltene Feststoff abgesaugt. Ausbeute: 1.7 g gelber Feststoff.

200 mg (0.49 mmol) der zuvor beschriebenen Zwischenstufe und 0.1 mL Triethylamin werden in 20 mL Dichlormethan suspendiert und bei 0 °C mit 50 µL Methansulfonsäurechlorid versetzt. Nach drei Stunden wird die Reaktionsmischung mit weiteren 0.2 mL Methansulfonsäurechlorid sowie einer Spatelspitze 4-Dimethylaminopyridin versetzt und 30 Minuten bei Raumtemperatur gerührt. Die Mischung wird einrotiert und direkt in der folgenden Reaktion eingesetzt. Ausbeute: 120 mg zähes gelbes Öl.

60 mg (0.12 mmol) der zuvor beschriebenen Methansulfonat-Zwischenstufe werden in 1 mL Dimethylformamid gelöst und mit 13 mg (0.15 mmol) Cyclopentylamin versetzt. Die Reaktionsmischung wird über Nacht bei 50 °C gerührt. Anschließend werden weitere 150 mg Cyclopentylamin zugegeben und zwei Stunden bei 70 °C gerührt. Die Mischung wird durch RP-HPLC gereinigt. Ausbeute: 22 mg hellgelber Feststoff.

Analog können die folgenden Verbindungen, ausgehend von N-{8-[2-Chlor-5-(3-methylamino-prop-1-ynyl)-phenyl]-4,5-dihydro-thiazolo[4,5-h]quinazolin-2-yl}-acetamid (Beispiel 1) hergestellt werden.

**Tabelle 5:**

| | | | | | | |
|---|---|---|---|---|---|---|
| 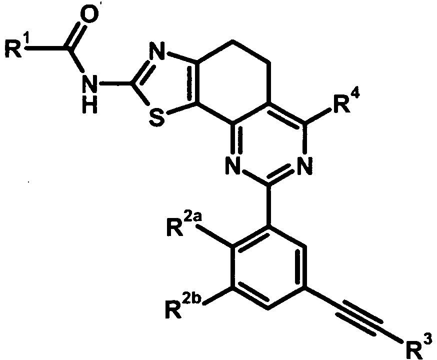 | | | | | | |

| **Beispiel** | **R¹** | **R^{2a}** | **R^{2b}** | **R⁴** | **R³** | **Analytik HPLC-MS** |
|---|---|---|---|---|---|---|
| 132 | H₃C-X₁ | 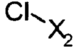 | H | H | 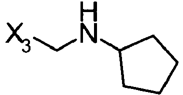 | MH+ = 478 |
| | | | | | | RT = 2.46 |
| | | | | | | Methode A |
| 133 | H₃C-X₁ | 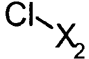 | H | H | 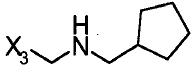 | MH+ = 493 |
| | | | | | | RT = 2.57 |
| | | | | | | Methode A |
| 134 | H₃C-X₁ | 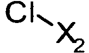 | H | H | 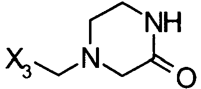 | |
| 135 | H₃C-X₁ | 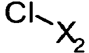 | H | H | 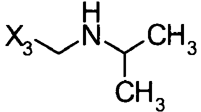 | MH+ = 452 |
| | | | | | | RT = 2.40 |
| | | | | | | Methode A |
| 136 | H₃C-X₁ | 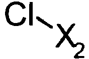 | H | H | 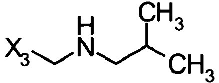 | MH+ = 467 |
| | | | | | | RT = 2.44 |
| | | | | | | Methode A |
| 137 | H₃C-X₁ | 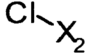 | H | H | 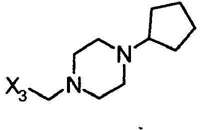 | MH+ = 547 |
| | | | | | | RT = 2.46 |
| | | | | | | Methode A |
| 138 | H₃C-X₁ | 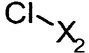 | H | H | 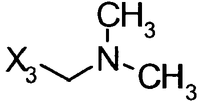 | MH+ = 438 |
| | | | | | | RT = 2.30 |
| | | | | | | Methode A |
| 139 | H₃C-X₁ | 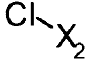 | H | H | 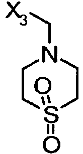 | MH+ = 528 |
| | | | | | | RT = 2.54 |
| | | | | | | Methode A |
| 140 | H₃C-X₁ | 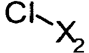 | H | H | 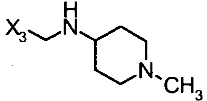 | MH+ = 507 |
| | | | | | | RT = 2.19 |
| | | | | | | Methode A |
| 141 | H₃C-X₁ | 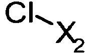 | H | H | 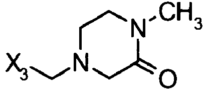 | MH+ = 507 |
| | | | | | | RT = 2.28 |
| | | | | | | Methode A |
| 142 | H₃C-X₁ | 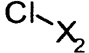 | H | H | 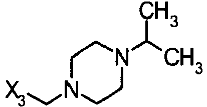 | MH+ = 521 |
| | | | | | | RT = 2.35 |
| | | | | | | Methode A |
| 143 | H₃C-X₁ | 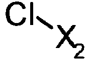 | H | H | 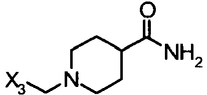 | |
| 144 | H₃C-X₁ | 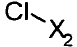 | H | H | 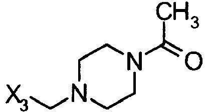 | |
| 145 | H₃C-X₁ | 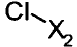 | H | H | 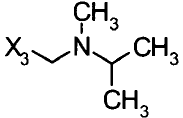 | MH+ = 466 |
| | | | | | | RT = 2.38 |
| | | | | | | Methode A |
| 146 | H₃C-X₁ | 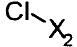 | H | H | 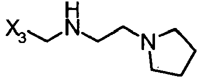 | MH+ = 507 |
| | | | | | | RT = 1.79 |
| | | | | | | Methode A |
| 147 | H₃C-X₁ | 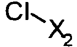 | H | H | 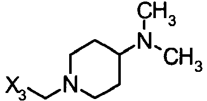 | |
| 148 | H₃C-X₁ | 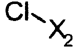 | H | H | 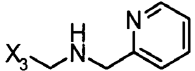 | MH+ = 501 |
| | | | | | | RT = 1.65 |
| | | | | | | Methode B |
| 149 | H₃C-X₁ | 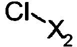 | H | H | 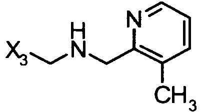 | MH+ = 515 |
| | | | | | | RT = 1.72 |
| | | | | | | Methode B |
| 150 | H₃C-X₁ | 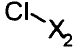 | H | H | 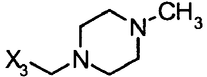 | MH+ = 493 |
| | | | | | | RT = 2.28 |
| | | | | | | Methode A |
| 151 | H₃C-X₁ | 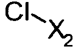 | H | H | 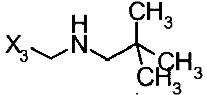 | MH+ = 480 |
| | | | | | | RT = 2.54 |
| | | | | | | Methode A |
| 152 | H₃C-X₁ | 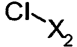 | H | H | 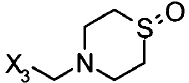 | |
| 153 | H₃C-X₁ | 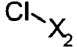 | H | H | 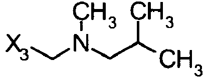 | MH+ = 480 |
| | | | | | | RT = 1.71 |
| | | | | | | Methode B |
| 154 | H₃C-X₁ | 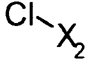 | H | H | 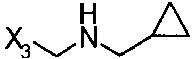 | MH+ = 464 |
| | | | | | | RT = 1.68 |
| | | | | | | Methode B |

### Beispiel 155) N-[8-(2-Chlor-5-piperidin-4-ylethynyl-phenyl)-4,5-dihydro-thiazolo(4, 5-h]quinazolin-2-yl]-acetamid

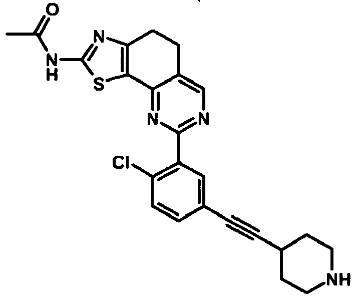

2.0 g (4.0 mmol) N-[8-(2-Chlor-5-iod-phenyl)-4,5-dihydro-thiazolo[4,5-h]quinazolin-2-yl]-acetamid werden unter Argon-Atmosphäre in 50 mL Tetrahydrofuran vorgelegt und mit 1.5 g (7 mmol) 4-Ethynyl-piperidin-1-carbonsäure tert-butylester und 0.5 ml (3 mmol) Diisopropylethylamin versetzt. Das Gemisch wird sauerstofffrei gehalten und 78 mg (0.1 mmol) Triphenylphosphinpalladium(II)-chlorid und 21 mg (0.1 mmol) Kupfer(I)-iodid zugesetzt. Es wird 5 Stunden bei 80 °C gerührt. Nach Abkühlen auf Raumtemperatur wird das Reaktionsgemisch mit Dichlormethan versetzt und mit verdünnter Ammoniaklösung gewaschen. Die organische Phase wird getrocknet und zur Trockene eingedampft. Der Rückstand wird chromatographisch gereinigt, entsprechende Fraktionen vereinigt und gefriergetrocknet. Das erhaltene Zwischenprodukt wird 2 Stunden in etherischer Salzsäure gerührt, abgesaugt und getrocknet. Ausbeute: 65 mg (Smp.: 162 °C; MH+ = 430; RT = 3.72; Methode A)

### Beispiel 156) N-{8-[5-(1-Acetyl-piperidin-4-ylethynyl)-2-chlor-phenyl]-4,5-dihydro-thiazolo[4,5-h]quinazolin-2-yl}-acetamid

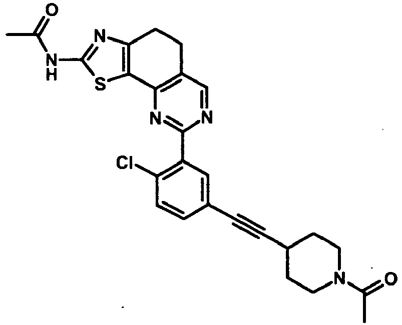

7 µl Essigsäure und 50 mg (0.16 mmol) 0-(1H-Benzotriazol-1-yl)-N,N,N',N'-tetramethyluroniumtetrafluorborat (TBTU) werden in 5 mL Dichlormethan vorgelegt, mit 32 µl Diisopropylethylamin versetzt und 0.5 Stunden bei Raumtemperatur gerührt. 60 mg (0.13 mmol) N-[8-(2-Chlor-5-piperidin-4-ylethynyl-phenyl)-4,5-dihydro-thiazolo[4, 5-h]quinazolin-2-yl]-acetamid (Beispiel 155) werden zugegeben, dann 16 Stunden bei Raumtemperatur gerührt. Anschließend wird das Reaktionsgemisch mit Kaliumcarbonatlösung und Dichlormethan extrahiert. Die organische Phase wird getrocknet und zur Trockene eingedampft. Der Rückstand wird chromatographisch gereinigt. Ausbeute: 15 mg (MH+ = 506; RT = 2.87; Methode A)

Analog können die folgenden Beispiele hergestellt werden:

**Tabelle 6:**

| | | | | | | |
|---|---|---|---|---|---|---|
| 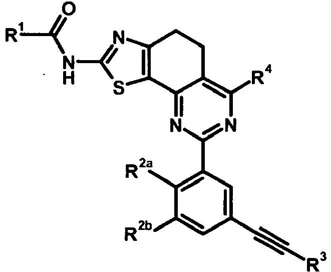 | | | | | | |

| **Beispiel** | **R¹** | **R^{2a}** | **R^{2b}** | **R⁴** | **R³** | **Analytik HPLC-MS** |
|---|---|---|---|---|---|---|
| 156 | H₃C-X₁ | 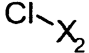 | H | H | 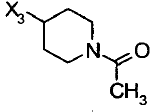 | MH+ = 506 |
| | | | | | | RT = 2.87 |
| | | | | | | Methode A |
| 157 | H₃C-X₁ | 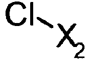 | H | H | 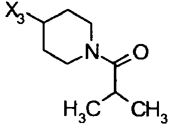 | MH+ = 534 |
| | | | | | | RT = 3.19 |
| | | | | | | Methode A |
| 158 | H₃C-X₁ | 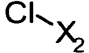 | H | H | 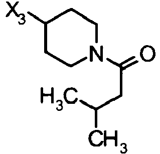 | MH+ = 548 |
| | | | | | | RT = 3.29 |
| | | | | | | Methode A |
| 159 | H₃C-X₁ | 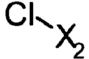 | H | H | 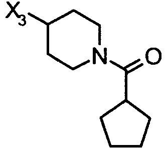 | MH+ = 560 |
| | | | | | | RT = 3.45 |
| | | | | | | Methode A |
| 160 | H₃C-X₁ | 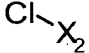 | H | H | 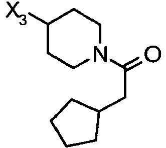 | MH+ = 574 |
| | | | | | | RT = 3.45 |
| | | | | | | Methode A |

### Beispiel 161) N-{8-[2-Chlor-5-(1-methansulfonyl-piperidin-4-ylethynyl)-phenyl]-4,5-dihydro-thiazolo[4,5-h]quinazolin-2-yl}-acetamid

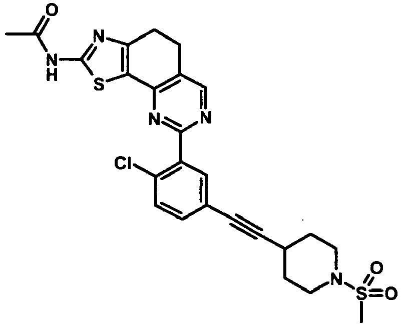

Eine Mischung aus 50 mg (0.11 mmol) N-[8-(2-Chlor-5-piperidin-4-ylethynyl-phenyl)-4,5-dihydro-thiazolo[4, 5-h]quinazolin-2-yl]-acetamid, 65 µL Triethylamin und 15 µL Methansulfonsäurechlorid in 1 mL Dichlormethan wird drei Stunden bei Raumtemperatur gerührt. Die Reaktionsmischung wird mit gesättigter wässriger Natriumhydrogencarbonat-Lösung gewaschen und die organische Phase eingeengt. Der verbliebene Rückstand wird mittels RP-HPLC gereinigt. Ausbeute: 23 mg gelber Feststoff (MH+ = 542; RT = 3.07; Methode A)

Analog können die folgenden Beispiele hergestellt werden:

**Tabelle 7:**

| | | | | | | |
|---|---|---|---|---|---|---|
| 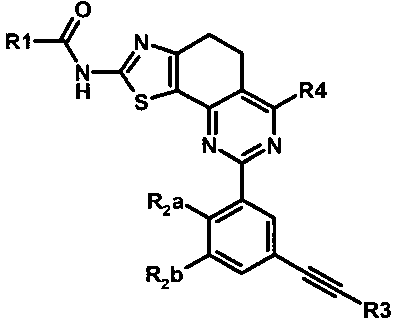 | | | | | | |

| **Beispiel** | **R¹** | **R^{2a}** | **R^{2b}** | **R⁴** | **R³** | **Analytik HPLC-MS** |
|---|---|---|---|---|---|---|
| 161 | H₃C-X₁ | 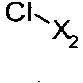 | H | H | 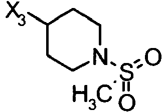 | MH+ = 542 |
| | | | | | | RT = 3.07 |
| | | | | | | Methode A |
| 162 | H₃C-X₁ | 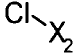 | H | H | 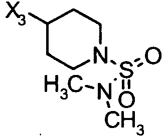 | MH+ = 571 |
| | | | | | | RT = 3.23 |
| | | | | | | Methode A |
| 163 | H₃C-X₁ | 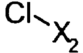 | H | H | 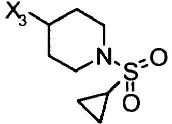 | MH+ = 568 |
| | | | | | | RT = 3.21 |
| | | | | | | Methode A |
| 164 | H₃C-X₁ | 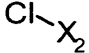 | H | H | 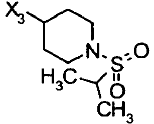 | MH+ = 570 |
| | | | | | | RT = 3.23 |
| | | | | | | Methode A |

### Beispiel 165) 4-[3-(2-Acetylamino-4,5-dihydro-thiazolo[4,5-h]quinazolin-8-yl)-4-chlor-phenylethynyl]-piperidin-1-carbonsäureisopropylamid

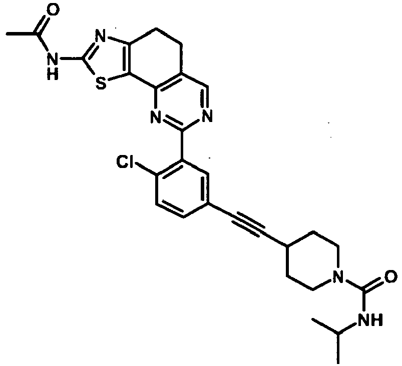

50 mg (0.11 mmol) N-[8-(2-Chlor-5-piperidin-4-ylethynyl-phenyl)-4,5-dihydro-thiazolo[4, 5-h]quinazolin-2-yl]-acetamid werden in 1 mL Acetonitril suspendiert und nacheinander mit 17 µL Triethylamin und 22 µM Isopropylisocyanat versetzt. Die gelbe Suspension wird drei Stunden bei Raumtemperatur gerührt und anschließend mit wässriger Kaliumcarbonat-Lösung gewaschen. Die organische Phase wird eingeengt und der Rückstand über RP-HPLC gereinigt. Ausbeute: 30 mg gelber Feststoff.

Analog können durch Einsatz der entsprechenden Isocyanate bzw. Carbamoylchloride die folgenden Beispiele hergestellt werden:

**Tabelle 8:**

| | | | | | | |
|---|---|---|---|---|---|---|
| 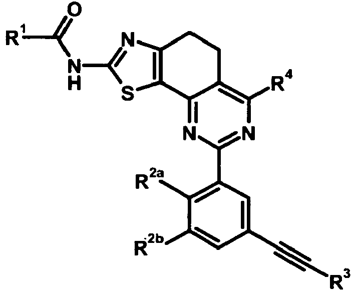 | | | | | | |

| **Beispiel** | **R¹** | **R^{2a}** | **R^{2b}** | **R⁴** | **R³** | **Analytik HPLC-MS** |
|---|---|---|---|---|---|---|
| 165 | H₃C-X₁ | 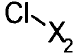 | H | H | 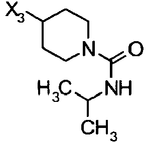 | MH+ = 549 |
| | | | | | | RT = 3.09 |
| | | | | | | Methode A |
| 166 | H₃C-X₁ | 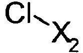 | H | H | 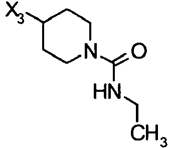 | MH+ = 535 |
| | | | | | | RT = 2.89 |
| | | | | | | Methode A |
| 167 | H₃C-X₁ | 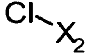 | H | H | 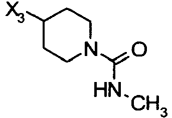 | MH+ = 521 |
| | | | | | | RT = 2.84 |
| | | | | | | Methode A |
| 168 | H₃C-X₁ | 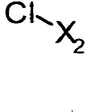 | H | H | 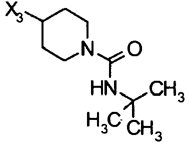 | MH+ = 563 |
| | | | | | | RT = 3.28 |
| | | | | | | Methode A |
| 169 | H₃C-X₁ | 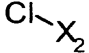 | H | H | 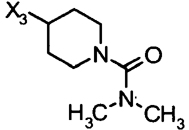 | MH+ = 535 |
| | | | | | | RT = 3.08 |
| | | | | | | Methode A |
| 170 | H₃C-X₁ | 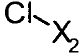 | H | H | 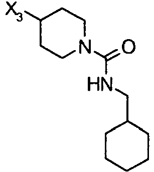 | MH+ = 603 |
| | | | | | | RT = 3.44 |
| | | | | | | Methode A |
| 171 | H₃C-X₁ | 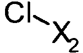 | H | H | 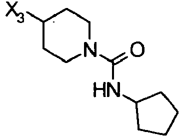 | MH+ = 575 |
| | | | | | | RT = 3.19 |
| | | | | | | Methode A |
| 172 | H₃C-X₁ | 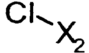 | H | H | 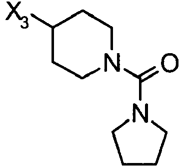 | MH+ = 561 |
| | | | | | | RT = 3.18 |
| | | | | | | Methode A |
| 173 | H₃C-X₁ | 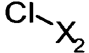 | H | H | 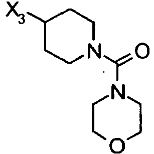 | MH+ = 577 |
| | | | | | | RT = 2.99 |
| | | | | | | Methode A |

### Beispiel 174) N-{8-[2-Chlor-5-(5-morpholin-4-yl-pent-1-ynyl)-phenyl]-4,5-dihydro-thiazolo[4,5-h]quinazolin-2-yl}-acetamid

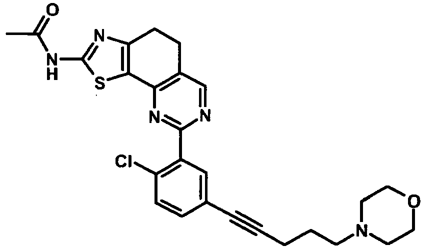

5.0 g (10.4 mmol) N-[8-(2-Chlor-5-iod-phenyl)-4,5-dihydro-thiazolo[4,5-h]quinazolin-2-yl]-acetamid werden unter Argon-Atmosphäre in 100 mL Tetrahydrofuran vorgelegt und mit 3.5 g (41.4 mmol) 4-Pentin-1-ol und 6.7 ml (41.4 mmol) Diisopropylethylamin versetzt. Das Gemisch wird sauerstofffrei gehalten und 727 mg (1.0 mmol) Triphenylphosphinpalladium(II)-chlorid und 197 mg (1.0 mmol) Kupfer(I)-iodid zugesetzt. Es wird 2.5 Stunden bei Raumtemperatur gerührt. Nach Abkühlen auf Raumtemperatur wird das Reaktionsgemisch eingeengt und mit Dichlormethan verrührt. Der orangefarbene Niederschlag wird abgesaugt und getrocknet. Ausbeute: 5.2 g

5.2 g (10.3 mmol) der zuvor beschriebenen Zwischenstufe, 3.9 mL (28.1 mmol) Triethylamin und 40 mg 4-Dimethylaminpyridin in 40 mL THF wird bei 0 °C mit 1.8 mL (23.4 mmol) Methansulfonylchlorid versetzt und über Nacht bei Raumtemperatur gerührt. Die Reaktionsmischung wird eingeengt und in wässrigem Ammoniak und Dichlormethan aufgenommen. Die organische Phase wird über Aktivkohle filtriert, getrocknet und eingeengt. Der Rückstand wird mittels MPLC (Dichlormethan/Methanol 100:5) gereinigt. Ausbeute: 2.2 g farbloses Öl.

80 mg (0.16 mmol) der zuvor beschriebenen Zwischenstufe und 40 mg (0.46 mmol) Morpholin in 1 mL DMF werden über Nacht bei Raumtemperatur gerührt und anschließend für sechs Stunden auf 70 °C erwärmt. Die Reaktionsmischung wird ohne weitere Aufarbeitung mittels RP-HPLC gereinigt. Ausbeute: 58 mg gelber Feststoff (MH+ = 508; RT = 2.46; Methode A)

**Tabelle 9:**

| | | | | | | |
|---|---|---|---|---|---|---|
| 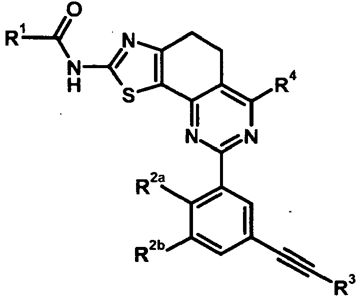 | | | | | | |

| **Beispiel** | **R¹** | **R^{2a}** | **R^{2b}** | **R⁴** | **R³** | **Analytik HPLC-MS** |
|---|---|---|---|---|---|---|
| 174 | H₃C-X₁ | 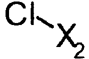 | H | H | 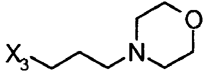 | MH+ = 508 |
| | | | | | | RT = 2.46 |
| | | | | | | Methode A |
| 175 | H₃C-X₁ | 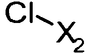 | H | H | 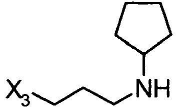 | MH+ = 506 |
| | | | | | | RT = 2.62 |
| | | | | | | Methode A |
| 176 | H₃C-X₁ | 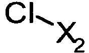 | H | H | 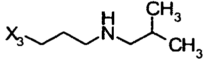 | MH+ = 494 |
| | | | | | | RT = 2.61 |
| | | | | | | Methode A |
| 177 | H₃C-X₁ | 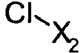 | H | H | 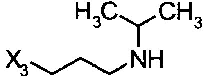 | MH+ = 480 |
| | | | | | | RT = 2.51 |
| | | | | | | Methode A |
| 178 | H₃C-X₁ | 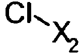 | H | H | 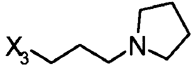 | MH+ = 492 |
| | | | | | | RT = 2.52 |
| | | | | | | Methode A |
| 179 | H₃C-X₁ | 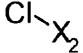 | H | H | 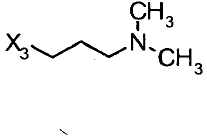 | MH+ = 466 |
| | | | | | | RT = 2.45 |
| | | | | | | Methode A |
| 180 | H₃C-X₁ | 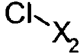 | H | H | 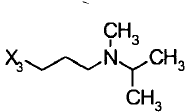 | MH+ = 494 |
| | | | | | | RT = 2.53 |
| | | | | | | Methode A |
| 181 | H₃C-X₁ | 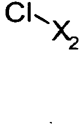 | H | H | 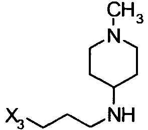 | MH+ = 535 |
| | | | | | | RT = 2.29 |
| | | | | | | Methode A |
| 182 | H₃C-X₁ | 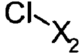 | H | H | 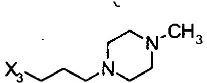 | MH+ = 521 |
| | | | | | | RT = 2.29 |
| | | | | | | Methode A |
| 183 | H₃C-X₁ | 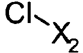 | H | H | 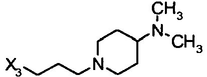 | MH+ = 549 |
| | | | | | | RT = 2.31 |
| | | | | | | Methode A |

### BIOLOGISCHER TEST

Die beispielhaft genannten Verbindungen der Formel **(I)** zeichnen sich durch eine Affinität zur PI3-Kinase, d.h. im Test durch einen IC₅₀-Wert von unter 600 nmol/Liter aus.

Um die inhibitorische Aktivität der Verbindungen auf die PI3Kγ ermitteln zu können, wurde das im folgenden beschriebene *in-vitro* Kinase Assay angewandt. Die Expression und Reinigung von Gβ₁γ₂-His und p101-GST/p110γ aus Sf9-Zellen (spodoptera frugiperda 9) wurde schon früher beschrieben (Maier et al., J. Biol. Chem. 1999 (274) 29311-29317). 10 µl der zu testenden Verbindung wurden auf 96 well PVDF-Filterplatten (0.45 µM) vorgelegt und für 20 min mit 30 µl Lipidvesikeln (PIP₂ (0.7 µg/well), Phosphatidylethanolamin (7.5 µg/well), Phosphatidylserin (7.5 µg/well), Sphingomyelin (0.7 µg/well) and Phosphatidylcholin (3.2 µg/well)) inkubiert, die 1-3 ng PI3Kγ und 20-60 ng Gβ₁γ₂-His enthielten. Durch Zugabe von 10 µl Reaktionspuffer (40 mM Hepes, pH 7,5, 100 mM NaCl, 1 mM EGTA, 1 mM β-Glycerophosphate, 1mM DTT, 7 mM MgCl₂ and 0.1 % BSA; 1µM ATP und 0.2 pCi [γ-³³P]-ATP) wurde die Reaktion gestartet und für 120 min bei Raumtemperatur inkubiert. Die Reaktionslösung wurde durch Anlegen eines Vakuums durch die Filter gesaugt und mit 200 µl PBS nachgewaschen. Nach dem Trocknen der Platten bei 50°C wurde die in den Platten verbliebene Radioaktivität nach Zugabe von 50 µl Scintillationsflüssigkeit mit Hilfe eines Top-Count Messgeräts bestimmt.

### INDIKATIONSGEBIETE

Wie gefunden wurde, zeichnen sich die Verbindungen der Formel **(I)** durch vielfältige Anwendungsmöglichkeiten auf therapeutischem Gebiet aus. Hervorzuheben sind solche Anwendungsmöglichkeiten, für welche die erfindungsgemäßen Verbindungen der Formel **(I)** aufgrund ihrer pharmazeutischen Wirksamkeit als PI3-Kinase Modulator bevorzugt zur Anwendung gelangen können.

Allgemein ausgedrückt sind dies Erkrankungen, bei deren Pathologie eine Aktivität von PI3-Kinasen beteiligt ist, insbesondere entzündliche und allergische Erkrankungen. Insbesondere seien entzündliche und allergische Atemwegserkrankungen, entzündliche Erkrankungen des Magen-Darm Traktes, entzündliche Erkrankungen des Bewegungsapparates, entzündliche und allergische Hauterkrankungen, entzündliche Augenerkrankungen, Erkrankungen der Nasenschleimhaut, entzündliche oder allergische Krankheitszustände, bei denen Autoimmun-Reaktionen beteiligt sind oder Nierenentzündungen genannt. Die Behandlung kann dabei symptomatisch, adaptiv, kurativ oder präventiv erfolgen.

Bevorzugt genannte Atemwegserkrankungen wären hierbei chronische und/oder obstruktive Atemwegserkrankungen. Die erfindungsgemäßen Verbindungen der Formel **(I)** können dabei, auf Grund ihrer pharmakologischen Eigenschaften eine Reduzierung der
● Gewebezerstörung
● der Entzündung der Atemwege
● der bronchialen Hyperreaktivität
● des Umbauprozesses der Lunge infolge der Entzündung
● der Verschlechterung der Krankheit (Progression)
bewirken. Besonders bevorzugt sind die erfindungsgemäßen Verbindungen zur Herstellung eines Medikaments zur Behandlung von Chronischer Bronchitis, akuter Bronchitis, Bronchitis aufgrund bakterieller oder viraler Infektion oder Pilzen oder Helminthen, allergischer Bronchitis, toxischer Bronchitis, chronisch obstruktiver Bronchitis (COPD), Asthma (intrinsisch oder allergisch), pediatrischem Asthma, Bronchiektasien, allergischer Alveolitis, allergischer oder nicht-allergischer Rhinitis, chronischer Sinusitis, zystischer Fibrose oder Mukoviszidose, alpha-1-Antitrypsin-Mangel, Husten, Lungenemphysem, interstitieller Lungenerkrankungen wie z.B. Lungenfibrose, Asbestose und Silikose und Alveolitis; hyperreaktiver Atemwege, Nasenpolypen, Lungenödemen wie z.B. toxischem Lungenödem und ARDS / IRDS, Pneumonitis aufgrund unterschiedlicher Genese wie Strahlen-induziert oder durch Aspiration oder infektiöse, Kollagenosen wie Lupus erythematodes, systemische Sklerodermie, Sarkoidose oder M. Boeck.

Ebenfalls geeignet sind die Verbindungen der Formel **(I)** zur Behandlung von Erkrankungen der Haut, wie z.B. Psoriasis, Kontakt-Dermatitis, atopische Dermatitis, Alopecia areata (kreisrunder Haarausfall), Erythema exsudativum multiforme (Stevens-Johnson-Syndrom), Dermatitis herpetiformis, Sklerodermie, Vitiligo, Nesselsucht (Urticaria), Lupus erythematodes, follikuläre und flächenhafte Pyodermien, endogene und exogene Akne, Akne rosacea sowie andere entzündliche oder allergische oder proliferative Hauterkrankungen.

Weiterhin sind die Verbindungen der Formel **(I)** zum therapeutischen Einsatz geeignet bei entzündlichen oder allergischen Krankheitszuständen, bei denen Autoimmun-Reaktionen beteiligt sind, wie z.B. entzündliche Darmerkrankungen, z.B. Morbus Crohn oder Colitis ulzerosa; Krankheiten aus dem Formenkreis der Arthritis, wie z.B. rheumatoide oder psoriatrische Arthritis, Osteoarthritis, rheumatoide Spondylitis und andere arthritische Zustände oder Multiple Sklerose.

Ferner seien folgende allgemeine entzündliche oder allergische Erkrankungen genannt, die sich durch Medikamente beinhaltend Verbindungen der Formel **(I)** behandeln lassen:
● Entzündungen am Auge, wie z.B. Bindehautentzündung (Konjunktivitis) verschiedener Arten, wie z.B. durch Infektionen mit Pilzen oder Bakterien, allergische Konjunktivitis, Reizkonjunktivitis, durch Medikamenten induzierten Konjunktivitis, Keratitis, Uveitis
● Krankheiten der Naseschleimhaut, wie z.B. allergische Rhinitis/Sinusitis oder Nasenpolypen
● Entzündliche oder allergische Krankheitszustände, wie z.B. systemische Lupus erythematodes, chronische Hepatitis, Nierenentzündungen, wie Glomerulonephritis, interstitielle Nephritis oder idiopathisches nephrotisches Syndrom.

Als weitere Krankheiten, die auf Grund der pharmakologischen Wirksamkeit der Verbindungen der Formel **(I)** mit einem Medikament selbige beinhaltend behandelt werden können, seien toxische oder septische Schocksyndrome, Atherosklerose, Mittelohrentzündung, (Otitis media), Hypertrophie des Herzens, Herzinsuffizienz, Schlaganfall, Ischämie-Reperfusionsschäden oder neurodegenerative Erkrankungen wie Parkinson'sche Krankheit oder Alzheimer genannt.

### KOMBINATIONEN

Die Verbindungen der Formel **(I)** können allein oder in Kombination mit anderen Wirkstoffen der Formel **(I)** zur Anwendung gelangen. Gegebenenfalls können die Verbindungen der Formel **(I)** auch in Kombination mit **W** eingesetzt werden, worin **W** einen pharmakologisch, aktiven Wirkstoff darstellt und beispielsweise ausgewählt ist, aus der Gruppe bestehend aus Betamimetika, Anticholinergika, Corticosteroiden, PDE4-Inhibitoren, LTD4-Antagonisten, EGFR-Hemmem, Dopamin-Agonisten, H1-Antihistaminika, PAF-Antagonisten und PI3-Kinase Inhibitoren, vorzugsweise PI3-δ-Kinase Inhibitoren. Weiterhin können zwei- oder dreifach Kombinationen von **W** mit den Verbindungen der Formel **(I)** kombiniert werden. Beispielhaft genannte Kombinationen von **W** wären:
- **W** stellt ein Betamimetikum dar, kombiniert mit einem Wirkstoff ausgewählt aus der Gruppe bestehend aus Anticholinergika, Corticosteroide, PDE4-Inhibitore, EGFR-Hemmern und LTD4-Antagonisten,
- **W** stellt ein Anticholinergikum dar, kombiniert mit einem Wirkstoff ausgewählt aus der Gruppe bestehend aus Betamimetika, Corticosteroiden, PDE4-Inhibitoren, EGFR-Hemmern und LTD4-Antagonisten,
- **W** stellt ein Corticosteroid dar, kombiniert mit einem Wirkstoff ausgewählt aus der Gruppe bestehend aus einem PDE4-Inhibitoren, EGFR-Hemmern und LTD4-Antagonisten
- **W** stellt einen PDE4-Inhibitor dar, kombiniert mit einem Wirkstoff ausgewählt aus der Gruppe bestehend aus einem EGFR-Hemmern und LTD4-Antagonisten
- **W** stellt einen EGFR-Hemmer dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluoro-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chloro-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl}-2-(tert.-butylamino)ethanol
gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodid und Methansulfonat besonders bevorzugt. Weiterhin genannte Verbindungen sind:
- 2,2-Diphenylpropionsäuretropenolester-methobromid
- 2,2-Diphenylpropionsäurescopinester-methobromid
- 2-Fluor-2,2-Diphenylessigsäurescopinester-methobromid
- 2-Fluor-2,2-Diphenylessigsäuretropenolester-methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester -Methobromid
- 9-Fluor-fluoren-9-carbonsäuretropenolester -Methobromid
- 9-Hydroxy-fluoren-9-carbonsäurescopinester -Methobromid
- 9-Fluor-fluoren-9-carbonsäurescopinester Methobromid
- 9-Methyl-fluoren-9-carbonsäuretropenolesterMethobromid
- 9-Methyl-fluoren-9-carbonsäurescopinesterMethobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinesterMethobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester -Methobromid
- 4,4'-Difluorbenzilsäuremethylestercyclopropyltropinester-Methobromid
- 9-Hydroxy-xanthen-9-carbonsäuretropenolester -Methobromid
- 9-Hydroxy-xanthen-9-carbonsäurescopinester Methobromid
- 9-Methyl-xanthen-9-carbonsäuretropenolester -Methobromid
- 9-Methyl-xanthen-9-carbonsäurescopinesterMethobromid
- 9-Ethyl-xanthen-9-carbonsäuretropenolester Methobromid
- 9-Difluormethyl-xanthen-9-carbonsäuretropenolester -Methobromid
- 9-Hydroxymethyl-xanthen-9-carbonsäurescopinester -Methobromid

Als Corticosteroide gelangen hierbei vorzugsweise Verbindungen zur Anwendung, die ausgewählt sind aus der Gruppe bestehend aus Prednisolon, Prednison, Butixocortpropionat, Flunisolid, Beclomethason, Triamcinolon, Budesonid, Fluticason, Mometason, Ciclesonid, Rofleponid, Dexamethason, Betamethason, Deflazacort, RPR-106541, NS-126, ST-26 und
- 6,9-Difluoro-17-[(2-furanylcarbonyl)oxy]-11-hydroxy-16-methyl-3-oxo-androsta-1,4-dien-17-carbothionsäure (S)-fluoromethylester
- 6,9-Difluoro-11-hydroxy-16-methyl-3-oxo-17-propionyloxy-androsta-1,4-dien-17-carbothionsäure (S)-(2-oxo-tetrahydro-furan-3S-yl)ester,
- Etiprednol-dichloroacetat
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, A-riflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, CI-1018, CDC-801, CDC-3052, D-22888, YM-58997, Z-15370 und
- N-(3,5-Dichloro-1-oxo-pyridin-4-yl)-4-difluoromethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Bromobenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluoromethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluoro-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorothieno[3,2-b]pyridin-5-yl)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure
gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl}-1-oxo-2-buten-1-yl]amino}-7-cyclopropylmethoxy-chinazolin
- 4-[(3-Chlor-4-fluor-phenyl)amino]-6g[4-((RF)-6-methyl-2-oxo-morpholin-4-yl)-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinyl-carbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy}-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino)-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-((3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazotin
- 4-[(3-Chlor-4-fluor-phenyl)amino]-6-(cis-4-{N-[(tetrahydropyran-4-yl)carbonyl]-N-methyl-amino)-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy)-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy)-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy)-7-methoxy-chinazolin
- 4-[(3-Chlör-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als PAF-Antagonisten gelangen hierbei vorzugsweise Verbindungen zur Anwendung, die ausgewählt sind aus der Gruppe bestehend aus
- 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin
- 6-(2-Chlorphenyl)-8,9-dihydro-1-methyl-8-[(4-morpholinyl)carbonyl]-4H,7H-cyclopenta-[4,5]thieno-[3,2-f][1,2,4]triazolo[4,3-a][1,4]diazepin
gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.
Als PI3-Kinase-δ -Inhibitoren gelangen vorzugsweise Verbindungen zur Anwendung, die ausgewählt sind aus der Gruppe bestehend aus:
IC87114, 2-(6-aminopurin-9-ylmethyl)-3-(2-chlorophenyl)-6,7-dimethoxy-3H-quinazolin- 4-one; 2-(6-aminopurin-o-ylmethyl)-6-bromo-3-(2-chlorophenyl)-3H-quinazolin-4-one; 2-(6-aminopurin-o-ylmethyl)-3-(2-chlorophenyl)-7-fluoro-3H-quinazol in-4-one; 2-(6-aminopurin-9-ylmethyl)-6-chloro-3-(2-chlorophenyl)-3H-quinazolin-4-one; 2-(6-aminopurin-9-ylmethyl)-3-(2-chlorophenyl)-5-fluoro-3H-quinazolin-4-one; 2-(6-aminopurin-o-ylmethyl)-5-chloro-3-(2-chloro-phenyl)-3H-quinazolin-4-one; 2-(6-aminopurin-9-ylmethyl)-3-(2-chlorophenyl)-5-methyl-3H-quinazolin-4-one; 2-(6-aminopurin-9-ylmethyl)-8-chloro-3-(2-chlorophenyl)-3H-quinazolin-4-one; 2-(6-aminopurin-9-ylmethyl)-3-biphenyl-2-yl-5-chloro-3H-quinazolin-4-one;5-chloro-2-(9H-purin-6-ylsulfanylmethyl)-3-o-tolyl-3H-quinazolin-4-one; 5-chloro-3-(2-fluorophenyl)-2-(9H-purin-6-yl-sulfanylmethyl)-3H-quinazolin-4- one; 2-(6-aminopurin-9-ylmethyl)-5-chloro-3-(2-fluorophenyl)-3H-quinazolin-4-one; 3-biphenyl-2-yl-5-chloro-2-(9H-purin-6-ylsulfanylmethyl)-3H-quinazolin-4-one; 5-chloro-3-(2-methoxyphenyl)-2-(9H-purin-6-yl-sulfanylmethyl)-3H-quinazolin-4-one; 3-(2-chlorophenyl)-5-fluoro-2-(9H-purin-6-yl-sulfanylmethyl)-3H-quinazolin-4-one; 3-(2-chlorophenyl)-6,7-dimethoxy-2-(9H-purin-6-yl-sulfanylmethyl)-3H-quinazolin-4-one; 6-bromo-3-(2-chlorophenyl)-2-(9H-purin-6-yl-sulfanylmethyl)-3H-quinazolin-4-one; 3-(2-chlorophenyl)-8-trifluoromethyl-2-(9H-purin-6-ylsulfanylmethyl)-3H-quinazolin-4-one; 3-(2-chlorophenyl)-2-(9H-purin-6-ylsulfanylmethyl)-3H-benzo[g]quinazolin-4-one; 6-chloro-3-(2-chlorophenyl)-2-(9H-purin-6-yl-sulfanylmethyl)-3H-quinazolin-4-one; 8-chloro-3-(2-chlorophenyl)-2-(9H-purin-6-yl-sulfanylmethyl)-3H-quinazolin-4-one; 3-(2-ch)orophenyl)-7-fluoro-2-(9H-purin-6-yl-sulfanylmethyl)-3H-quinazolin-4-one; 3-(2-chlorophenyl)-7-nitro-2-(9H-purin-6-yl-sulfanylmethyl)-3H-quinazolin-4-one; 3-(2-chlorophenyl)-6-hydroxy-2-(9H-purin-6-yl-sulfanylmethyl)-3H-quinazolin-4-one; 5-chloro-3-(2-chlorophenyl)-2-(9H-purin-6-yl-sulfanylmethyl)-3H-quinazolin-4- one; 3-(2-chlorophenyl)-5-methyl-2-(9H-purin-6-yl-sulfanylmethyl)-3H-quinazolin-4-one; 3-(2-chlorophenyl)-6,7-difluoro-2-(9H-purin-6-yl-sulfanylmethyl)-3H-quinazolin-4-one; 3-(2-chlorophenyl)-6-fluoro-2-(9H-purin-6-yl-sulfanylmethyl)-3H-quinazolin-4-one; 2-(6-aminopurin-9-ylmethyl)-3-(2-isopropylphenyl)-5-methyl-3H-quinazolin-4-one; 2-(6-aminopurin-9-ylmethyl)-5-methyl-3-o-tolyl-3H-quinazolin-4-one; 3-(2-fluorophenyl)-5-methyl-2-(9H-purin-6-yl-sulfanylmethyl)-3H-quinazolin-4-one;2-(6-aminopurin-9-ylmethyl)-5-chloro-3-o-tolyl-3H-quinazolin-4-one; 2-(6-aminopurin-9-ylmethyl)-5-chloro-3-(2-methoxy-phenyl)-3H-quinazolin-4- one; 2-(2-amino-9H-purin-6-ylsulfanylmethyl)-3-cyclopropyl-5-methyl-3H-quinazolin-4-one; 3-cyclopropylmethyl-5-methyl-2-(9H-purin-6-ylsulfanylmethyl)-3H-quinazolin-4-one; 2-(6-aminopurin-9-ylmethyl)-3-cyclopropylmethyl-5-methyl-3H-quinazolin-4-one; 2-(2-amino-9H-purin-6-ylsulfanylmethyl)-3-cyclopropylmethyl-5-methyl-3H-quinazolin-4-one; 5-methyl-3-phenethyl-2-(9H-purin-6-ylsulfanylmethyl)-3H-quinazolin-4-one; 2-(2-amino-9H-purin-6-ylsulfanylmethyl)-5-methyl-3-phenethyl-3H-quinazolin-4-one; 3-cyclopentyl-5-methyl-2-(9H-purin-6-ylsulfanylmethyl)-3H-quinazolin-4-one;2-(6-aminopurin-9-ylmethyl)-3-cyclopentyl-5-methyl-3H-quinazolin-4-one; 3-(2-chloropyridin-3-yl)-5-methyl-2-(9H-purin-6-ylsulfanylmethyl)-3H- quinazolin-4-one; 2-(6-aminopurin-9-ylmethyl)-3-(2-chloropyridin-3-yl)-5-methyl-3H-quinazolin-4-one; 3-methyl-4-[5-methyl-4-oxo-2-(9H-purin-6-ylsulfanylmethyl)-4H-quinazolin-3-yl]-benzoic acid; 3-cyclopropyl-5-methyl-2-(9H-purin-6-ylsulfanylmethyl)-3H-quinazolin-4-one; 2-(6-aminopurin-9-ylmethyl)-3-cyclopropyl-5-methyl-3H-quinazolin-4-one; 5-methyl-3-(4-nitrobenzyl)-2-(9H-purin-6-ylsulfanylmethyl)-3H-quinazolin-4-one; 3-cyclohexyl-5-methyl-2-(9H-purin-6-ylsulfanylmethyl)-3H-quinazolin-4-one; 2-(6-aminopurin-9-ylmethyl)-3-cyclohexyl-5-methyl-3H-quinazolin-4-one; 2-(2-amino-9H-purin-6-ylsulfanylmethyl)-3-cyclo-hexyl-5-methyl-3H-quinazolin-4-one; 5-methyl-3-(E-2-phenylcyclopropyl)-2-(9H-purin-6-ylsulfanylmethyl)-3H-quinazolin-4-one; 3-(2-chlorophenyl)-5-fluoro-2-[(9H-purin-6-ylamino)methyl]-3H-quinazolin-4-one; 2-[(2-amino-9H-purin-6-ylamino)methyl]-3-(2-chlorophenyl)-5-fluoro-3H-quinazolin-4-one; 5-methyl-2-[(9H-purin-6-ylamino)methyl]-3-o-tolyl-3H-quinazolin-4-one; 2-[(2-amino-9H-purin-6-ylamino)methyl]-5-methyl-3-o-tolyl-3H-quinazolin-4- one; 2-[(2-fluoro-9H-purin-6-ylamino)methyl]-5-methyl-3-o-tolyl-3H-quinazolin-4-one; (2-chlorophenyl)-dimethylamino-(9H-purin-6-ylsulfanylmethyl)-3H-quinazolin-4-one; 5-(2-benzyfoxyethoxy)-3-(2-chlorophenyl)-2-(9H-purin-6-ylsulfanylmethyl)-3H-quinazolin-4-one; 6-aminopurine-9-carboxylic acid 3-(2-chlorophenyl)-5-fluoro-4-oxo-3,4-dihydro-quinazolin-2-ylmethyl ester; N-[3-(2-chlorophenyl)-5-fluoro-4-oxo-3,4-dihydro-quinazolin-2-ylmethyl]-2-(9H-purin-6-ylsulfanyl)-acetamide; 2-[1-(2-fluoro-9H-purin-6-ylamino)ethyl]-5-methyl-3-o-tolyl-3H-quinazolin-4- one; 5-methyl-2-[1-(9H-purin-6-ylamino)ethyl]-3-o-tolyl-3H-quinazolin-4-one; 2-(6-dimethylaminopurin-9-ylmethyl)-5-methyl-3-o-tolyl-3H-quinazolin-4-one; 5-methyl-2-(2-methyl-6-oxo-1,6-dihydro-purin-7-ylmethyl)3-o-tolyl-3H-quinazolin-4-one; 5-methyl-2-(2-methyl-6-oxo-1,6-dihydro-purin-9-ylmethyl)-3-o-tolyl-3H- quinazolin-4-one; 2-(amino-dimethylaminopurin-9-ylmethyl)-5-methyl-3-o-tolyl-3H-quinazolin-4-one; 2-(2-amino-9H-purin-6-ylsulfanylmethyl)-5-methyl-3-o-tolyl-3H-quinazolin-4-one; 2-(4-amino-1,3,5-triazin-2-ylsulfanylmethyl)-5-methyl-3-o-tolyl-3H-quinazolin- 4-one; 5-methyl-2-(7-methyl-7H-purin-6-ylsulfanylmethyl)-3-o-tolyl-3H-quinazolin-4-one; 5-methyl-2-(2-oxo-1,2-dihydro-pyrimidin-4-ylsulfanylmethyl)-3-o-tolyl-3H-quinazolin-4-one; 5-methyl-2-purin-7-ylmethyl-3-o-tolyl-3H-quinazolin-4-one;5-methyl-2-purin-9-ylmethyl-3-o-tolyl-3H-quinazolin-4-one; 5-methyl-2-(9-methyl-9H-purin-6-ylsulfanylmethyl)-3-o-tolyl-3H-quinazolin-4-one; 2-(2,6-diamino-pyrimidin-4-ylsulfanylmethyl)-5-methyl-3-o-tolyl-3H-quinazolin-4-one; 5-methyl-2-(5-methyl-[1,2,4]triazolo[1,5-a]pyrimidin-7-ylsulfanylmethyl)-3-0-tolyl-3H-quinazolin-4-one; 5-methyl-2-(2-methylsulfanyl-9H-purin-6-ylsulfanylmethyl)-3-o-tolyl-3H-quinazolin-4-one; 2-(2-hydroxy-9H-purin-6-ylsulfanylmethyl)-5-methyl-3-o-tolyl-3H-quinazolin-4-one; 5-methyl-2-(1-methyl-1H-imidazol-2-ylsulfanylmethyl)-3-o-tolyl-3H-quinazolin-4-one; 5-methyl-3-0-tolyl-2-( H-[1,2,4]triazol-3-ylsulfanylmethyl)-3H-quinazolin-4-one; 2-(2-amino-6-chloro-purin-9-ylmethyl)-5-methyl-3-o-tolyl-3H-quinazolin-4-one; 2-(6-aminopurin-7-ylmethyl)-5-methyl-3-o-tolyl-3H-quinazolin-4-one; 2-(7-amino-1,2,3-triazolo[4,5-d]pyrimidin-3-yl-methyl)-5-methyl-3-o-tolyl-3H- quinazolin-4-one; 2-(7-amino-1,2,3-triazolo[4, 5-d]pyrimidin-1-yl-methyl)-5-methyl-3-o-tolyl-3H-quinazolin-4-one; 2-(6-amino-9H-purin-2-ylsulfanylmethyl)-5-methyl-3-o-tolyl-3H-quinazolin-4- one; 2-(2-amino-6-ethylamino-pyrimidin-4-ylsulfanylmethyl)-5-methyl-3-o-tolyl-3H-quinazolin-4-one; 2-(3-amino-5-methylsulfanyl-1,2,4-triazol-1-yl-methyl)-5-methyl-3-o-tolyl-3Hquinazolin-4-one; 2-(5-amino-3-methylsulfanyl-1,2,4-triazol-1-ylmethyl)-5-methyl-3-o-tolyl-3H- quinazolin-4-one; 5-methyl-2-(6-methylaminopurin-9-ylmethyl)-3-o-tolyl-3H-quinazolin-4-one; 2-(6-benzylaminopurin-9-yl methyl)-5-methyl-3-o-tolyl-3H-quinazol in-4-one; 2-(2,6-diaminopurin-9-ylmethyl)-5-methyl-3-o-tolyl-3H-quinazolin-4-one; 5-methyl-2-(9H-purin-6-ylsulfanylmethyl)-3-o-tolyl-3H-quinazolin-4-one; 3-isobutyl-5-methyl-2-(9H-purin-6-ylsulfanylmethyl)-3H-quinazolin-4-one; N-{2-[5-Methyl-4-oxo-2-(9H-purin-6-ylsulfanylmethyl)-4H-quinazolin-3-yl]-phenyl}-acetamide; 5-methyl-3-(E-2-methylcyclohexyl)-2-(9H-purin-6-ylsulfanylmethyl)-3H-quinazolin-4-one; 2-[5-methyl-4-oxo-2-(9H-purin-6-ylsulfanylmethyl)-4H-quinazolin-3-yl]-benzoic acid; 3-{2-[(2-dimethylaminoethyl)methylamino]phenyl}-5-methyl-2-(9H-purin-6-ylsulfanylmethyl)-3H-quin-azolin-4-one; 3-(2-chlorophenyl)-5-methoxy-2-(9H-purin-6-ylsulfanylmethyl)-3H-quinazolin- 4-one; 3-(2-chlorophenyl)-5-(2-morpholin-4-yl-ethylamino)-2-(9H-purin-6- ylsul-fanylmethyl)-3H-quinazolin-4-one; 3-benzyl-5-methoxy-2-(9H-purin-6-ylsulfanylmethyl)-3H-quinazolin-4-one; 2-(6-aminopurin-9-ylmethyl)-3-(2-benzyloxyphenyl)-5-methyl-3H-quinazolin-4-one; 2-(6-aminopurin-9-ylmethyl)-3-(2-hydroxyphenyl)-5-methyl-3H-quinazolin-4-one; 2-(1-(2-amino-9H-purin-6-ylamino)ethyl)-5-methyl-3-o-tolyl-3H-quinazolin-4-one; 5-methyl-2-[1-(9H-purin-6-ylamino)propyl]-3-o-tolyl-3H-quinazolin-4-one; 2-(1-(2-fluoro-9H-purin-6-ylamino)propyl)-5-methyl-3-o-tolyl-3H-quinazolin-4- one; 2-(1-(2-amino-9H-purin-6-ylamino)propyl)-5-methyl-3-o-tolyl-3H-quinazolin-4-one; 2-(2-benzyloxy-1-(9H-purin-6-ylamino)ethyl)-5-methyl-3-o-tolyl-3H-quinazolin- 4-one; 2-(6-aminopurin-9-ylmethyl)-5-methyl-3-{2-(2-(1-methylpyrrolidin-2-yl)-ethoxy)-phenyl}-3H-quinazolin-4-one; 2-(6-aminopurin-9-ylmethyl)-3-(2-(3-dimethylamino-propoxy)-phenyl)-5-methyl-3H-quinazolin-4-one; 2-(6-aminopurin-9-ylmethyl)-5-methyl-3-(2-prop-2-ynyloxyphenyl)-3H-quinazolin-4-one; 2-(2-(1-(6-aminopurin-9-ylmethyl)-5-methyl-4-oxo-4H-quinazol in-3-yl]-phenoxy}-acetamide; 5-chloro-3-(3,5-d ifluoro-phenyl)-2-[1-(9H-purin-6-ylamino)-propyl]-3H- quinazolin-4-one; 3-phenyl-2-[1-(9H-purin-6-ylamino)-propyl]-3H-quinazolin-4-one; 5-fluoro-3-phenyl-2-[1-(9 H-pu ri n-6-ylami no)-propyl]-3H-quinazolin-4-one; 3-(2,6-difluoro-phenyl)-5-methyl-2-[1-(9H-purin-6-ylamino)-propyl]-3H-quinazolin-4-one; 6-fluoro-3-phenyl-2-[1-(9H-purin-6-ylamino)-ethyl]-3H-quinazolin-4-one; 3-(3,5-difluoro-phenyl)5-methyl-2-[1-(9H-purin-6-ylamino)-ethyl]-3H-quinazolin-4-one; 5-fluoro-3-phenyl-2-[1-(9H-purin-6-ylamino)-ethyl]-3H-quinazolin-4-one; 3-(2,3-difluoro-phenyl)-5-methyl-2-[1-(9H-purin-6-ylamino)-ethyl]-3H-quinazolin-4-one; 5-methyl-3-phenyl-2-[1-(9H-purin-6-ylamino)-ethyl]-3H-quinazolin-4-one; 3-(3-chloro-phenyl)-5-methyl-2-[1-(9H-purin-6-ylamino)-ethyl]-3H-quinazolin-4-one; 5-methyl-3-phenyl-2-[(9H-purin-6-ylamino)-methyl]-3H-quinazolin-4-one; 2-[(2-amino-9H-purin-6-ylamino)-methyl]-3-(3,5-difluoro-phenyl)-5-methyl-3H-quinazolin-4-one; 3-{2-[(2]-diethylamino-ethyl)-methyl-amino]-phenyl)-5-methyl-2-[(9H-purin-6- ylamino)-methyl]-3H-quinazolin-4-one; 5-chloro-3-(2-fluoro-phenyl)-2-[(9H-purin-6-ylaminormethyl]-3H-quinazolin-4-one; 5-chloro-2-[(9H-purin-6-ylamino)-methyl]-3-o-tolyl-3H-quinazolin-4-one; 5-chloro-3-(2-chloro-phenyl)-2-[(9H-purin-6-ylamino)-methyl]-3H-quinazolin-4- one; 6-fluoro-3-(3-fluoro-phenyl)-2-[1-(9H-pu rin-6-ylamino)-ethyl]-3H-quinazolin-4-one; 2-[1-(2-amino-9H-purin-6-ylamino)-ethyl]-5-chloro-3-(3-fluoro-phenyl)-3H-quinazolin-4-one; und deren pharmazeutisch verträglichen Salze und Solvate.

### FORMULIERUNGEN

Die erfindungsgemäßen Verbindungen können oral, transdermal, inhalativ, parenteral oder sublingual verabreicht werden. Die erfindungsgemäßen Verbindungen liegen hierbei als aktive Bestandteile in üblichen Darreichungsformen vor, beispielsweise in Zusammensetzungen, die im wesentlichen aus einem inerten pharmazeutischen Träger und einer effektiven Dosis des Wirkstoffs bestehen, wie beispielsweise Tabletten, Dragees, Kapseln, Oblaten, Pulver, Lösungen, Suspensionen, Emulsionen, Sirupe, Suppositorien, transdermale Systeme etc.. Eine wirksame Dosis der erfindungsgemäßen Verbindungen liegt bei einer oralen Anwendung zwischen 0.1 und 5000, vorzugsweise zwischen 1 und 500, besonders bevorzugt zwischen 5-300 mg/Dosis, bei intravenöser, subcutaner oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern, ethanolischen oder wässrigen Lösungen bevorzugt. Gleichfalls ist es möglich, die erfindungsgemäßen Verbindungen als Infusionslösung, vorzugsweise in einer physiologischen Kochsalzlösung oder Nährsalzlösung einzusetzen.

Die erfindungsgemäßen Verbindungen können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Eine therapeutisch wirksame Tagesdosis beträgt zwischen 1 und 2000 mg, bevorzugt 10-500 mg pro Erwachsener

Die nachfolgenden Beispiele illustrieren die vorliegende Erfindung ohne sie jedoch in ihrem Umfang zu beschränken:

### Pharmazeutische Formulierungsbeispiele

| A) | Tabletten | pro Tablette | |
|---|---|---|---|
| | Wirkstoff | 100 | mg |
| | Milchzucker | 140 | mg |
| | Maisstärke | 240 | mg |
| | Polyvinylpyrrolidon | 15 | mg |
| | Magnesiumstearat | 5 | mg |
| | | 500 | mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpresst.

| B) | Tabletten | pro Tablette | |
|---|---|---|---|
| | Wirkstoff | 80 | mg |
| | Maisstärke | 190 | mg |
| | Milchzucker | 55 | mg |
| | Mikrokristalline Cellulose | 35 | mg |
| | Polyvinylpyrrolidon | 15 | mg |
| | Natrium-carboxymethylstärke | 23 | mg |
| | Magnesiumstearat | 2 | mg |
| | | 400 | mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

| C) | Dragées | pro Dragée | |
|---|---|---|---|
| | Wirkstoff | 5 | mg |
| | Maisstärke | 41,5 | mg |
| | Milchzucker | 30 | mg |
| | Polyvinylpyrrolidon | 3 | mg |
| | Magnesiumstearat | 0,5 | mg |
| | | 80 | mg |

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekeme mit einem Durchmesser von 6 mm gepresst. Die so hergestellten Dragéekerne werden auf bekannte Weise mit einer Schicht überzogen, die im wesentlichsten aus Zucker und Talkum besteht. Die fertigen Dragées werden mit Wachs poliert.

| D) | Kapseln | pro Kapsel | |
|---|---|---|---|
| | Wirkstoff | 50 | mg |
| | Maisstärke | 268,5 | mg |
| | Magnesiumstearat | 1.5 | mg |
| | | 320 | mg |

Substanz und Maisstärke werden gemischt und mit Wasser befeuchtet. Die feuchte Masse wird gesiebt und getrocknet. Das trockene Granulat wird gesiebt und mit Magnsiumstearat gemischt. Die Endmischung wird in Hartgelatinekapseln Größe 1 abgefüllt.

| E) | Ampullenlösung | |
|---|---|---|
| Wirkstoff | 50 | mg |
| Natriumchlorid | 50 | mg |
| Aqua pro inj. | 5 | ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| F) | Suppositorien | |
|---|---|---|
| Wirkstoff | 50 | mg |
| Adeps solidus | 1650 | mg |
| | 1700 | mg |

Das Hartfett wird geschmolzen. Bei 40°C wird die gemahlene Wirksubstanz homogen dispergiert. Es wird auf 38°C abgekühlt und in schwach vorgekühlte Suppositorienformen ausgegossen

| G) | orale Suspension | |
|---|---|---|
| Wirkstoff | 50 | mg |
| Hydroxyethylcellulose | 50 | mg |
| Sorbinsäure | 5 | mg |
| Sorbit (70%ig) | 600 | mg |
| Glycerin | 200 | mg |
| Aroma | 15 | mg |
| Wasser ad | 5 | ml |

Destilliertes Wasser wird auf 70°C erhitzt. Hierin wird unter Rühren Hydroxyethyl-cellulose gelöst. Nach Zugabe von Sorbitlösung und Glycerin wird auf Raumtemperatur abgekühlt. Bei Raumtemperatur werden Sorbinsäure, Aroma und Substanz zugegeben. Zur Entlüftung der Suspension wird unter Rühren evakuiert.
und 50 mg Wirkstoff.

## Patentansprüche

1. Verbindungen der allgemeinen Formel (I) 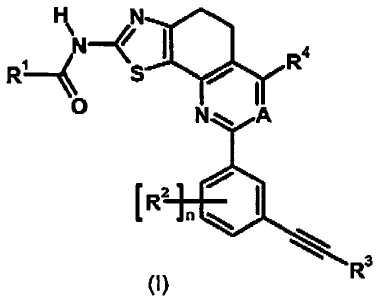 worin
A CH oder N,
n 1, 2, 3 oder 4,
R¹ Wasserstoff oder einen Rest bestehend aus C₁₋₄-Alkyl. OR^{1.1} und NR^{1.1}R^{1.2};
R^{1.1}, R^{1.2} gleich oder verschieden, H oder C₁₋₄-Alkyl; oder
NR^{1.1}R^{1.2} einen 5- bis 6- gliedrigen Heterocyclus, gegebenenfalls enthaltend ein weiteres N-Atom;
R² gleich oder verschieden, Wasserstoff oder
einen Rest ausgewählt aus der Gruppe bestehend aus F, Cl, Br, I, CN, CF₃, CF₂H, CFH₂ und NH₂;
oder
einen Rest ausgewählt aus der Gruppe bestehend aus - O-C₁₋₄-Alkyl, C₁₋₄-Alkyl und C₂₋₆-Alkenyl;
R⁴ Wasserstoff, OH, NH₂, oder
einen Rest ausgewählt aus der Gruppe bestehend aus C₁₋₆-Alkyl, C₂₋₆-Alkenyl, C₉₋₆-Cycloalkyl, -N(C₁₋₄-Alkyl)2 und -NH(C₁₋₄-Alkyl);
R³ einen Rest ausgewählt aus der Gruppe bestehend aus: 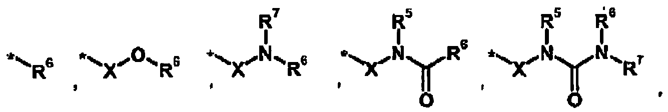 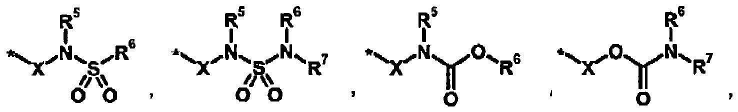 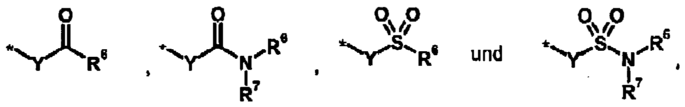 worin
X einen Rest ausgewählt aus der Gruppe bestehend aus C₁₋₆-Alkylen, C₂₋₆-Alkonylen, C₁₋₅-Alkinylen, C₃₋₇₋Cycloalkylen, C₅₋₇-Cycloalkenylen und -C₁₋₄-alkylen-C₃₋₇-cycloalkylen-;
Y eine Bindung oder X;
R⁵. R⁶, R⁷ gleich oder verschieden, Wasserstoff oder einen Rest ausgewählt aus der Gruppe bestehend aus C₁₋₆-Alkyl, C₂₋₆-Alkenyl, C₂₋₆-Alkinyl, C₁₋₆-Haloalkyl, C₃₋₈-Cycloalkyl, C₃₋₈-Cycloalkenyl, C₃₋₇-Cycloalkyl-C₁₋₄-alkyl-, Aryl, Heteroaryl, Spiro, Heterocyoloalkyl-, Aryl-C₁₋₆-alkyl-, Heteroaryl-C₁₋₆-alkyl- und Heterocycloalkyl-C₁₋₆-alkyl-,
oder
NR⁶R⁷ bilden einen fünf-, sechs- oder siebengliedrigen Ring bestehend aus,
Kohlenstoffatomen und gegebenenfalls einem Stickstoff -, Sauerstoff- oder Schwefelatom als weitere Heteroatome oder
einen Ring ausgewählt aus der Gruppe bestehend aus: 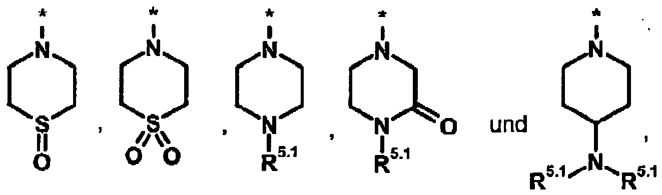
R^{5.1} gleich oder verschieden, Wasserstoff oder einen Rest ausgewählt aus der Gruppe bestehend aus C₁₋₆-Alkyl, C₃₋₆-Cycloalkyl, -CO-C₁₋₃-Alkyl und CONH₂;
oder
**R⁵** und **R⁶** bilden gemeinsam eine gesättigte oder ungesättigte Alkylen-Brücke und gegebenenfalls ein weiteres Stickstoff-, Sauerstoff- oder Schwefel-Atom enthalten kann;
oder **R³** ist gleich 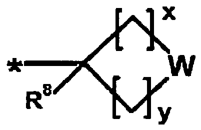
x, y gleich oder verschieden 0, 1, 2, 3, 4 oder 5;
W O, NR⁹ oder CR⁹R¹⁰;
**R⁸** H, OR^{8.1}, NR^{3.1}R^{8.2} oder C₁₋₆-Alkyl;
R^{8.1}, R^{8.2} gleich oder verschieden, Wasserstoff, COR^{8.1.1}, CONR^{8.1.1}R^{8.1.2}, SO₂NR^{8.1.1}R^{8.1.2} oder SO₂R^{8.1.1}
oder einen Rest ausgewählt aus der Gruppe bestehend aus C₁₋₆-Alkyl, C₃₋₆-Alkenyl. C₃₋₆-Alkinyl, C₃₋₈-Cycloalkyl und C₃₋₇-Cycloalkyl-C₁₋₄-alkyl-, oder
NR^{8.1}R^{8.2} bilden zusammen einen fünf-, sechs- oder siebengliedrigen Ring der gegebenenfalls ein weiteres Heteroatom enthalten kann;
R^{8.1.1}, R^{8.1.2} gleich oder verschieden, Wasserstoff oder einen Rest ausgewählt aus der Gruppe bestehend aus C₁₋₆-Alkyl, C₃₋₈-Cycloalkyl und C₃₋₇-Cycloalkyl-C₁₋₄-alkyl-,oder
NR^{8.1.1}R^{8.1.2} bilden zusammen einen fünf-, oder sechsgliedrigen Ring, der gegebenenfalls ein weiteres Heteroatom enthalten kann;
**R⁹, R¹⁰** gleich oder verschieden, einen Rest, gegebenenfalls substituiert mit OMe, CN, F, Cl oder Br, ausgewählt aus der Gruppe bestehend aus C₁₋₆-Alkyl, C₂₋₆-Alkenyl, C₂₋₆-Alkinyl, C₃₋₈-Cycloalkyl, C₃₋₈-Cycloalkenyl, C₃₋₇-Cycloalkyl-C₁₋₄-alkyl-, Aryl, Heteroaryl, Spiro, Heterocycloalkyl, Aryl-C₇₋₁₁-alkyl- (Aryl-C₁₋₆-alkyl-)und Heteroaryl-C₆₋₁₀-alkyl- (Heteroaryl-C₁₋₆-alkyl-); oder
R⁹, R¹⁰ gleich oder verschieden, Wasserstoff, COR^{9.1}, CONR^{9.1}R^{9.2}, SO₂R^{9.1} oder SO₂NR^{9.1}R^{9.2};
R^{9.1}, R^{9.2} gleich oder verschieden, Wasserstoff oder einen Rest ausgewählt aus der Gruppe bestehend aus C₁₋₆-Alkyl, C₂₋₆-Alkenyl, C₂₋₆-Alkinyl, C₁₋₆-Haloalkyl, C₃₋₈-Cycloalkyl, C₃₋₇-Cycloalkyl-C₁₋₄-alkyl-, Aryl, Heteroaryl, Spiro, Heterocycloalkyl, Aryl-C₁₋₆-alkyl- und Heteroaryl-C₁₋₆-alkyl-;
oder
NR^{9.1}R^{9.2} bilden zusammen einen fünf- oder sechsgliedrigen Ring, der gegebenenfalls ein weiteres Heteroatom enthalten kann,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, Solvate und Hydrate,
bedeuten.

2. Verbindungen der Formel (IA) nach Anspruch 1, 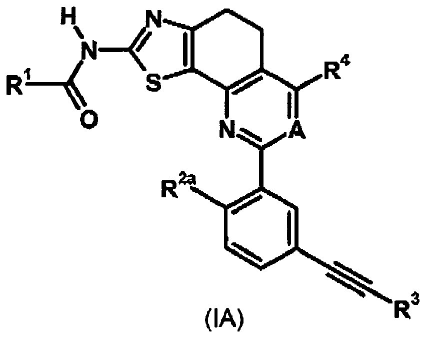 worin
A, R¹, R³, und R⁴ die angegebenen Bedeutungen haben können und
R^{2a} einen Rest ausgewählt aus der Gruppe bestehend aus F, Cl, Br, I, CN, CF₃, CF₂H, CFH₂ und NH₂;
oder
einen Rest ausgewählt aus der Gruppe bestehend aus - O-C₁₋₄-Alkyl, C₁₋₄-Alkyl und C₂₋₆-Alkenyl,
bedeutet.

3. Verbindungen nach Anspruch 1 oder 2, worin
R³ die angegebenen Bedeutungen haben kann und
n 1 oder 2,
R¹ C₁₋₄-Alkyl oder NR^{1.1}R^{1.2};
R^{1.1}, R^{1.2} gleich oder verschieden, H oder C₁₋₄-Alkyl;
R² bzw. R^{2a} gleich oder verschieden, Wasserstoff, F oder Cl;
und
R⁴ Wasserstoff;
bedeuten.

4. Verbindungen nach einem der Ansprüche 1 bis 3. worin
R¹. R², und R⁴ die angegebenen Bedeutungen haben können und
R³ einen Rest ausgewählt aus der Gruppe bestehend aus: 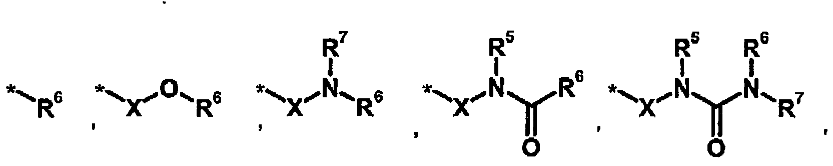 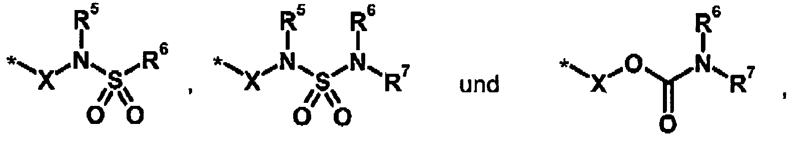 worin
X C₁₋₃-Alkylen.
R⁵, R⁶, R⁷ gleich oder verschieden, Wasserstoff oder einen Rest ausgewählt aus der Gruppe bestehend aus C₁₋₆-Alkyl, C₂₋₆-Alkenyl, C₁₋₆-Haloalkyl, C₃₋₈-Cycloalkyl, C₃₋₇-Cycloalkyl-C₁₋₄-alkyl-, Aryl, Heteroaryl, Heterocycloalkyl-, Aryl-C₁₋₅-alkyl-, Heteroaryl-C₁₋₅-alkyl-, Heterocycloalkyl-C₁₋₅-alkyl- und N(C₁₋₃-Alkyl)₂-C₁₋₄-alkyl-
oder
NR⁶R⁷ bilden einen fünf- oder sechsgliedrigen Ring bestehend aus Kohlenstoffatomen und gegebenenfalls einem Stickstoff oder Sauerstoffatom als weiteres Heteroatom,
oder
NR⁶R⁷ bilden einen Ring ausgewählt aus der Gruppe bestehend aus: 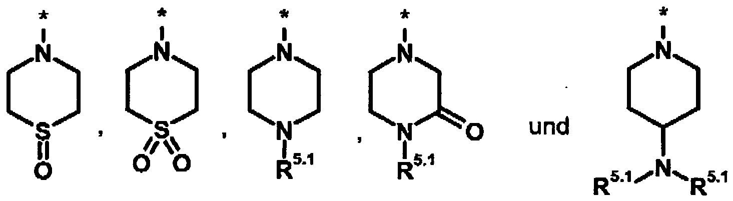
R^{5.1} gleich oder verschieden, Wasserstoff oder einen Rest ausgewählt aus der Gruppe bestehend aus C₁₋₆-Alkyl, C₃₋₈-Cycloalkyl, -CO-C₁₋₃-Alkyl und CONH₂;
bedeutet.

5. Verbindungen nach einem der Ansprüche 1 bis 3, worin
R¹, R², und R⁴ die angegebenen Bedeutungen haben können und
R³ einen Rest: 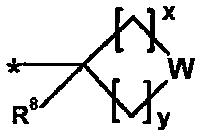
x, y gleich oder verschieden 0, 1, 2 oder 3,
W NR⁹ oder CR⁹R¹⁰.
R⁸ H, OR^{8.1} oder NR^{8.1}R^{8.2},
R^{8.1}, R^{8.2} gleich oder verschieden, Wasserstoff, COR^{8.1.1}, CONR^{8.1.1}R^{8.1.2}, oder C₁₋₆-Alkyl;
NR^{8.1}R^{8.2} bilden zusammen einen fünf- oder sechs- gliedrigen Ring der gegebenenfalls ein weiteres Heteroatom enthalten kann;
R^{8.1.1}, R^{8.1.2} gleich oder verschieden, Wasserstoff oder ein C₁₋₆-Alkyl,
R⁹, R¹⁰ gleich oder verschieden, gegebenenfalls substituiert mit OMe, CN, F, Cl oder Br, ausgewählt aus der Gruppe bestehend aus C₁₋₆-Alkyl, C₃₋₈-Cycloalkyl, C₃₋₇-Cycloalkyl-C₁₋₄-alkyl- oder
R⁹, R¹⁰ gleich oder verschieden, Wasserstoff, COR^{9.1}, CONR^{9.1}R^{9.2}, SO₂R^{9.1} oder SO₂NR^{9.1}R^{9.2};
R^{9.1}, R^{9.2} gleich oder verschieden, Wasserstoff oder einen Rest ausgewählt aus der Gruppe bestehend aus C₁₋₆-Alkyl und C₃₋₈-Cycloalkyl,
oder
NR^{9.1}R^{9.2} bilden zusammen einen fünf- oder sechsgliedrigen Ring, der gegebenenfalls Sauerstoff als ein weiteres Heteroatom enthalten kann,
bedeuten.

6. Verbindungen gemäß einem der Ansprüche 1 bis 5 zur Verwendung als Arzneimittel.

7. Verwendung der Verbindungen gemäß einem der Ansprüche 1 bis 5,zur Herstellung eines Arzneimittels zur Behandlung von entzündlichen und allergischen Erkrankungen der Atemwege..

8. Verwendung nach Anspruch 7, **dadurch gekennzeichnet, dass** es sich um eine Erkrankung handelt, die ausgewählt ist aus der Gruppe bestehend aus Chronischer Bronchitis, akuter Bronchitis, Bronchitis aufgrund bakterieller oder viraler Infektion oder Pilzen oder Helminthen, allergischer Bronchitis, toxischer Bronchitis, chronisch obstruktiver Bronchitis (COPD), Asthma (intrinsisch oder allergisch), pediatrischem Asthma, Bronchiektasien, allergischer Alveolitis, allergischer oder nicht-allergischer Rhinitis, chronischer Sinusitis, zystischer Fibrose oder Mukoviszidose, alpha-1-Antitrypsin-Mangel, Husten, Lungenemphysem, interstitieller Lungenerkrankungen, Alveolitis, hyperreaktiver Atemwege, Nasenpolypen, Lungenödemen, Pneumonitis aufgrund unterschiedlicher Genese wie Strahlen-induziert oder durch Aspiration oder infektiöse, Kollagenosen wie Lupus eryth, systemische Sklerodermie, Sarkoidose und M. Boeck.

9. Verwendung nach Anspruch 7, **dadurch gekennzeichnet, dass** es sich um eine Erkrankung handelt, die ausgewählt ist aus der Gruppe bestehend aus allergischer Rhinitis, allergischer Sinusitis und Nasenpolypen.

10. Pharmazeutische Formulierung enthaltend eine Verbindung der Formel (I)gemäß einem der Ansprüche 1 bis 5.

11. Arzneimittelkombinationen, die neben einer oder mehrerer Verbindungen der Formel (I)gemäß einem der Ansprüche 1 bis 5 als weiteren Wirkstoff einen oder mehrere Verbindungen enthalten, die ausgewählt sind aus den Klassen der Betamimetika, Anticholinergika, Corticosteroiden, weitere PDE4-Inhibitoren, LTD4-Antagonisten, EGFR-Hemmem, Dopamin-Agonisten, H1-Antihistaminika, PAF-Antagonisten und PI3-Kinase Inhibitoren oder zwei- oder dreifach Kombinationen davon.

12. Verfahren zur Herstellung von Verbindungen der allgemeinen Formel (I), 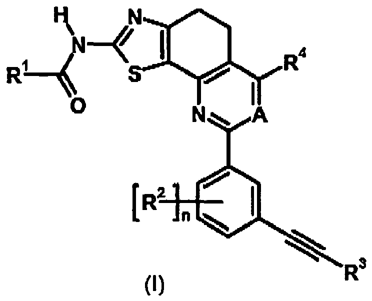 worin
A, R¹ bis R⁴ die IN ANSPRUCH 1 angegebenen Bedeutungen haben können,
**dadurch gekennzeichnet, dass**
(a) eine Verbindung der Formel (II) 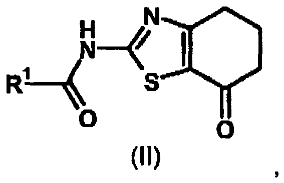 worin R¹ die angegebene Bedeutung hat,
mit einer Verbindung der Formel 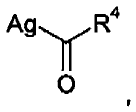 worin R⁴ die angegebenen Bedeutung hat und Ag eine Abgangsgruppe bedeutet, umgesetzt wird, und
(b) die aus Schritt (a) resultierende Verbindung der allgemeinen Formel (III) 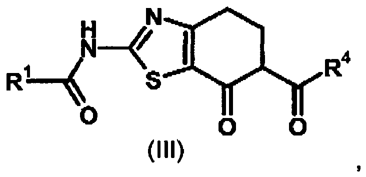 worin R¹ und R⁴ die angegebene Bedeutung haben,
mit einer Verbindung der allgemeinen Formel 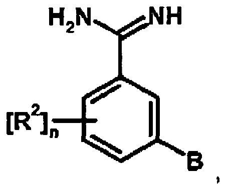 worin R² und n die angegebenen Bedeutungen haben und B eine Abgangsgruppe bedeutet, umgesetzt wird, und
(c) die aus Schritt (b) resultierende Verbindung der allgemeinen Formel (IV) 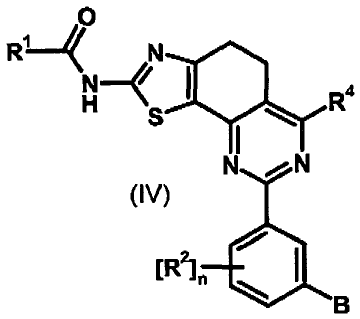 worin R¹, R², R⁴ und n die angegebenen Bedeutungen haben und B eine Abgangsgruppe bedeutet,
mit einer Verbindung der allgemeinen Formel 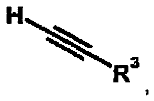 worin R³ die angegebenen Bedeutung hat,
umgesetzt wird.

13. Verbindungen gemäß der allgemeinen Formel (IV), 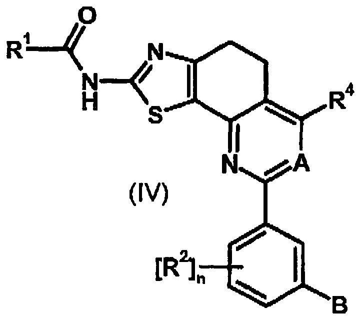 worin
worin A, R¹, R², R⁴ und n die in Anspruch 1 angegebenen Bedeutungen haben und B eine Abgangsgruppe bedeutef,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
mit der Maßgabe, dass R¹ nicht Methyl sein kann, wenn R2= H, B = Cl und R⁴=H bedeutet.

## Claims

**1.** Compounds of general formula (I) 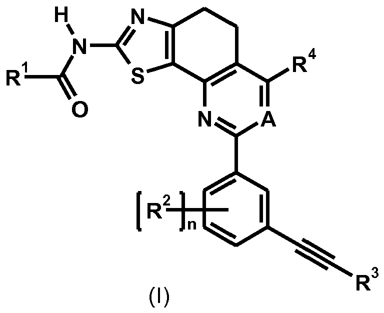 wherein
A denotes CH or N,
n denotes 1, 2, 3 or 4,
R¹ denotes hydrogen or a group consisting of C₁₋₄-alkyl, OR^{1.1} and NR^{1.1}R^{1.2};
R^{1.1}, R^{1.2} which may be identical or different, denote H or C₁₋₄-alkyl; or
NR^{1.1}R^{1.2} denotes a 5- to 6-membered heterocycle, optionally containing a further N-atom;
R² which may be identical or different, denote hydrogen or a group selected from among F, Cl, Br, I, CN, CF₃, CF₂H, CFH₂ and NH₂;
or
a group selected from among -O-C₁₋₄-alkyl, C₁₋₄-alkyl and C₂₋₆-alkenyl;
R⁴ denotes hydrogen, OH, NH₂, or
a group selected from among C₁₋₆-alkyl, C₂₋₆-alkenyl, C₃₋₆-cycloalkyl, -N(C₁-₄-alkyl)₂ and -NH(C₁₋₄-alkyl);
R³ denotes a group selected from among: 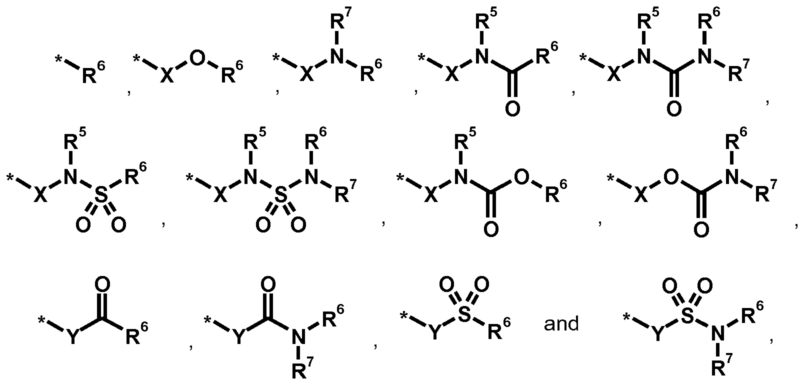 wherein
X denotes a group selected from among C₁₋₆-alkylene, C₂₋₅-alkenylene, C₁₋₅-alkynylene, C₃₋₇-cycloalkylene, C₅₋₇-cycloalkenylene and -C₁₋₄-alkylene-C₃₋₇-cycloalkylene;
Y denotes a bond or X;
R⁵, R⁶, R⁷ which may be identical or different, denote hydrogen or a group selected from among C₁₋₆-alkyl, C₂₋₆-alkenyl, C₂₋₆-alkynyl, C₁₋₆-haloalkyl, C₃₋₈-cycloalkyl, C₃₋₈-cycloalkenyl, C₃₋₇-cycloalkyl-C₁₋₄-alkyl, aryl, heteroaryl, spiro, heterocycloalkyl, aryl-C₁₋₆-alkyl, heteroaryl-C₁₋₆-alkyl and heterocycloalkyl-C₁₋₆-alkyl,
or
NR⁶R⁷ form a five-, six- or seven-membered ring consisting of carbon atoms and optionally a nitrogen, oxygen or sulphur atom as further heteroatoms or
a ring selected from among: 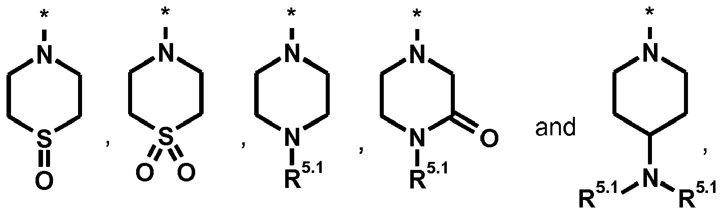
R^{5.1} which may be identical or different, denote hydrogen or a group selected from among C₁₋₆-alkyl, C₃₋₈-cycloalkyl, -CO-C₁₋₃-alkyl and CONH₂;
or
R⁵ and R⁶ together form a saturated or unsaturated alkylene bridge and may optionally contain a further nitrogen, oxygen or sulphur atom;
or **R**³ is 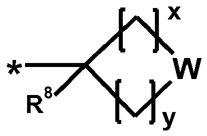
x, y which may be identical or different denote 0, 1, 2, 3, 4 or 5;
W denotes O, NR⁹ or CR⁹R¹⁰;
**R⁸** denotes H, OR^{8.1}, NR^{8.1}R^{8.2} or optionally substituted C₁₋₆-alkyl;
R^{8.1}, R^{8.2} which may be identical or different, denote hydrogen, COR^{8.1.1}, CONR^{8.1.1}R^{8.1.2}, SO₂NR^{8.1.1}R^{8.1.2} or SO₂R^{8.1.1} or a group selected from among C₁₋₆-alkyl, C₃₋₆-alkenyl, C₃₋₆-alkynyl, C₃₋₈-cycloalkyl and C₃₋₇-cycloalkyl-C₁₋₄-alkyl, or
NR^{8.1}R^{8.2} together form a five-, six- or seven-membered ring which may optionally contain a further heteroatom;
R^{8.1.1}, R^{8.1.2} which may be identical or different, denote hydrogen or a group selected from among C₁₋₆-alkyl, C₃₋₈-cycloalkyl and C₃₋₇-cycloalkyl-C₁₋₄-alkyl, or
NR^{8.1.1}R^{8.1.2} together form a five- or six-membered ring, which may optionally contain a further heteroatom;
**R⁹, R¹⁰** which may be identical or different, denote a group, optionally substituted by OMe, CN, F, Cl or Br, selected from among C₁₋₆-alkyl, C₂₋₆-alkenyl, C₂₋₆-alkynyl, C₃₋₈-cycloalkyl, C₃₋₈-cycloalkenyl, C₃₋₇-cycloalkyl-C₁₋₄-alkyl, aryl, heteroaryl, spiro, heterocycloalkyl, aryl-C₇₋₁₁-alkyl- (aryl-C₁₋₆-alkyl-)and heteroaryl-C₆₋₁₀-alkyl (heteroaryl-C₁₋₆-alkyl); or
R⁹, R¹⁰, which may be identical or different, denote hydrogen, COR^{9.1}, CONR^{9.1}R^{9.2}, SO₂R^{9.1} or SO₂NR^{9.1}R⁹.²;
R^{9.1}, R^{9.2} which may be identical or different, denote hydrogen or a group selected from among C₁₋₆-alkyl, C₂₋₆-alkenyl, C₂₋₆-alkynyl, C₁₋₆-haloalkyl, C₃₋₈-cycloalkyl, C₃₋₇-cycloalkyl-C₁₋₄-alkyl, aryl, heteroaryl, spiro, heterocycloalkyl, aryl-C₁₋₆-alkyl- and heteroaryl-C₁₋₆-alkyl-;
or
NR^{9.1}R^{9.2} together form a five- or six-membered ring, which may optionally contain a further heteroatom,
optionally in the form of the tautomers, the racemates, the enantiomers, the diastereomers and the mixtures thereof, and optionally the pharmacologically acceptable acid addition salts, solvates and hydrates thereof.

**2.** Compounds of formula (IA) according to claim 1, 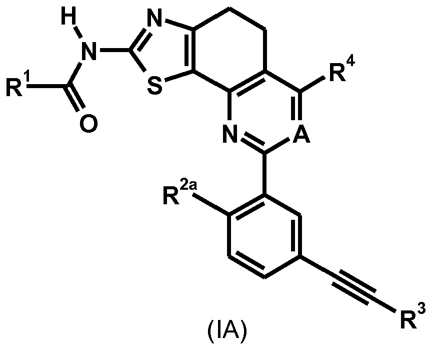 wherein
A, R¹, R³, and R⁴ may have the meanings stated and
R^{2a} denotes a group selected from among F, Cl, Br, I, CN, CF₃, CF₂H, CFH₂ and NH₂;
or
a group selected from among -O-C₁₋₄-alkyl, C₁₋₄-alkyl and C₂₋₆-alkenyl.

**3.** Compounds according to claim 1 or 2, wherein
R³ may have the meanings stated and
n denotes 1 or 2,
R¹ denotes C₁₋₄-alkyl or NR^{1.1}R^{1.2};
R^{1.1}, R^{1.2} which may be identical or different, denote H or C₁₋₄-alkyl;
R² and/or R^{2a} which may be identical or different, denote hydrogen, F or Cl;
and
R⁴ denotes hydrogen.

**4.** Compounds according to one of claims 1 to 3, wherein
R¹, R², and R⁴ may have the meanings stated and
R³ denotes a group selected from among: 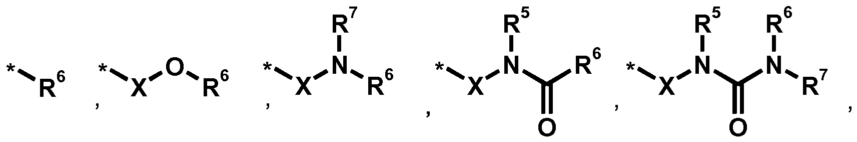 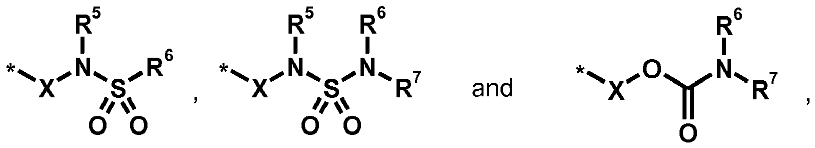 wherein
X denotes C₁₋₃-alkylene,
R⁵, R⁶, R⁷ which may be identical or different, denote hydrogen or a group selected from among C₁₋₆-alkyl, C₂₋₆-alkenyl, C₁₋₆-haloalkyl, C₃₋₈-cycloalkyl, C₃₋₇-cycloalkyl-C₁₋₄-alkyl, aryl, heteroaryl, heterocycloalkyl, aryl-C₁₋₅-alkyl, heteroaryl-C₁₋₅-alkyl, heterocycloalkyl-C₁₋₅-alkyl- and N(C₁₋₃-alkyl)₂C₁₋₄-alkyl-
or
NR⁶R⁷ form a five- or six-membered ring consisting of carbon atoms and optionally a nitrogen or oxygen atom as a further heteroatom,
or
NR⁶R⁷ form a ring selected from among: 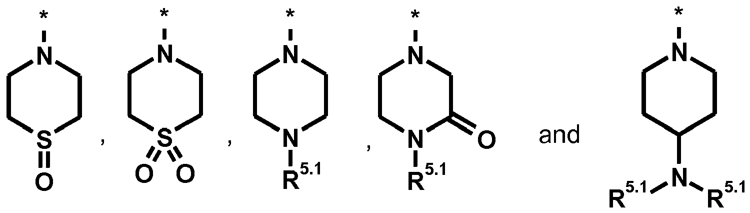
R^{5.1} which may be identical or different, denotes hydrogen or a group selected from among C₁₋₆-alkyl, C₃₋₈-cycloalkyl, -CO-C₁₋₃-alkyl and CONH₂.

**5.** Compounds according to one of claims 1 to 3, wherein R¹, R², and R⁴ may have the meanings stated and
R³ denotes a group: 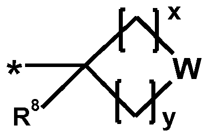
x, y which may be identical or different denote 0, 1, 2 or 3,
W denotes NR⁹ or CR⁹R¹⁰;
R⁸ denotes H, OR^{8.1} or NR^{8.1}R^{8.2},
R^{8.1}, R^{8.2} which may be identical or different, denote hydrogen, COR^{8.1.1}, CONR^{8.1.1}R^{8.1.2}, or C₁₋₆-alkyl;
NR^{8.1}R^{8.2} together form a five- or six-membered ring which may optionally contain a further heteroatom;
R^{8.1.1}, R^{8.1.2} which may be identical or different, denote hydrogen or a C₁₋₆-alkyl,
R⁹, R¹⁰ which may be identical or different, denote a group, optionally substituted by OMe, CN, F, Cl or Br, selected from among C₁₋₆-alkyl, C₃₋₈-cycloalkyl, C₃₋₇-cycloalkyl-C₁₋₄-alkyl or
R⁹, R¹⁰ which may be identical or different, denote hydrogen, COR^{9.1}, CONR^{9.1}R^{9.2}, SO₂R^{9.1} or SO₂NR^{9.1}R^{9.2};
R^{9.1}, R^{9.2} which may be identical or different, denote hydrogen or a group selected from among C₁₋₆-alkyl and C₃₋₈-cycloalkyl,
or
NR^{9.1}R^{9.2} together form a five- or six-membered ring, which may optionally contain oxygen as a further heteroatom.

**6.** Compounds according to one of claims 1 to 5 for use as medicaments.

**7.** Use of the compounds according to one of claims 1 to 5 for preparing a medicament for the treatment of inflammatory and allergic diseases of the airways.

**8.** Use according to claim 7, **characterised in that** it relates to a disease selected from among chronic bronchitis, acute bronchitis, bronchitis caused by bacterial or viral infection or fungi or helminths, allergic bronchitis, toxic bronchitis, chronic obstructive bronchitis (COPD), asthma (intrinsic or allergic), paediatric asthma, bronchiectasis, allergic alveolitis, allergic or non-allergic rhinitis, chronic sinusitis, cystic fibrosis or mucoviscidosis, alpha-1-antitrypsin deficiency, cough, pulmonary emphysema, interstitial lung diseases, alveolitis, hyperreactive airways, nasal polyps, pulmonary oedema, pneumonitis of different origins, e.g. radiation-induced or caused by aspiration, or infectious pneumonitis, collagenoses such as lupus eryth, systemic scleroderma, sarcoidosis and Boeck's disease.

**9.** Use according to claim 7, **characterised in that** it relates to a disease selected from among allergic rhinitis, allergic sinusitis and nasal polyps.

**10.** Pharmaceutical formulation containing a compound of formula (I) according to one of claims 1 to 5.

**11.** Medicament combinations which contain, in addition to one or more compounds of formula (I) according to one of claims 1 to 5 as a further active substance, one or more compounds which are selected from the categories of the betamimetics, anticholinergics, corticosteroids, other PDE4-inhibitors, LTD4-antagonists, EGFR-inhibitors, dopamine agonists, H1-antihistamines, PAF-antagonists and PI3-kinase inhibitors or double or triple combinations thereof.

**12.** Process for preparing compounds of general formula (I), 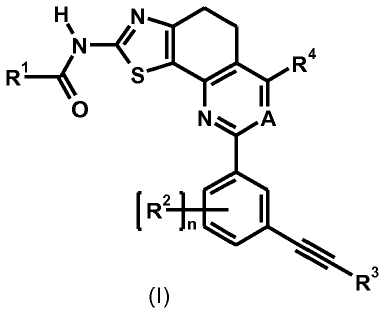 wherein
A, R¹ to R⁴ may have the meanings specified in claim 1, **characterised in that**
(a) a compound of formula (II) 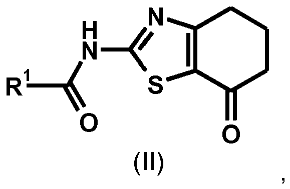 wherein R¹ has the meaning specified,
is reacted with a compound of formula 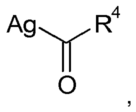 wherein R⁴ has the specified meaning and Ag denotes a leaving group, and
(b) the compound of general formula (III) 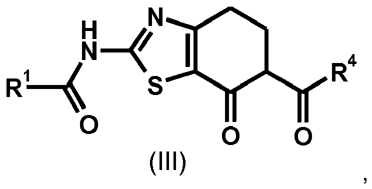 resulting from step (a),
wherein R¹ and R⁴ have the meanings specified,
is reacted with a compound of general formula 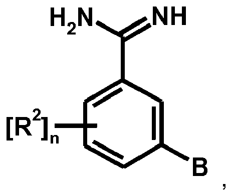 wherein R² and n have the meanings specified and B denotes a leaving group, and
(c) the compound of general formula (IV) 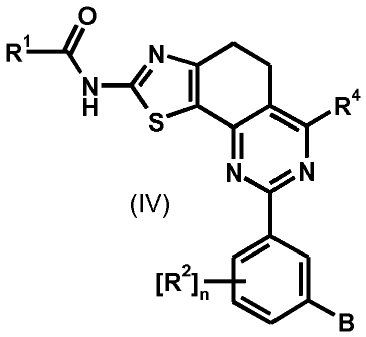 resulting from step (b),
wherein R¹, R², R⁴ and n have the meanings specified and B denotes a leaving group, is reacted with a compound of general formula 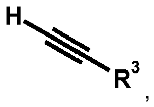 wherein R³ has the specified meaning.

**13.** Compounds according to general formula (IV), 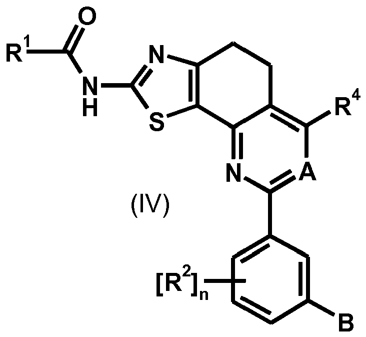 wherein A, R¹, R², R⁴ and n have the meanings specified in claim 1 and B denotes a leaving group,
optionally in the form of the tautomers, the racemates, the enantiomers, the diastereomers and the mixtures thereof, and optionally the pharmacologically acceptable acid addition salts thereof,
with the proviso that R¹ cannot be methyl if R²= H, B = Cl and R⁴=H.

**1.** Compounds of general formula (I) 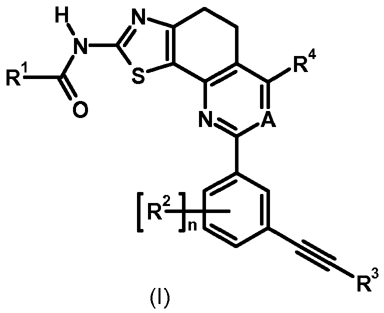 wherein
A denotes CH or N,
n denotes 1, 2, 3 or 4,
R¹ denotes hydrogen or a group consisting of C₁₋₄-alkyl, OR^{1.1} and NR^{1.1}R^{1.2};
R^{1.1}, R^{1.2} which may be identical or different, denote H or C₁₋₄-alkyl; or
NR^{1.1}R^{1.2} denotes a 5- to 6-membered heterocycle, optionally containing a further N-atom;
R² which may be identical or different, denote hydrogen or
a group selected from among F, Cl, Br, I, CN, CF₃, CF₂H, CFH₂ and NH₂; or
a group selected from among -O-C₁₋₄-alkyl, C₁₋₄-alkyl and C₂₋₆-alkenyl;
R⁴ denotes hydrogen, OH, NH₂, or
a group selected from among C₁₋₆-alkyl, C₂₋₆-alkenyl, C₃₋₆-cycloalkyl, -N(C₁₋₄-alkyl)₂ and -NH(C₁₋₄-alkyl);
R³ denotes a group selected from among: 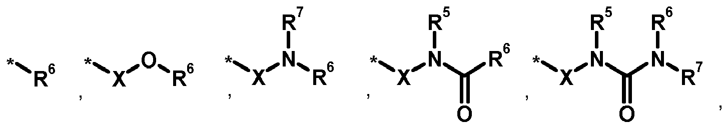 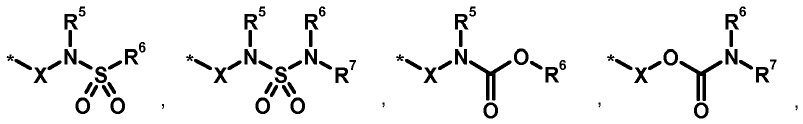 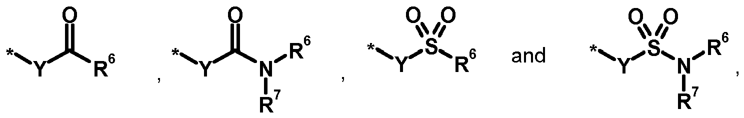 wherein
X denotes a group selected from among C₁₋₆-alkylene, C₂₋₅-alkenylene, C₁₋₅-alkynylene, C₃₋₇-cycloalkylene, C₅₋₇-cycloalkenylene and -C₁₋₄-alkylene-C₃₋₇-cycloalkylene;
Y denotes a bond or X;
R⁵, R⁶, R⁷ which may be identical or different, denote hydrogen or a group selected from among C₁₋₆-alkyl, C₂₋₆-alkenyl, C₂₋₆-alkynyl, C₁₋₆-haloalkyl, C₃₋₈-cycloalkyl, C₃₋₈-cycloalkenyl, C₃₋₇-cycloalkyl-C₁₋₄-alkyl, aryl, heteroaryl, spiro, heterocycloalkyl, aryl-C₁₋₆-alkyl, heteroaryl-C₁₋₆-alkyl and heterocycloalkyl-C₁₋₆-alkyl,
or
NR⁶R⁷ form a five-, six- or seven-membered ring consisting of carbon atoms and optionally a nitrogen, oxygen or sulphur atom as further heteroatoms or
a ring selected from among: 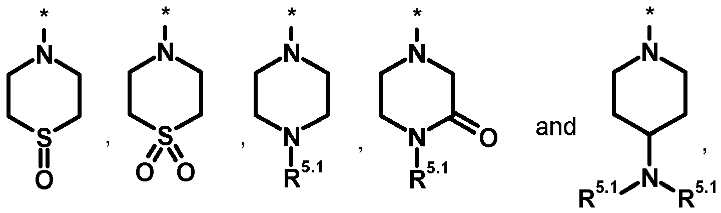
R^{5.1} which may be identical or different, denote hydrogen or a group selected from among C₁₋₆-alkyl, C₃₋₈-cycloalkyl, -CO-C₁₋₃-alkyl and CONH₂;
or
R⁵ and R⁶ together form a saturated or unsaturated alkylene bridge and may optionally contain a further nitrogen, oxygen or sulphur atom;
or **R³** is 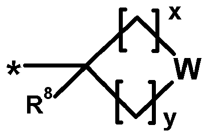
x, y which may be identical or different denote 0, 1, 2, 3, 4 or 5;
W denotes O, NR⁹ or CR⁹R¹⁰;
**R⁸** denotes H, OR^{8.1}, NR^{8.1}R^{8.2} or optionally substituted C₁₋₆-alkyl;
R^{8.1}, R^{8.2} which may be identical or different, denote hydrogen, COR^{8.1.1}, CONR^{8.1.1}R^{8.1.2}, SO₂NR^{8.1.1}R^{8.1.2} or SO₂R^{8.1.1}
or a group selected from among C₁₋₆-alkyl, C₃₋₆-alkenyl, C₃₋₆-alkynyl, C₃₋₈-cycloalkyl and C₃₋₇-cycloalkyl-C₁₋₄-alkyl, or
NR^{8.1}R^{8.2} together form a five-, six- or seven-membered ring which may optionally contain a further heteroatom;
R^{8.1.1}, R^{8.1.2} which may be identical or different, denote hydrogen or a group selected from among C₁₋₆-alkyl, C₃₋₈-cycloalkyl and C₃₋₇-cycloalkyl-C₁₋₄-alkyl, or
NR^{8.1.1}R^{8.1.2} together form a five- or six-membered ring, which may optionally contain a further heteroatom;
**R⁹, R¹⁰** which may be identical or different, denote a group, optionally substituted by OMe, CN, F, Cl or Br, selected from among C₁₋₆-alkyl, C₂₋₆-alkenyl, C₂₋₆-alkynyl, C₃₋₈-cycloalkyl, C₃₋₈-cycloalkenyl, C₃₋₇-cycloalkyl-C₁₋₄-alkyl, aryl, heteroaryl, spiro, heterocycloalkyl, aryl-C₇₋₁₁-alkyl- (aryl-C₁₋₆-alkyl-)and heteroaryl-C₆₋₁₀-alkyl (heteroaryl-C₁₋₆-alkyl); or
R⁹, R¹⁰, which may be identical or different, denote hydrogen, COR^{9.1}, CONR^{9.1}R^{9.2}, SO₂R^{9.1} or SO₂NR^{9.1}R^{9.2};
R^{9.1}, R^{9.2} which may be identical or different, denote hydrogen or a group selected from among C₁₋₆-alkyl, C₂₋₆-alkenyl, C₂₋₆-alkynyl, C₁₋₆-haloalkyl, C₃₋₈-cycloalkyl, C₃₋₇-cycloalkyl-C₁₋₄-alkyl, aryl, heteroaryl, spiro, heterocycloalkyl, aryl-C₁₋₆-alkyl- and heteroaryl-C₁₋₆-alkyl-;
or
NR^{9.1}R^{9.2} together form a five- or six-membered ring, which may optionally contain a further heteroatom,
optionally in the form of the tautomers, the racemates, the enantiomers, the diastereomers and the mixtures thereof, and optionally the pharmacologically acceptable acid addition salts, solvates and hydrates thereof.

**2.** Compounds of formula (IA) according to claim 1, 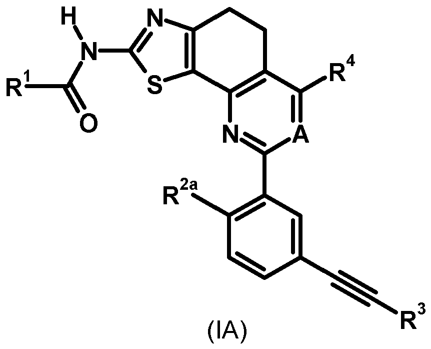 wherein
A, R¹, R³, and R⁴ may have the meanings stated and
R^{2a} denotes a group selected from among F, Cl, Br, I, CN, CF₃, CF₂H, CFH₂ and NH₂;
or
a group selected from among -O-C₁₋₄-alkyl, C₁₋₄-alkyl and C₂₋₆-alkenyl.

**3.** Compounds according to claim 1 or 2, wherein
R³ may have the meanings stated and
n denotes 1 or 2,
R¹ denotes C₁₋₄-alkyl or NR^{1.1}R^{1.2};
R^{1.1}, R^{1.2} which may be identical or different, denote H or C₁₋₄-alkyl;
R² and/or R^{2a} which may be identical or different, denote hydrogen, F or Cl;
and
R⁴ denotes hydrogen.

**4.** Compounds according to one of claims 1 to 3, wherein
R¹, R², and R⁴ may have the meanings stated and
R³ denotes a group selected from among: 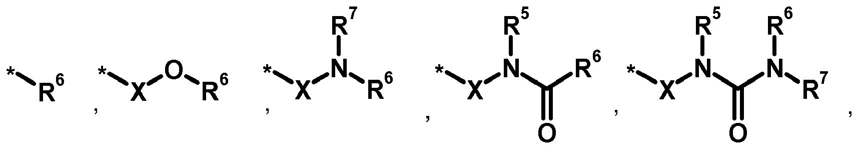 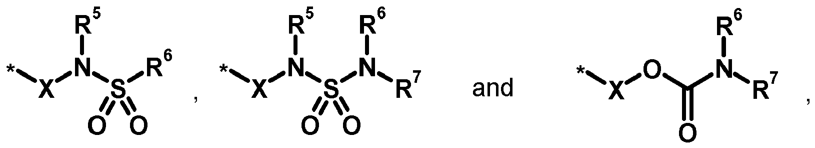 wherein
X denotes C₁₋₃-alkylene,
R⁵, R⁶, R⁷ which may be identical or different, denote hydrogen or a group selected from among C₁₋₆-alkyl, C₂₋₆-alkenyl, C₁₋₆-haloalkyl, C₃₋₈-cycloalkyl, C₃₋₇-cycloalkyl-C₁₋₄-alkyl, aryl, heteroaryl, heterocycloalkyl, aryl-C₁₋₅-alkyl, heteroaryl-C₁₋₅-alkyl, heterocycloalkyl-C₁₋₅-alkyl- and N(C₁₋₃-alkyl)₂-C₁₋₄-alkyl-
or
NR⁶R⁷ form a five- or six-membered ring consisting of carbon atoms and optionally a nitrogen or oxygen atom as a further heteroatom,
or
NR⁶R⁷ form a ring selected from among: 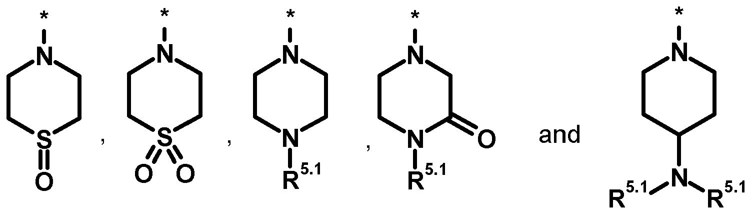
R^{5.1} which may be identical or different, denotes hydrogen or a group selected from among C₁₋₆-alkyl, C₃₋₈-cycloalkyl, -CO-C₁₋₃-alkyl and CONH₂.

**5.** Compounds according to one of claims 1 to 3, wherein
R¹, R², and R⁴ may have the meanings stated and
R³ denotes a group: 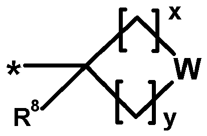
x, y which may be identical or different denote 0, 1, 2 or 3,
W denotes NR⁹ or CR⁹R¹⁰;
R⁸ denotes H, OR^{8.1} or NR^{8.1}R^{8.2},
R^{8.1}, R^{8.2} which may be identical or different, denote hydrogen, COR^{8.1.1}, CONR^{8.1.1}R^{8.1.2}, or C₁₋₆-alkyl;
NR^{8.1}R^{8.2} together form a five- or six-membered ring which may optionally contain a further heteroatom;
R^{8.1.1}, R^{8.1.2} which may be identical or different, denote hydrogen or a C₁₋₆-alkyl,
R⁹, R¹⁰ which may be identical or different, denote a group, optionally substituted by OMe, CN, F, Cl or Br, selected from among C₁₋₆-alkyl, C₃₋₈-cycloalkyl, C₃₋₇-cycloalkyl-C₁₋₄-alkyl or
R⁹, R¹⁰ which may be identical or different, denote hydrogen, COR^{9.1}, CONR^{9.1}R^{9.2}, SO₂R^{9.1} or SO₂NR^{9.1}R^{9.2};
R^{9.1}, R^{9.2} which may be identical or different, denote hydrogen or a group selected from among C₁₋₆-alkyl and C₃₋₈-cycloalkyl,
or
NR^{9.1}R^{9.2} together form a five- or six-membered ring, which may optionally contain oxygen as a further heteroatom.

**6.** Compounds according to one of claims 1 to 5 for use as medicaments.

**7.** Use of the compounds according to one of claims 1 to 5 for preparing a medicament for the treatment of inflammatory and allergic diseases of the airways.

**8.** Use according to claim 7, **characterised in that** it relates to a disease selected from among chronic bronchitis, acute bronchitis, bronchitis caused by bacterial or viral infection or fungi or helminths, allergic bronchitis, toxic bronchitis, chronic obstructive bronchitis (COPD), asthma (intrinsic or allergic), paediatric asthma, bronchiectasis, allergic alveolitis, allergic or non-allergic rhinitis, chronic sinusitis, cystic fibrosis or mucoviscidosis, alpha-1-antitrypsin deficiency, cough, pulmonary emphysema, interstitial lung diseases, alveolitis, hyperreactive airways, nasal polyps, pulmonary oedema, pneumonitis of different origins, e.g. radiation-induced or caused by aspiration, or infectious pneumonitis, collagenoses such as lupus eryth, systemic scleroderma, sarcoidosis and Boeck's disease.

**9.** Use according to claim 7, **characterised in that** it relates to a disease selected from among allergic rhinitis, allergic sinusitis and nasal polyps.

**10.** Pharmaceutical formulation containing a compound of formula (I) according to one of claims 1 to 5.

**11.** Medicament combinations which contain, in addition to one or more compounds of formula (I) according to one of claims 1 to 5 as a further active substance, one or more compounds which are selected from the categories of the betamimetics, anticholinergics, corticosteroids, other PDE4-inhibitors, LTD4-antagonists, EGFR-inhibitors, dopamine agonists, H1-antihistamines, PAF-antagonists and PI3-kinase inhibitors or double or triple combinations thereof.

**12.** Process for preparing compounds of general formula (I), 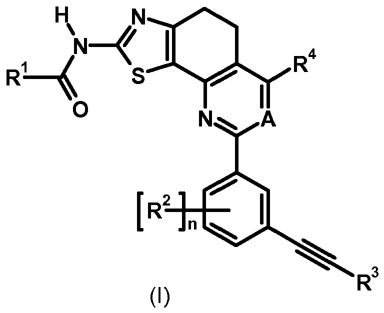 wherein
A, R¹ to R⁴ may have the meanings specified in claim 1, **characterised in that**
(a) a compound of formula (II) 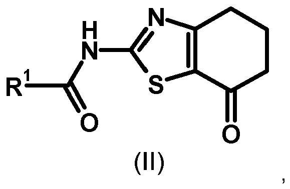 wherein R¹ has the meaning specified,
is reacted with a compound of formula 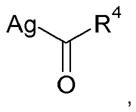 wherein R⁴ has the specified meaning and Ag denotes a leaving group, and
(b) the compound of general formula (III) 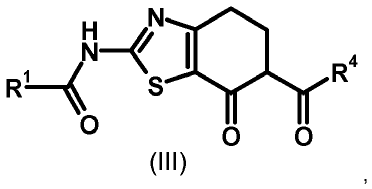 resulting from step (a),
wherein R¹ and R⁴ have the meanings specified,
is reacted with a compound of general formula 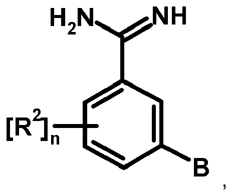 wherein R² and n have the meanings specified and B denotes a leaving group, and
(c) the compound of general formula (IV) 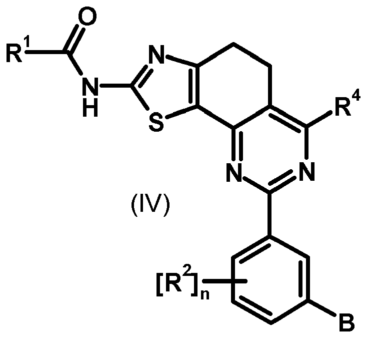
resulting from step (b),
wherein R¹, R², R⁴ and n have the meanings specified and B denotes a leaving group, is reacted with a compound of general formula 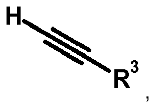 wherein R³ has the specified meaning.

**13.** Compounds according to general formula (IV), 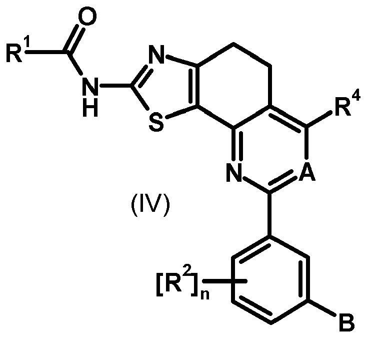 wherein A, R¹, R², R⁴ and n have the meanings specified in claim 1 and B denotes a leaving group,
optionally in the form of the tautomers, the racemates, the enantiomers, the diastereomers and the mixtures thereof, and optionally the pharmacologically acceptable acid addition salts thereof,
with the proviso that R¹ cannot be methyl if R²= H, B = Cl and R⁴=H.

## Revendications

**1.** Composés de formule générale (I) 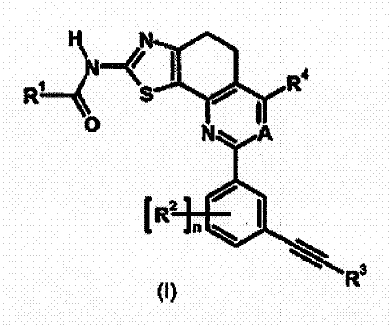 dans laquelle
A représente CH ou N,
n est 1, 2, 3 ou 4,
R¹ représente un atome d'hydrogène ou un radical consistant en un groupe alkyle en C₁ à C₄, OR^{1.1} et NR^{1.1}R^{1.2} ;
R^{1.1}, R^{1.2} identiques ou différents, représentent H ou un groupe alkyle en C₁ à C₄ ; ou
NR^{1.1}R^{1.2} représente un hétérocycle de 5 à 6 chaînons, contenant éventuellement un autre atome de N ;
R² identiques ou différents représentent un atome d'hydrogène ou un radical choisi dans le groupe consistant en F, Cl, Br, I, CN, CF₃, CF₂H, CFH₂ et NH₂ ;
ou
un radical choisi dans le groupe consistant en groupes O-alkyle en C₁ à C₄, alkyle en C₁ à C₄ et alcényle en C₂ à C₆ ;
R⁴ représente un atome d'hydrogène, OH, NH₂ ou un radical choisi dans le groupe consistant en groupes alkyle en C₁ à C₆, alcényle en C₂ à C₆, cycloalkyle en C₃ à C₆, -N (alkyle en C₁ à C₄)₂ et - NH(alkyle en C₁ à ₄) ;
R³ représente un radical choisi dans le groupe consistant en : 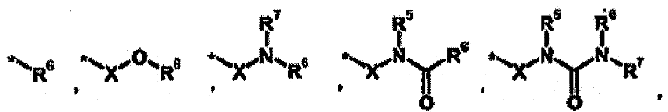 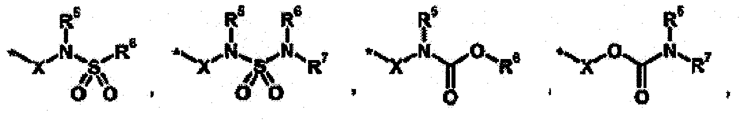 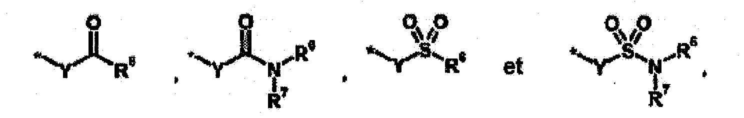 où
X représente un radical choisi dans le groupe consistant en groupes alkylène en C₁ à C₆, alcénylène en C₂ à C₅, alcinylène en C₁ à C₅, cycloalkylène en C₃ à C₇, cycloalcénylène en C₅ à C₇ et alkylène en C₁ à C₄-cycloalkylène en C₃ à C₇ ;
Y représente une liaison ou X ;
R⁵,R⁶,R⁷ identiques ou différents, représentent un atome d'hydrogène ou un radical choisi dans le groupe consistant en groupes alkyle en C₁ à C₆, alcényle en C₂ à C₆, alcinyle en C₂ à C₆, halogénoalkyle en C₁ à C₆, cycloalkyle en C₃ à C₈, cycloalcényle en C₃ à C₈, cycloalkyle en C₃ à C₇-alkyle en C₁ à C₄, aryle, hétéroaryle, spiro, hétérocycloalkyle, aryl-alkyle en C₁ à C₆, hétéroaryl-alkyle en C₁ à C₆ et hétérocycloalkyle-alkyle en C₁ à C₆,
ou
NR⁶R⁷ forment un cycle à cinq, six ou sept chaînons consistant en atomes de carbone et éventuellement un atome d'azote, d'oxygène ou de soufre comme autres hétéroatomes ou
un cycle choisi dans le groupe consistant en : 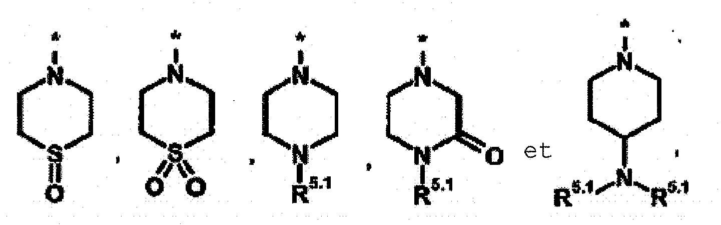
R^{5.1} identiques ou différents, représentent un atome d'hydrogène ou un radical choisi dans le groupe consistant en groupe alkyle en C₁ à C6, cycloalkyle en C₃ à C₈, -CO-alkyle en C₁ à C₃ et CONH₂ ;
ou
R⁵ et R⁶ forment conjointement un pont alkylène saturé ou insaturé et peuvent contenir éventuellement un autre atome d'azote, d'oxygène ou de soufre ;
ou R³ est 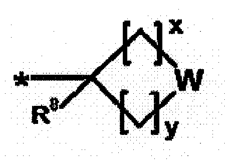
x,y identiques ou différents sont 0, 1, 2, 3, 4 ou 5 ;
W représente O, NR⁹ ou CR⁹R¹⁰ ;
R⁸ représente H, OR^{8.1}, NR^{8.1}R^{8.2} ou un groupe alkyle en C₁ à C₆ ;
R^{8.1},R^{8.2} identiques ou différents représentent un atome d'hydrogène, COR^{8.1.1}, CONR^{8.1.1}R^{8.1.2}, SO₂NR^{8.1.1}R^{8.1.2} ou SO₂R^{8.1.1}
ou un radical choisi dans le groupe consistant en groupe alkyle en C₁ à C₆, alcényle en C₃ à C₆, alcinyle en C₃ à C₆, cycloalkyle en C₃ à C₈ et cycloalkyle en C₃ à C₇-alkyle en C₁ à C₄, ou
NR^{8.1}R^{8.2} forment conjointement un cycle à cinq, six ou sept chaînons qui peut contenir éventuellement un autre hétéroatome ;
R^{8.1.1}R^{8.1.2} identiques ou différents, représentent un atome d'hydrogène ou un radical choisi dans le groupe consistant en groupe alkyle en C₁ à C₆, cycloalkyle en C₃ à C₈ et cycloalkyle en C₃ à C₇-alkyle en C₁ à C₄, ou
NR^{8.1.1}R^{8.1.2} forment conjointement un cycle à cinq ou six chaînons qui peut contenir éventuellement un autre hétéroatome ;
R⁹, R¹⁰ identiques ou différents, représentent un radical éventuellement substitué par OMe, CN, F, Cl ou Br, choisi dans le groupe consistant en groupes alkyle en C₁ à C₆, alcényle en C₂ à C₆, alcinyle en C₂ à C₆, cycloalkyle en C₃ à C₈, cycloalcényle en C₃ à C₈, cycloalkyle en C₃ à C₇-alkyle en C₁ à C₄, aryle, hétéroaryle, spiro, hétérocycloalkyle, aryle-alkyle en C₇ à C₁₁-(aryl-alkyle en C₁ à C₆) et hétéroaryle-alkyle en C₆ à C₁₀-(hétéroaryl-alkyle en C₁ à C₆) ; ou
R⁹, R¹⁰ identiques ou différents, représentent un atome d'hydrogène, COR^{9.1}, CONR^{9.1}R^{9.2}, SO₂R^{9.1} ou SO₂NR^{9.1}R^{9.2} ;
R^{9.1}, R^{9.2} identiques ou différents, représentant un atome d'hydrogène, ou un radical choisi dans le groupe consistant en groupes alkyle en C₁ à C6, alcényle en C₂ à C₆, alcinyle en C₂ à C₆, halogénoalkyle en C₁ à C₆, cycloalkyle en C₃ à C₈, cycloalkyle en C₃ à C₇-alkyle en C₁ à C₄, aryle, hétéroaryle, spiro, hétérocycloalkyle, aryl-alkyle en C₁ à C₆ et hétéroaryl-alkyle en C₁ à C₆ ;
ou
NR^{9.1}R^{9.2} forment conjointement un cycle à cinq ou six chaînons qui peut contenir éventuellement un autre hétéroatome,
éventuellement sous la forme de leurs tautomères, leurs racémates, leurs énantiomères, leurs diastéréomères et leurs mélanges, et éventuellement leurs sels, solvates et hydrates pharmacologiquement inoffensifs.

**2.** Composés de formule (IA) selon la revendication 1, 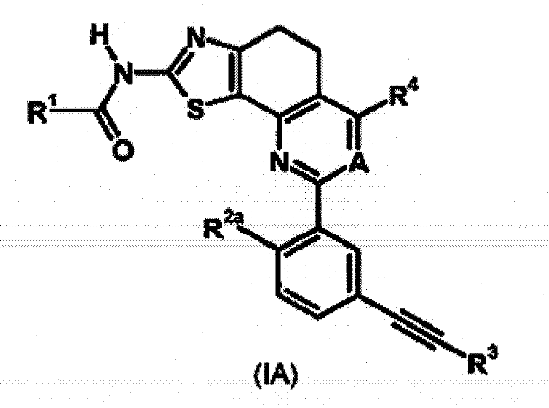 dans laquelle
A, R¹, R³ et R⁴ ont les significations indiquées et
R^{2a} représente un radical choisi dans le groupe consistant en F, Cl, Br, I, CN, CF₃, CF₂H, CFH₂ et NH₂ ;
ou
un radical choisi dans le groupe consistant en groupes O-alkyle en C₁ à C₄, alkyle en C₁ à C₄ et alcényle en C₂ à C₈.

**3.** Composés selon la revendication 1 ou 2, dans lesquels
R³ peut avoir les significations indiquées et
n est 1 ou 2,
R¹ représente un groupe alkyle en C₁ à C₄ ou NR^{1.1}R^{1.2},
R^{1.1}, R^{1.2} identiques ou différents, représentent H ou un groupe alkyle en C₁ à C₄ ;
R² ou R^{2a} identiques ou différents, représentent un atome d'hydrogène, F ou Cl ;
et
R⁴ représente un atome d'hydrogène.

**4.** Composés selon l'une des revendications 1 à 3, dans lesquels
R¹, R² et R⁴ peuvent avoir les significations indiquées et
R³ représente un radical choisi dans le groupe consistant en : 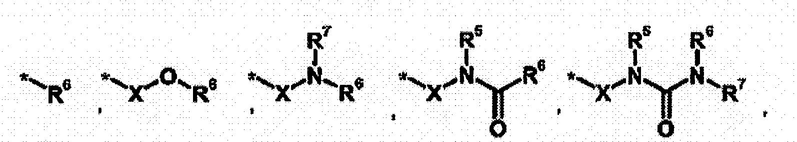 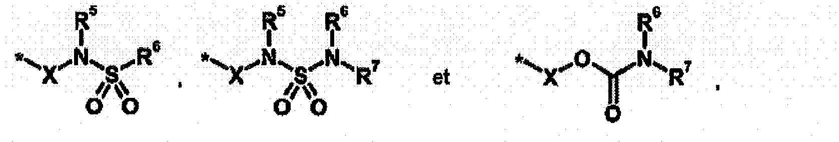 dans lequel
X représente un groupe alkylène en C₁ à C₃,
R⁵,R⁶,R⁷ identiques ou différentes, représentent un atome d'hydrogène ou un radical choisi dans le groupe consistant en groupes alkyle en C₁ à C₆, alcényle en C₂ à C₆, halogénoalkyle en C₁ à C₆, cycloalkyle en C₃ à C₈, cycloalkyle en C₃ à C₇-alkyle en C₁ à C₄, aryle, hétéroaryle, hétérocycloalkyle, aryl-alkyle en C₁ à C₅, hétéroaryl-alkyle en C₁ à C₅ et hétérocycloalkyle-alkyle en C₁ à C₅, et N (alkyle en C₁ à C₃)₂-alkyle en C₁ à C₄
ou
NR⁶R⁷ forment un cycle à cinq ou six chaînons consistant en atomes de carbone et éventuellement, en un atome d'azote ou un atome d'oxygène comme autre hétéroatome,
ou
NR⁶R⁷ forment un cycle choisi dans le groupe consistant en : 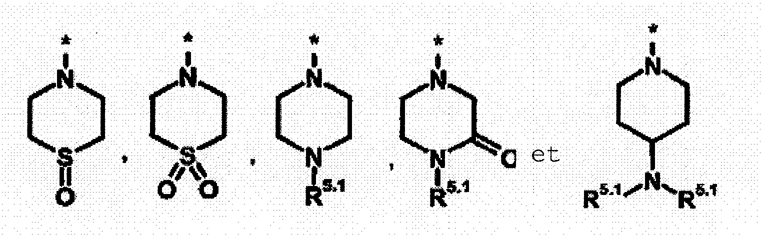
R^{5.1} identiques ou différents, représentent un atome d'hydrogène ou un radical choisi dans le groupe consistant en groupe alkyle en C₁ à C₆, cycloalkyle en C₃ à C₈, -CO-alkyle en C₁ à C₃ ou CONH₂ ;

**5.** Composés selon l'une des revendications 1 à 3, dans lesquels
R¹, R² et R⁴ peuvent avoir les significations indiquées et
R³ représente un radical : 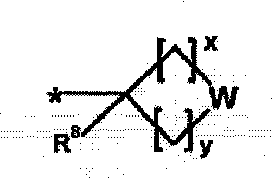
x,y identiques ou différents sont 0, 1, 2 ou 3,
W représente NR⁹ ou CR⁹R¹⁰ ;
R⁸ représente H_{,} OR^{8.1} ou NR^{8.1}R^{8.2},
R^{8.1}, R^{8.2} identiques ou différents représentent un atome d'hydrogène, COR^{8.1}, CONR^{8.1.1}R^{8.1.2}, ou un groupe alkyle en C₁ à C₆ ;
NR^{8.1}R^{8.2} forment conjointement un cycle à cinq ou six chaînons qui peut contenir éventuellement un autre hétéroatome ;
R^{8.1.1}, R^{8.1.2} identiques ou différents, représentent un atome d'hydrogène ou un groupe alkyle en C₁ à C₆,
R⁹, R¹⁰ identiques ou différents, éventuellement substitués par OMe, CN, F, Cl ou Br, choisi dans le groupe consistant en groupes alkyle en C₁ à C₆, cycloalkyle en C₃ à C₈, cycloalkyle en C₃ à C₇-alkyle en C₁ à C₄ ou
R⁹, R¹⁰ identiques ou différents, représentent un atome d'hydrogène, COR^{9.1}, CONR^{9.1}R^{9.2}, SO₂R^{9.1} ou SO₂NR^{9.1}R^{9.2} ;
R^{9.1}, R^{9.2} identiques ou différents, représentent un atome d'hydrogène, ou un radical choisi dans le groupe consistant en groupes alkyle en C₁ à C₆ et cycloalkyle en C₃ à C₈ ;
ou
NR^{9.1}R^{9.2} forment conjointement un cycle à cinq ou six chaînons qui peut contenir éventuellement de l'oxygène comme autre hétéroatome.

**6.** Composés selon l'une des revendications 1 à 5, pour l'utilisation comme médicament.

**7.** Utilisation des composés selon l'une des revendications 1 à 5, pour la production d'un médicament pour le traitement de maladies inflammatoires et allergiques des voies respiratoires.

**8.** Utilisation selon la revendication 7, **caractérisée en ce qu'**il s'agit d'une maladie qui est choisie dans le groupe consistant en bronchite chronique, bronchite aiguë, bronchite due à une infection bactérienne ou virale ou des champignons ou des vers, bronchite allergique, bronchite toxique, bronchopneumopathie chronique obstructive (BPCO), asthme (intrinsèque ou allergique), asthme pédiatrique, bronchiectasies, alvéolite allergique, rhinite allergique ou non allergique, sinusite chronique, fibrose kystique ou mucoviscidose, déficit en antitrypsine alpha-1, toux, emphysème pulmonaire, maladies pulmonaires interstitielles, alvéolite, hyperréactivité des voies respiratoires, polypes nasaux, oedèmes pulmonaires, pneumopathie d'origines diverses telles qu'induite par rayonnement ou par aspiration ou d'origine infectieuse, collagénoses telles que lupus érythémateux systémique, sclérodermie, sarcoïdose et maladie de Boeck.

**9.** Utilisation selon la revendication 7, **caractérisée en ce qu'**il s'agit d'une maladie qui est choisie dans le groupe consistant en rhinite allergique, sinusite allergique et polypes nasaux.

**10.** Formulation pharmaceutique contenant un composé de formule (I) selon l'une des revendications 1 à 5.

**11.** Combinaisons médicamenteuses qui contiennent, outre un ou plusieurs composés de formule (I) selon l'une des revendications 1 à 5, comme autre substance active, un ou plusieurs composés qui sont choisis dans les classes des bêtamimétiques, des anticholinergiques, des corticoïdes, d'autres inhibiteurs de PDE4, des antagonistes de LTD4, des inhibiteurs de l'EGFR, des agonistes dopaminergiques, des anti-histaminiques H1, des antagonistes de PAF et des inhibiteurs de la P13-kinase ou des combinaisons doubles ou triples de ceux-ci.

**12.** Procédé de production de composés de formule générale (I), 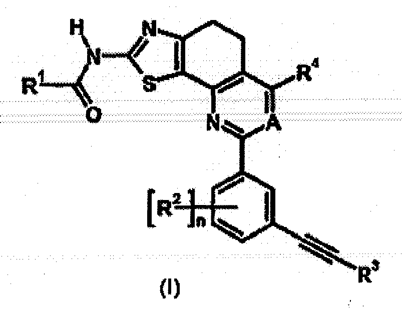 dans laquelle
A, R¹ à R⁴ peuvent avoir les significations indiquées dans la revendication 1,
**caractérisé en ce que**
(a) on fait réagir un composé de formule (II) 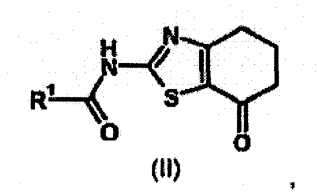 dans laquelle R¹ a la signification indiquée,
avec un composé de formule 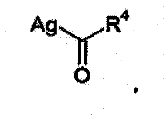 dans laquelle R⁴ a la signification indiquée et Ag est un groupe partant, et
(b) on fait réagir le composé obtenu de l'étape (a), de
formule générale (III) 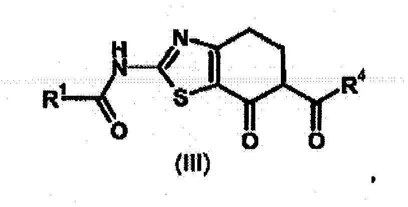 dans laquelle R¹ et R⁴ ont la signification indiquée,
avec un composé de formule générale 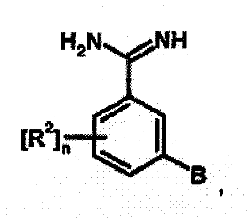 dans laquelle R² et n ont la signification indiquée et B représente un groupe partant, et
(c) on fait réagir le composé obtenu de l'étape (b) de formule générale (IV) 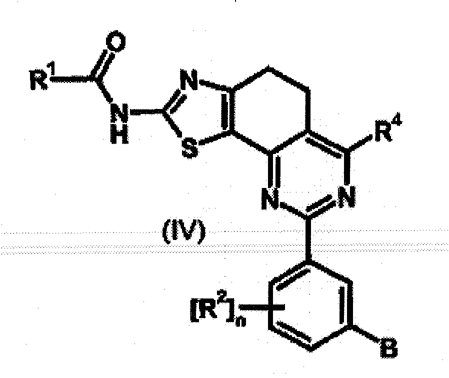 dans laquelle R¹, R², R⁴ et n ont les significations indiquées et B est un groupe partant,
avec un composé de formule générale 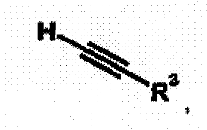 où R³ a la signification indiquée.

**13.** Composés selon la formule générale (IV) 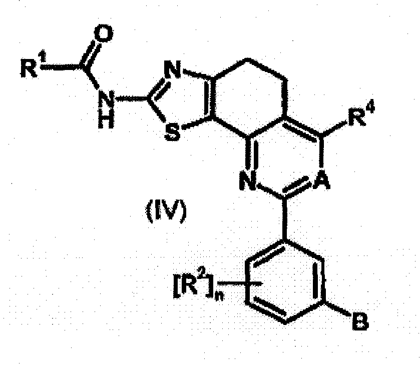 dans laquelle
dans laquelle A, R¹, R², R⁴ et n ont les significations indiquées dans la revendication 1 et B est un groupe partant,
éventuellement sous la forme de leurs tautomères, leurs racémates, leurs énantiomères, leurs diastéréomères et leurs mélanges, et éventuellement leurs sels d'addition acides pharmacologiquement inoffensifs,
à condition que R¹ ne puisse pas être un groupe méthyle, si R² = H, B = Cl et R⁴ = H.

**1.** Composés de formule générale (I) 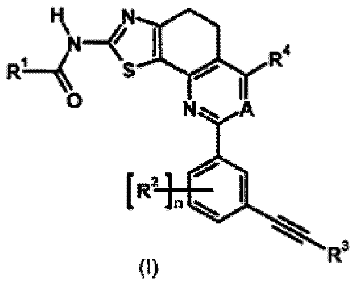 dans laquelle
A représente CH ou N,
n est 1, 2, 3 ou 4,
R¹ représente un atome d' hydrogène ou un radical consistant en un groupe alkyle en C₁ à C₄, OR^{1.1} et NR^{1.1}R^{1.2} ;
R^{1.1}, R^{1.2} identiques ou différents, représentent H ou un groupe alkyle en C₁ à C₄ ; ou
NR^{1.1}R^{1.2} représente un hétérocycle de 5 à 6 chaînons, contenant éventuellement un autre atome de N ;
R² identiques ou différents représentent un atome d'hydrogène ou
un radical choisi dans le groupe consistant en F, Cl, Br, I, CN, CF₃, CF₂H, CFH₂ et NH₂ ;
ou
un radical choisi dans le groupe consistant en groupes O-alkyle en C₁ à C₄, alkyle en C₁ à C₄ et alcényle en C₂ à C₆ ;
R⁴ représente un atome d'hydrogène, OH, NH₂ ou un radical choisi dans le groupe consistant en groupes alkyle en C₁ à C₆, alcényle en C₂ à C₆, cycloalkyle en C₃ à C₆, -N(alkyle en C₁ à C₄)₂ et - NH(alkyle en C₁ à ₄) ;
R³ représente un radical choisi dans le groupe consistant en : 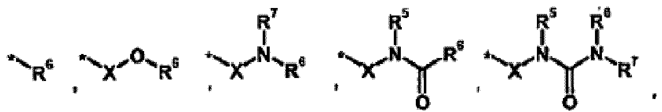 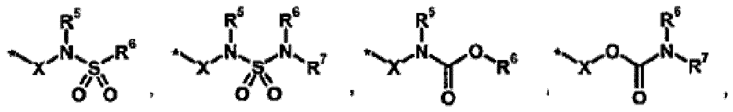 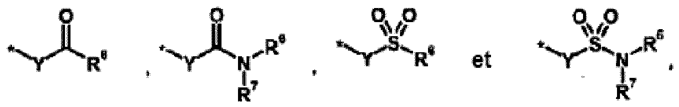 où
X représente un radical choisi dans le groupe consistant en groupes alkylène en C₁ à C₆, alcénylène en C₂ à C₅, alcinylène en C₁ à C₅, cycloalkylène en C₃ à C₇, cycloalcénylène en C₅ à C₇ et alkylène en C₁ à C₄-cycloalkylène en C₃ à C₇ ;
Y représente une liaison ou X ;
R⁵,R⁶,R⁷ identiques ou différents, représentent un atome d'hydrogène ou un radical choisi dans le groupe consistant en groupes alkyle en C₁ à C₆, alcényle en C₂ à C₆, alcinyle en C₂ à C₆, halogénoalkyle en C₁ à C₆, cycloalkyle en C₃ à C₈, cycloalcényle en C₃ à C₈, cycloalkyle en C₃ à C₇-alkyle en C₁ à C₄, aryle, hétéroaryle, spiro, hétérocycloalkyle, aryl-alkyle en C₁ à C₆, hétéroaryl-alkyle en C₁ à C₆ et hétérocycloalkyle-alkyle en C₁ à C₆,
ou
NR⁶R⁷ forment un cycle à cinq, six ou sept chaînons consistant en atomes de carbone et éventuellement un atome d'azote, d'oxygène ou de soufre comme autres hétéroatomes ou
un cycle choisi dans le groupe consistant en : 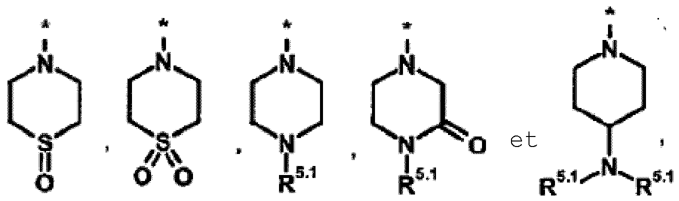
R^{5.1} identiques ou différents, représentent un atome d'hydrogène ou un radical choisi dans le groupe consistant en groupe alkyle en C₁ à C₆, cycloalkyle en C₃ à C₈, -CO-alkyle en C₁ à C₃ et CONH₂ ;
ou
R⁵ et R⁶ forment conjointement un pont alkylène saturé ou insaturé et peuvent contenir éventuellement un autre atome d'azote, d'oxygène ou de soufre ;
ou R³ est 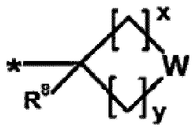
x,y identiques ou différents sont 0, 1, 2, 3, 4 ou 5 ;
W représente O, NR⁹ ou CR⁹R¹⁰ ;
R⁸ représente H, OR^{8.1}, NR^{8.1}R^{8.2} ou un groupe alkyle en C₁ à C₆ ;
R^{8.1}, R^{8.2} identiques ou différents représentent un atome d'hydrogène, COR^{8.1.1}, CONR^{8.1.1}R^{8.1.2}, SO₂NR^{8.1.1}R^{8.1.2} ou SO₂R^{8.1.1}
ou un radical choisi dans le groupe consistant en groupe alkyle en C₁ à C₆, alcényle en C₃ à C₆, alcinyle en C₃ à C₆, cycloalkyle en C₃ à C₈ et cycloalkyle en C₃ à C₇-alkyle en C₁ à C₄, ou
NR^{8.1}R^{8.2} forment conjointement un cycle à cinq, six ou sept chaînons qui peut contenir éventuellement un autre hétéroatome ;
R^{8.1.1},R^{8.1.2} identiques ou différents, représentent un atome d'hydrogène ou un radical choisi dans le groupe consistant en groupe alkyle en C₁ à C₆, cycloalkyle en C₃ à C₈ et cycloalkyle en C₃ à C₇-alkyle en C₁ à C₄, ou
NR^{8.1.1}R^{8.1.2} forment conjointement un cycle à cinq ou six chaînons qui peut contenir éventuellement un autre hétéroatome ;
R⁹,R¹⁰ identiques ou différents, représentent un radical éventuellement substitué par OMe, CN, F, Cl ou Br, choisi dans le groupe consistant en groupes alkyle en C₁ à C₆, alcényle en C₂ à C₆, alcinyle en C₂ à C₆, cycloalkyle en C₃ à C₈, cycloalcényle en C₃ à C₈, cycloalkyle en C₃ à C₇-alkyle en C₁ à C₄, aryle, hétéroaryle, spiro, hétérocycloalkyle, aryle-alkyle en C₇ à C₁₁-(aryl-alkyle en C₁ à C₆) et hétéroaryle-alkyle en C₆ à C₁₀-(hétéroaryl-alkyle en C₁ à C₆) ; ou
R⁹,R¹⁰ identiques ou différents, représentent un atome d'hydrogène, COR^{9.1}, CONR^{9.1}R⁹.², SO₂R^{9.1} ou SO₂NR^{9.1}R^{9.2} ;
R^{9.1},R⁹.² identiques ou différents, représentant un atome d'hydrogène, ou un radical choisi dans le groupe consistant en groupes alkyle en C₁ à C₆, alcényle en C₂ à C₆, alcinyle en C₂ à C₆, halogénoalkyle en C₁ à C₆, cycloalkyle en C₃ à C₈, cycloalkyle en C₃ à C₇-alkyle en C₁ à C₄, aryle, hétéroaryle, spiro, hétérocycloalkyle, aryl-alkyle en C₁ à C₆ et hétéroaryl-alkyle en C₁ à C₆ ;
ou
NR^{9.1}R^{9.2} forment conjointement un cycle à cinq ou six chaînons qui peut contenir éventuellement un autre hétéroatome,
éventuellement sous la forme de leurs tautomères, leurs racémates, leurs énantiomères, leurs diastéréomères et leurs mélanges, et éventuellement leurs sels, solvates et hydrates pharmacologiquement inoffensifs.

**2.** Composés de formule (IA) selon la revendication 1, 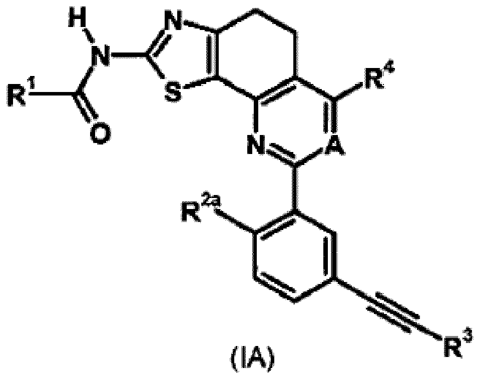 dans laquelle
A, R¹, R³ et R⁴ ont les significations indiquées et
R^{2a} représente un radical choisi dans le groupe consistant en F, Cl, Br, I, CN, CF₃, CF₂H, CFH₂ et NH₂ ;
ou
un radical choisi dans le groupe consistant en groupes O-alkyle en C₁ à C₄, alkyle en C₁ à C₄ et alcényle en C₂ à C₈.

**3.** Composés selon la revendication 1 ou 2, dans lesquels
R³ peut avoir les significations indiquées et
n est 1 ou 2,
R¹ représente un groupe alkyle en C₁ à C₄ ou NR^{1.1}R^{1.2},
R^{1.1},R^{1.2} identiques ou différents, représentent H ou un groupe alkyle en C₁ à C₄ ;
R² ou R^{2a} identiques ou différents, représentent un atome d'hydrogène, F ou Cl ;
et
R⁴ représente un atome d'hydrogène.

**4.** Composés selon l'une des revendications 1 à 3, dans lesquels
R¹, R² et R⁴ peuvent avoir les significations indiquées et
R³ représente un radical choisi dans le groupe consistant en : 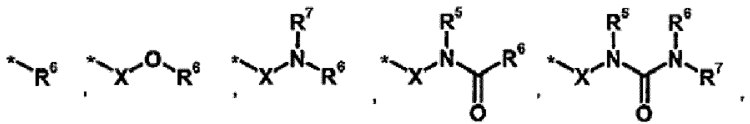 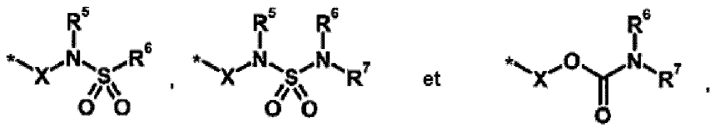 dans lequel
X représente un groupe alkylène en C₁ à C₃,
R⁵,R⁶,R⁷ identiques ou différentes, représentent un atome d'hydrogène ou un radical choisi dans le groupe consistant en groupes alkyle en C₁ à C₆, alcényle en C₂ à C₆, halogénoalkyle en C₁ à C₆, cycloalkyle en C₃ à C₈, cycloalkyle en C₃ à C₇-alkyle en C₁ à C₄, aryle, hétéroaryle, hétérocycloalkyle, aryl-alkyle en C₁ à C₅, hétéroaryl-alkyle en C₁ à C₅ et hétérocycloalkyle-alkyle en C₁ à C₅, et N (alkyle en C₁ à C₃)₂-alkyle en C₁ à C₄
ou
NR⁶R⁷ forment un cycle à cinq ou six chaînons consistant en atomes de carbone et éventuellement, en un atome d'azote ou un atome d'oxygène comme autre hétéroatome,
ou
NR⁶R⁷ forment un cycle choisi dans le groupe consistant en : 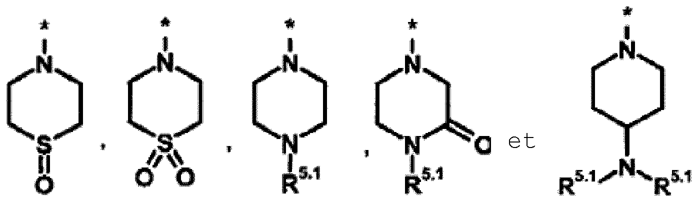
R^{5.1} identiques ou différents, représentent un atome d'hydrogène ou un radical choisi dans le groupe consistant en groupe alkyle en C₁ à C₆, cycloalkyle en C₃ à C₈, -CO-alkyle en C₁ à C₃ ou CONH₂ ;

**5.** Composés selon l'une des revendications 1 à 3, dans lesquels
R¹, R² et R⁴ peuvent avoir les significations indiquées et
R³ représente un radical : 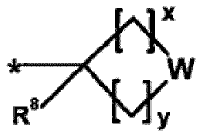
x,y identiques ou différents sont 0, 1, 2 ou 3,
W représente NR⁹ ou CR⁹R¹⁰ ;
R⁸ représente H, OR^{8.1} ou NR^{8.1}R^{8.2},
R^{8.1},R^{8.2} identiques ou différents représentent un atome d'hydrogène, COR^{8.1.1}, CONR^{8.1.1}R^{8.1.2}, ou un groupe alkyle en C₁ à C₆ ;
NR^{8.1}R^{8.2} forment conjointement un cycle à cinq ou six chaînons qui peut contenir éventuellement un autre hétéroatome ;
R^{8.1.1},R^{8.1.2} identiques ou différents, représentent un atome d'hydrogène ou un groupe alkyle en C₁ à C₆,
R⁹,R¹⁰ identiques ou différents, éventuellement substitués par OMe, CN, F, Cl ou Br, choisi dans le groupe consistant en groupes alkyle en C₁ à C₆, cycloalkyle en C₃ à C₈, cycloalkyle en C₃ à C₇-alkyle en C₁ à C₄ ou
R⁹,R¹⁰ identiques ou différents, représentent un atome d'hydrogène, COR^{9.1}, CONR^{9.1}R^{9.2}, SO₂R⁹.¹ ou SO₂NR^{9.1}R^{9.2} ;
R^{9.1}_{,}R^{9.2} identiques ou différents, représentent un atome d'hydrogène, ou un radical choisi dans le groupe consistant en groupes alkyle en C₁ à C₆ et cycloalkyle en C₃ à C₈ ;
ou
NR^{9.1}R^{9.2} forment conjointement un cycle à cinq ou six chaînons qui peut contenir éventuellement de l'oxygène comme autre hétéroatome.

**6.** Composés selon l'une des revendications 1 à 5, pour l'utilisation comme médicament.

**7.** Utilisation des composés selon l'une des revendications 1 à 5, pour la production d'un médicament pour le traitement de maladies inflammatoires et allergiques des voies respiratoires.

**8.** Utilisation selon la revendication 7, **caractérisée en ce qu'**il s'agit d'une maladie qui est choisie dans le groupe consistant en bronchite chronique, bronchite aiguë, bronchite due à une infection bactérienne ou virale ou des champignons ou des vers, bronchite allergique, bronchite toxique, bronchopneumopathie chronique obstructive (BPCO), asthme (intrinsèque ou allergique), asthme pédiatrique, bronchiectasies, alvéolite allergique, rhinite allergique ou non allergique, sinusite chronique, fibrose kystique ou mucoviscidose, déficit en antitrypsine alpha-1, toux, emphysème pulmonaire, maladies pulmonaires interstitielles, alvéolite, hyperréactivité des voies respiratoires, polypes nasaux, oedèmes pulmonaires, pneumopathie d'origines diverses telles qu'induite par rayonnement ou par aspiration ou d'origine infectieuse, collagénoses telles que lupus érythémateux systémique, sclérodermie, sarcoïdose et maladie de Boeck.

**9.** Utilisation selon la revendication 7, **caractérisée en ce qu'**il s'agit d'une maladie qui est choisie dans le groupe consistant en rhinite allergique, sinusite allergique et polypes nasaux.

**10.** Formulation pharmaceutique contenant un composé de formule (I) selon l'une des revendications 1 à 5.

**11.** Combinaisons médicamenteuses qui contiennent, outre un ou plusieurs composés de formule (I) selon l'une des revendications 1 à 5, comme autre substance active, un ou plusieurs composés qui sont choisis dans les classes des bêtamimétiques, des anticholinergiques, des corticoïdes, d'autres inhibiteurs de PDE4, des antagonistes de LTD4, des inhibiteurs de l'EGFR, des agonistes dopaminergiques, des anti-histaminiques H1, des antagonistes de PAF et des inhibiteurs de la P13-kinase ou des combinaisons doubles ou triples de ceux-ci.

**12.** Procédé de production de composés de formule générale (I), 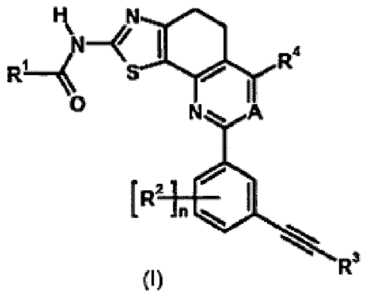 dans laquelle
A, R¹ à R⁴ peuvent avoir les significations indiquées dans la revendication 1,
**caractérisé en ce que**
(a) on fait réagir un composé de formule (II) 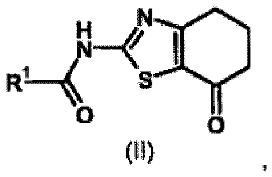 dans laquelle R¹ a la signification indiquée,
avec un composé de formule 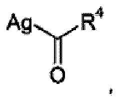 dans laquelle R⁴ a la signification indiquée et Ag est un groupe partant, et
(b) on fait réagir le composé obtenu de l'étape (a), de formule générale (III) 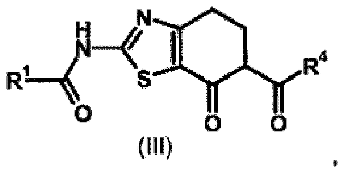 dans laquelle R¹ et R⁴ ont la signification indiquée,
avec un composé de formule générale 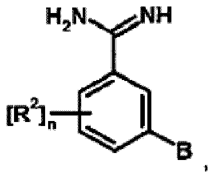 dans laquelle R² et n ont la signification indiquée et B représente un groupe partant, et
(c) on fait réagir le composé obtenu de l'étape (b) de formule générale (IV) 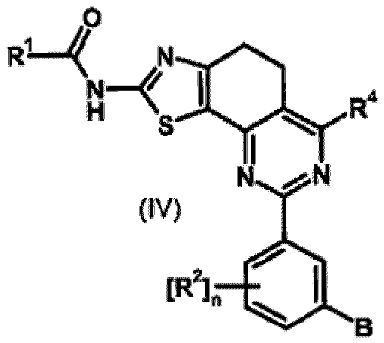 dans laquelle R¹, R², R⁴ et n ont les significations indiquées et B est un groupe partant,
avec un composé de formule générale 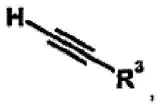 où R³ a la signification indiquée.

**13.** Composés selon la formule générale (IV) 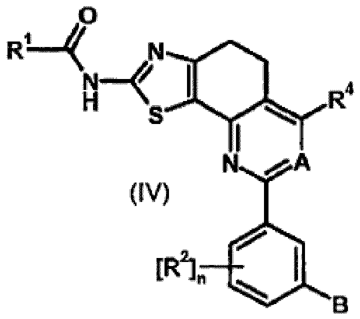 dans laquelle
dans laquelle A, R¹, R², R⁴ et n ont les significations indiquées dans la revendication 1 et B est un groupe partant,
éventuellement sous la forme de leurs tautomères, leurs racémates, leurs énantiomères, leurs diastéréomères et leurs mélanges, et éventuellement leurs sels d'addition acides pharmacologiquement inoffensifs,
à condition que R¹ ne puisse pas être un groupe méthyle, si R² = H, B = Cl et R⁴ = H.
